# EUROPEAN PATENT APPLICATION

(11) **EP 4 101 929 A1**
(43) Date of publication of application: **14.12.2022**
(21) Application number: 20906717.2
(22) Date of filing: 25.12.2020
(51) Int. Cl.: C12N 15/11, A61K 38/12, A61P 7/02, A61P 37/06, A61P 43/00, C07K 5/12, C07K 7/50, C07K 7/64, C07K 14/715, C07K 14/745, C07K 19/00, C12P 21/04, C40B 40/08, C40B 40/10

(54) **LIBRARY CONSTRUCTION METHOD, CYCLIC PEPTIDE, FXIIA BINDER AND IFNGR1 BINDER**

(30) Priority: 27.12.2019 JP 2019239426; 06.08.2020 JP 2020134234
(71) Applicant: The University of Tokyo, Bunkyo-ku, Tokyo 113-8654 (JP)
(72) Inventor: SUGA, Hiroaki, Tokyo 113-8654 (JP); KATOH, Takayuki, Tokyo 113-8654 (JP)
(74) Representative: Mewburn Ellis LLP
(86) International application number: PCT/JP2020/048981
(87) International publication number: WO 2021/132661

(57) **Abstract**

The purpose of the present invention is to provide a method of producing a library including two or more cyclic peptides, wherein at least one of the cyclic peptides included in the library has a structure composed of 4 to 30 amino acids or derivatives thereof and contains, in the structure, at least one selected from cyclic β-, γ-, and δ-amino acids (cAAs), including a step of preparing an mRNA library encoding a peptide having a sequence represented by the formula (1); -(Xaa)ₙ₁- [in the formula (1), Xaas are each an arbitrary amino acid or derivative thereof, at least one Xaa is one selected from cyclic β-, γ-, and δ-amino acids (cAAs) and n1 is an integer of 2 to 28] and a step of using the mRNA library to express the peptide in a cell-free translation system and produce a library.

## Description

### Technical Field

The present invention relates to a production method for a library, a cyclic peptide, binding agents for FXIIa and IFNGR1, and the like.

### Background Art

Non-natural amino acids other than α-amino acids, such as β- and γ-ones are present in natural products and they are known to contribute to bioactivity, peptidase resistance, and target specificity, and moreover, membrane permeability.

In peptide synthesis using a ribosome translation system, 20 natural α-amino acids are generally used, so that it is the common practice to obtain a naturally occurring peptide containing a non-natural amino acid such as β- or γ-amino acid by the post translational modification or nonribosomal peptide synthesis.

A method of using genetic code manipulation for the ribosomal incorporation of β- or γ-amino acid or the like into a peptide chain has been studied, but it is presumed to be impossible to achieve consecutive elongation because of an essentially low incorporation efficiency of a non-natural amino acid.

The incorporation efficiency is presumed to be low mainly because the introduction speed of β-aminoacyl-tRNA into the A site of the ribosome by EF-Tu is slow and the speed of peptidyl transfer between β-amino acids is slow. Moreover, the slow peptidyl transfer is presumed to cause ribosomal stalling and drop-off of peptidyl-tRNA from the ribosome.

For example, Patent Document 1 discloses, as a method of improving the incorporation efficiency of a non-natural amino acid, a method of using a translation factor EF-P and a recombined tRNA^{Pro1E2} for ribosomal synthesis. According to the method disclosed in Patent Document 1, use of tRNA^{Pro1E2} improves the incorporation efficiency of a specific D-amino acid and non-cyclic β-amino acid, making it possible to perform incorporation of seven consecutive ones into a peptide.

### Citation List

### Patent Document

Patent Document 1: WO2019/077887

### Summary

### Technical Problem

From the standpoint of structural diversity or improving the properties of a compound, there is a demand for the development of a method of efficiently producing various peptides containing a non-natural amino acid.

It is said that a non-natural amino acid such as D-amino acid or non-cyclic β-amino acid can be incorporated at an improved efficiency by the method of Patent Document 1 using tRNA. Patent Document 1, however, has not studied the ribosomal synthesis of a peptide containing a non-natural amino acid other than the aforesaid non-natural amino acid.

### Solution to Problem

The present inventors have conducted intensive investigation in order to solve the aforesaid problem. As a result, it has been found that a cyclic peptide containing a cyclic β-, γ-, or δ-amino acid is expressed using a cell-free translation system, leading to the completion of the present invention. It is to be noted that the term "cyclic β-, γ-, or δ-amino acid" as used herein may be called "cAA".

The present invention is as follows.
[1] A method of producing a library including two or more cyclic peptides, wherein at least one of the cyclic peptides contained in the library has a cyclic structure having 4 to 30 amino acids or derivatives thereof and contains, in the cyclic structure, at least one selected from cyclic β-, γ-, and δ-amino acids (cAAs), including:
   a step of preparing an mRNA library encoding a peptide having a sequence represented by the following formula (1);

   -(Xaa)ₙ₁- (1)

   [in the formula (1),
   Xaas are each an arbitrary amino acid or derivative thereof,
   at least one of Xaas is one selected from cyclic β-, γ-, and δ-amino acids (cAAs), and
   n1 is an integer of 2 to 28]; and
   a step of using the mRNA library to express the peptide in a cell-free translation system and produce the library.
[2] A method of producing a library including two or more cyclic peptides, wherein at least one of the cyclic peptides contained in the library has a cyclic structure having 4 to 30 amino acids or derivatives thereof and contains, in the cyclic structure, at least one selected from cyclic β-, γ-, and δ-amino acids (cAAs), including:
   a step of preparing an mRNA library encoding a peptide having a sequence represented by the following formula (1);

   -(Xaa)ₙ₁- (1)

   [in the formula (1),
   Xaas are each an arbitrary amino acid or derivative thereof,
   at least one of Xaas is one selected from cyclic β-, γ-, and δ-amino acids (cAAs), and
   n1 is an integer of 2 to 28];
   a step of binding puromycin to the 3' end of each of the mRNAs of the mRNA library to produce a puromycin-bound mRNA library; and
   a step of using the puromycin-bound mRNA library to express the peptide in a cell-free translation system and produce a peptide-mRNA complex library.
[3] The method of producing a library as described in [1] or [2], wherein the cell-free translation system contains a tRNA charged with one selected from cyclic β-, γ-, and δ-amino acids (cAAs) and the tRNA is a tRNA having a D-arm structure interactive with EF-P or a tRNA not having a D-arm structure interactive with EF-P.
[4] The method of producing a library as described in any of [1] to [3], wherein the cell-free translation system contains a tRNA charged with one selected from cyclic β-, γ-, and δ-amino acids (cAAs) and the tRNA is at least one selected from tRNA^{Pro1E2} and tRNA^{GluE2}.
[5] The method of producing a library as described in any of [1] to [4], wherein the peptide having the sequence represented by the formula (1) is represented by the following formula (2);

   Xaa1-(Xaa)ₙ₁-Xaa2-(Xaaₓ)ₘ (2)

   [in the formula (2),
   Xaas are each an arbitrary amino acid or derivative thereof,
   at least one of Xaas is one selected from cyclic β-, γ-, and δ-amino acids (cAAs),
   Xaa1 and Xaa2 are each an amino acid or derivative thereof which forms a ring of the cyclic peptide,
   Xaaₓ is an arbitrary amino acid or derivative thereof, and
   n1 is an integer of 2 to 28, and
   m is an integer of 0 to 10.
[6] The method of producing a library as described in any of [1] to [5], wherein the cyclic β-, γ-, and δ-amino acids are each represented by any of the following formulas (I-1); 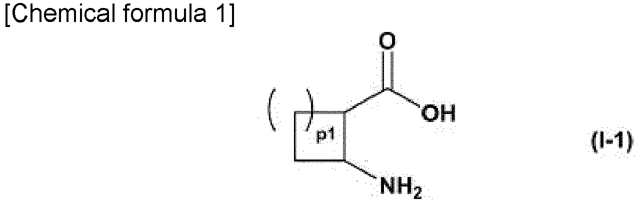
   (in the formula (I-1), p1 is any integer of 1 to 4), the following formula (I-2); 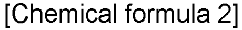 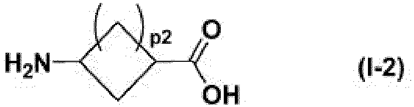
   (in the formula (1-2), p2 is any integer of 1 to 4); the following formula (I-3); 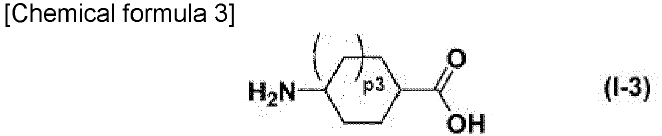
   (in the formula (I-3), p3 is an integer of 1 or 2); and
   the following formula (I-4);

      H₂N-Ar-COOH (I-4)
   (in the formula (I-4), Ar is a divalent aromatic group whose aromatic ring may be substituted with one or more substituents).
[7] The method of producing a library as described in any of [1] to [6], wherein two or more cAAs are contained at random in the sequence represented by the formula (1).
[8] The method of producing a library as described in any of [1] to [7], wherein two or more consecutive cAAs are contained in the sequence represented by the formula (1).
[9] The method of producing a library as described in any of [1] to [8], wherein 3 or more and 15 or less consecutive cAAs are contained in the sequence represented by the formula (1) and are each a cyclic β-amino acid represented by (1S,2S)-2-ACPC: 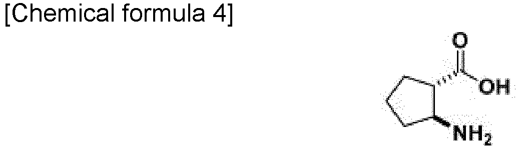
[10] The method of producing a library as described in any of [1] to [8], wherein the cAA is at least one cyclic β- or γ-amino acid (i) selected from: 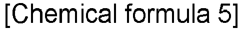 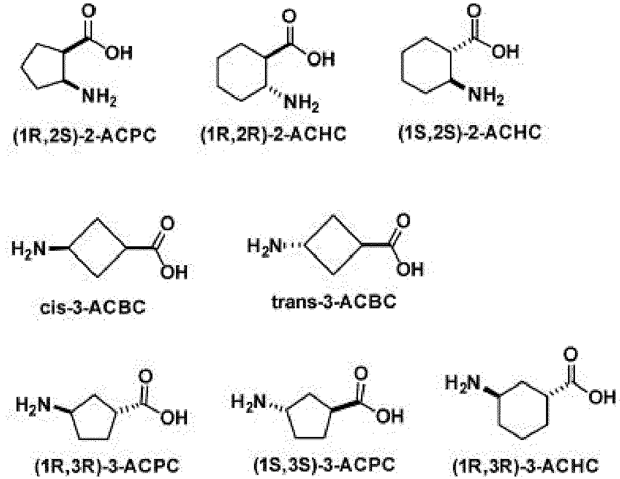 and/or at least one cyclic β- or γ-amino acid (ii) selected from: 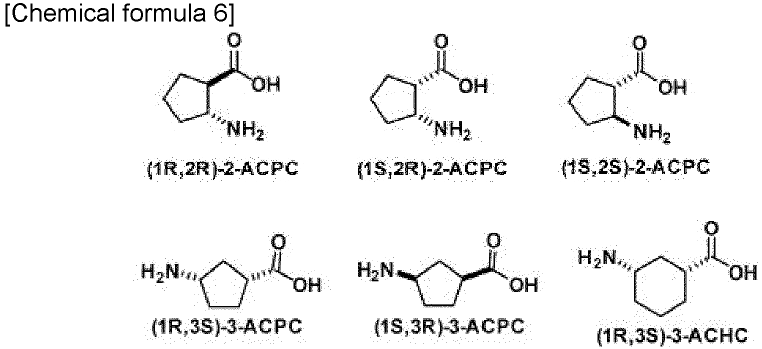 and
   the cell-free translation system contains a tRNA charged with the cyclic β- or γ-amino acid (i) and having a D-arm structure interactive with EF-P, and/or
   a tRNA charged with the cyclic β- or γ-amino acid (ii) and not having a D-arm structure interactive with EF-P.
[11] The method of producing a library as described in [10], wherein the tRNA charged with the cyclic β- or γ-amino acid (i) and having a D-arm structure interactive with EF-P is tRNA^{Pro1E2} and the tRNA charged with the cyclic β- or γ-amino acid (ii) and not having a D-arm structure interactive with EF-P is tRNA^{GluE2}.
[12] A cyclic peptide including a cyclic structure composed of 4 to 30 amino acids or derivatives thereof or a pharmaceutically acceptable salt of the cyclic peptide, wherein
   of the 4 to 30 amino acids or derivatives of the cyclic structure, two amino acids or derivatives thereof, that is, Xaa1 and Xaa2, contain a structure for forming the cyclic structure,
   the Xaa1 and the Xaa2 have a linked structure via an amino acid sequence composed of 2 to 28 amino acids or derivatives thereof,
   the amino acid sequence composed of 2 to 28 amino acids or derivatives thereof is composed of at least one selected from cyclic β-, γ-, and δ-amino acids (cAAs) and an arbitrary amino acid or derivative thereof.
[13] The cyclic peptide or pharmaceutically acceptable salt thereof as described in [12], wherein the cyclic peptide is represented by the following formula (3);

   Xaa1-(Xaa)ₙ₁-Xaa2-(Xaaₓ)ₘ (3)

   [in the formula (3),
   Xaas are each an arbitrary amino acid or derivative thereof,
   at least one of the Xaas is a cyclic β-amino acid (cβAA),
   Xaa1 and Xaa2 are amino acids or derivatives thereof forming the ring of the cyclic peptide,
   n1 is an integer of 10 to 16,
   Xaaₓ is an arbitrary amino acid or derivative thereof, and
   m is an integer of 0 to 10], and
   supposing that Xaa1 is a first amino acid, at least one cAA is present in a 5 to 9-th position.
[14] The cyclic peptide or pharmaceutically acceptable salt thereof as described in [13], wherein at least one of Xaas is a basic amino acid or derivative thereof.
[15] The cyclic peptide or pharmaceutically acceptable salt thereof as described in [13] or [14], wherein (Xaa)ₙ₁ in the formula (3) is any one selected from the following f1 to f4: 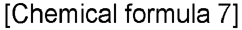 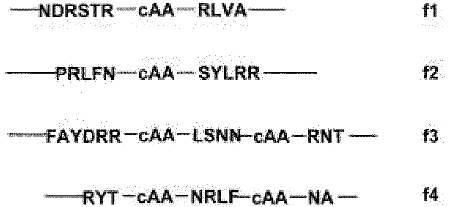 [in the formula, cAA is a cyclic β-amino acid].
[16] The cyclic peptide or pharmaceutically acceptable salt thereof as described in [13], wherein the cyclic peptide is represented by the following formula (3-1); 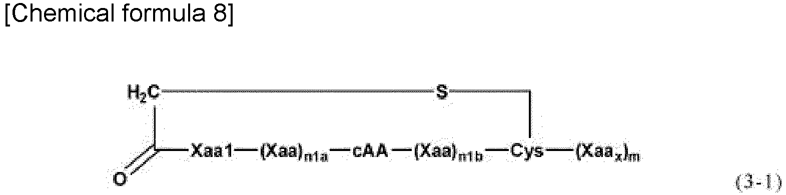 [in the formula (3-1),
   Xaas are each an arbitrary amino acid or derivative thereof,
   cAA is a cyclic β-amino acid,
   n1a is an integer of 3 to 7 and n1b is an integer of 2 to 8 (with the proviso that the sum of n1a and n1b is an integer of 9 to 15),
   Xaaₓ is an arbitrary amino acid or derivative thereof,
   Xaa1 is Phe or Tyr, and
   m is an integer of 0 to 10, with the proviso that (Xaa)ₙ₁ₐ does not contain cAA].
[17] The cyclic peptide or pharmaceutically acceptable salt thereof as described in [16], wherein at least one of Xaas is a basic amino acid or derivative thereof.
[18] The cyclic peptide or pharmaceutically acceptable salt thereof as described in [16] or [17], wherein a difference (absolute value) between n1a and n1b is 4 or less.
[19] The cyclic peptide or pharmaceutically acceptable salt thereof as described in any of [13] to [18], wherein the cyclic peptide is any one of the following F1 to F4; 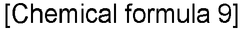 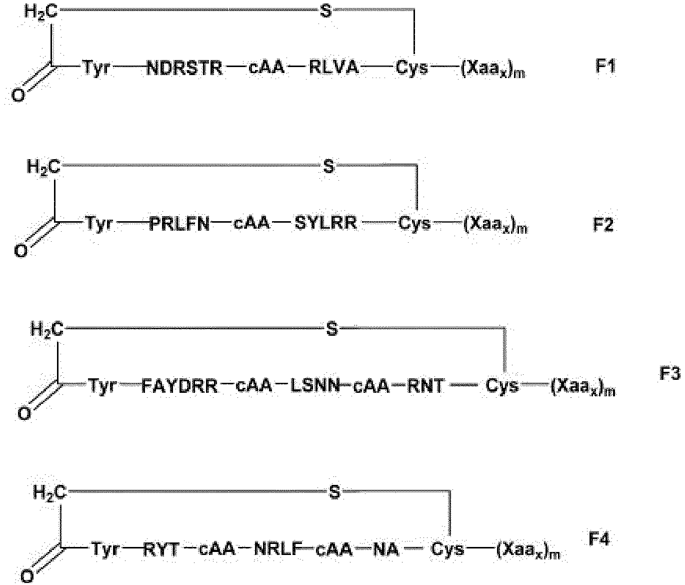 [in the formula,
   cAAs are each independently a cyclic β-amino acid,
   Xaaₓ is an arbitrary amino acid or derivative thereof, and
   m is an integer of 0 to 10].
[20] The cyclic peptide or pharmaceutically acceptable salt thereof as described in any of [13] to [19], wherein the cyclic β-amino acid is represented by the following formula (I-1); 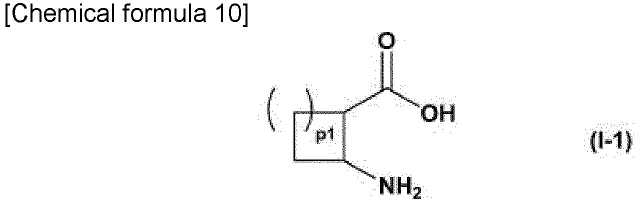 (in the formula (I-1), p1 is any integer of 1 to 4).
[21] The cyclic peptide or pharmaceutically acceptable salt thereof as described in [12], wherein the cyclic peptide is represented by the following formula (4);

   Xaa1-(Xaa)ₙ₁-Xaa2-(Xaaₓ)ₘ (4)

   [in the formula (4),
   Xaas are each an arbitrary amino acid or derivative thereof,
   at least one of Xaas is a cyclic β-amino acid (cAA),
   Xaa1 and Xaa2 are amino acids or derivatives thereof forming the ring of the cyclic peptide,
   n1 is an integer of 9 to 15,
   Xaaₓ is an arbitrary amino acid or derivative thereof, and
   m is an integer of 0 to 10], and
   supposing that Xaa1 is a first amino acid, at least one cAA is present as a 5 to 8-th one.
[22] The cyclic peptide or pharmaceutically acceptable salt thereof as described in [21], wherein (Xaa)ₙ₁ in the formula (4) is any one selected from the following i1-1 to i1-6: 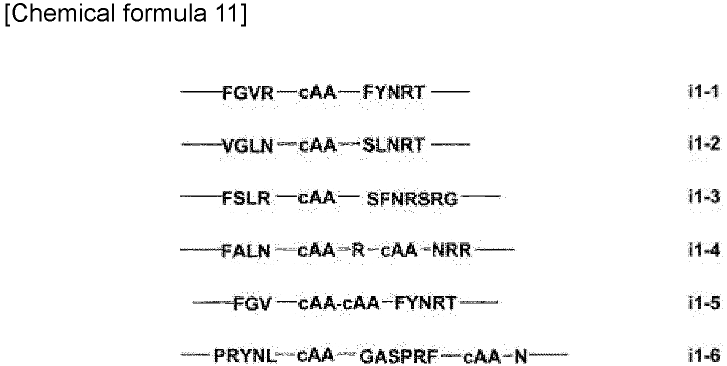 [in the formula, cAA is a cyclic β-amino acid].
[23] The cyclic peptide or pharmaceutically acceptable salt thereof as described in [21], wherein the cyclic peptide is represented by the following formula (4-1); 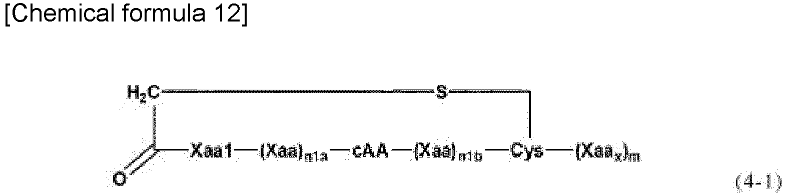 [in the formula (4-1),
   Xaa is an arbitrary amino acid or derivative thereof,
   cAA is a cyclic β-amino acid,
   n1a is an integer of 3 to 6 and n1b is an integer of 5 to 8 (with the proviso that the sum of n1a and n1b is an integer of 8 to 14),
   Xaaₓ is an arbitrary amino acid or derivative thereof,
   Xaa1 is Phe or Tyr, and
   m is an integer of 0 to 10, with the proviso that (Xaa)ₙ₁ₐ does not contain cAA].
[24] The cyclic peptide or pharmaceutically acceptable salt thereof as described in any of [21] to [23], wherein the cyclic peptide is any of the following I1-1 to 11-6; 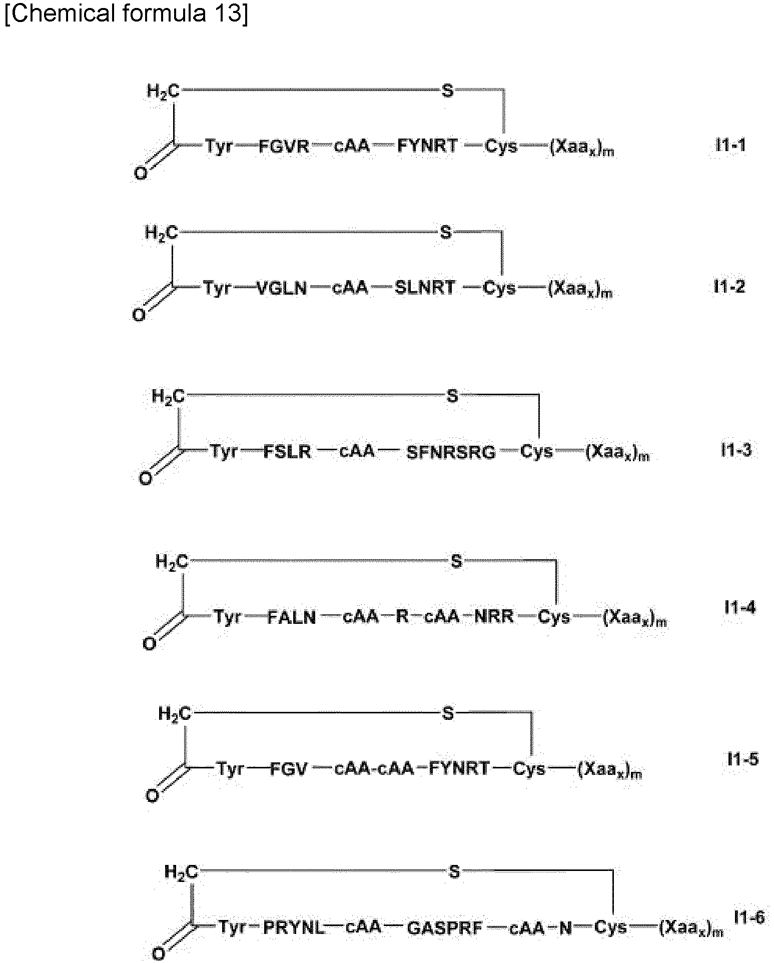 [in the formula,
   cAAs are each independently a cyclic β-amino acid,
   Xaaₓ is an arbitrary amino acid or derivative thereof, and
   m is an integer of 0 to 10].
[25] The cyclic peptide or pharmaceutically acceptable salt thereof as described in any of [21] to [24], wherein the cyclic β-amino acid is represented by the following formula (I-1); 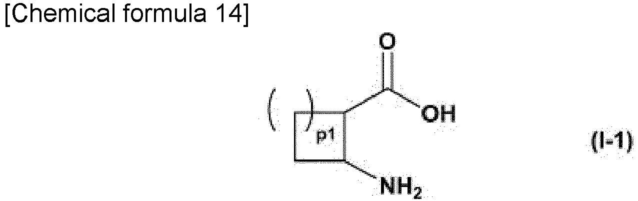 (in the formula (I-1), p1 is any integer of 1 to 4).
[26] A binding agent for activated blood coagulation factor XII (FXIIa), containing the cyclic peptide or pharmaceutically acceptable salt thereof as described in any of [13] to [20].
[27] A binding agent for type II interferon receptor complex (IFNGR1), containing the cyclic peptide or pharmaceutically acceptable salt thereof as described in any of [21] to [25].

### Advantageous Effects of Invention

The present invention can provide a method of producing a library including cyclic peptides containing cyclic β-, γ-, and δ-amino acids.

### Brief Description of Drawings

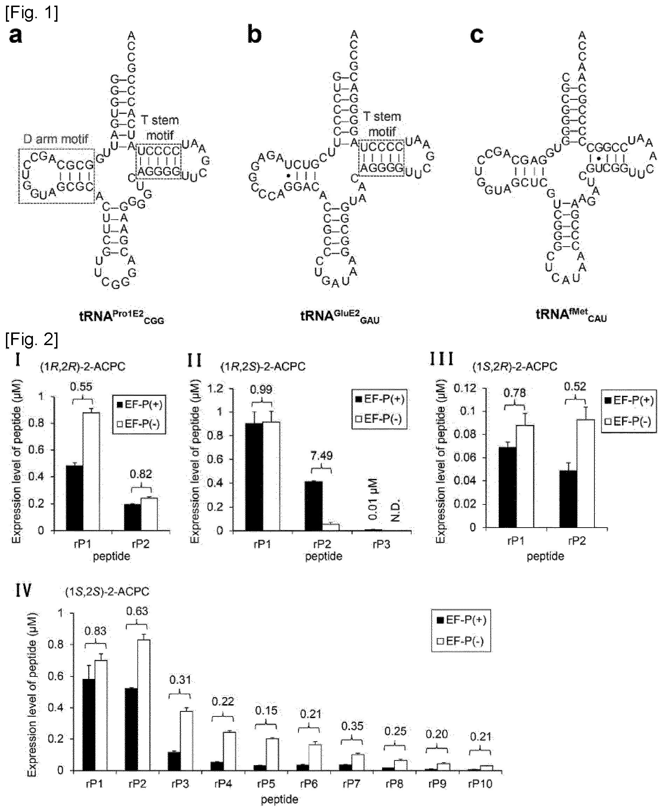

Fig. 1 shows the secondary structure of tRNA used for the incorporation of a cAA in Examples, in which Fig. 1a shows the secondary structure of tRNA^{Pro1E2}_{CGG}. The D-arm structure (D-arm motif) for EF-P binding consists of a D-loop of 9 bases closed by a stable 4 base pair stem. In addition, it has a T-stem (T-stem motif) for EF-Tu binding. Fig. 1b shows the secondary structure of tRNA^{GluE2}_{GAU}. It has a T-stem (T-stem motif) for EF-Tu binding, which is the same as that of tRNA^{Pro1E2}. Fig. 1c shows tRNA^{fMet}_{CAU} used for the incorporation of ^{CIAc}D-Phe by an initiation codon AUG.
Figs. 2I, 2II, 2III, and 2IV respectively show the results of the expression levels of peptides when (1R,2R)-2-ACPC, (1R,2S)-2-ACPC, (1S,2R)-2-ACPC, and (1S,2S)-2-ACPC were introduced into peptides rP1 to rP10. The 2-ACPC was assigned to a CCG codon by using pre-charged 2-ACPC-tRNA^{Pro1E2}_{CGG}. The black bar indicates the translation performed in the presence of EF-P (EF-P(+)) and the white bar indicates the translation performed in the absence of EF-P (EF-P(-)). The numeral on the bar graph is a ratio (EF-P(+)/EF-P(-)) of the expression level in the presence of EF-P to that in the absence of EF-P. The concentration of EF-P when the translation was performed in the presence of EF-P was 5 µM. The reaction time was set at 30 minutes. The term "N.D." means "not detected".

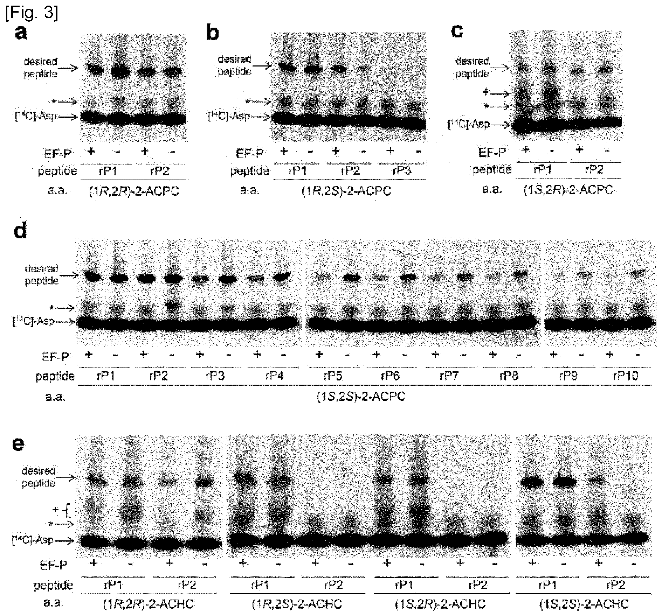

Figs. 3a, 3b, 3c, 3d, and 3e respectively show the tricine SDS-PAGE analysis results when (1R,2R)-2-ACPC, (1R,2S)-2-ACPC, (1S,2R)-2-ACPC, (1S,2S)-2-ACPC, and 2-ACHC were introduced into peptides, in which + indicates an impurity derived from the drop-off of peptidyl-tRNA and * indicates a peak of a template-independent impurity derived from the translation system.

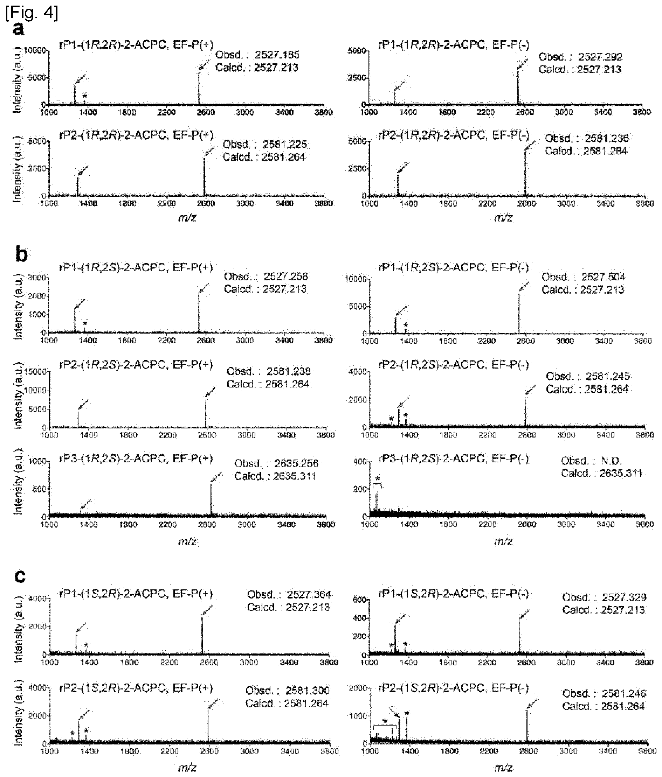

Figs. 4a, 4b, and 4c respectively show the MALDI-TOF MS spectra of peptides containing (1R,2R)-2-ACPC, (1R,2S)-2-ACPC, and (1S,2R)-2-ACPC, respectively. The concentration of EF-P in the translation in the presence of EF-P was 5 µM. The arrow indicates the peak of a desired peptide. The arrow on the high-molecular-weight side (high m/z side) indicates a monovalent ion and the arrow on the low-molecular-weight side (low m/z side) indicates a divalent ion. The terms "Calcd." and "Obsd." mean a calculated value and an observed value, respectively. The term "N.D." means "not detected". The * is a peak of a template-independent impurity derived from the translation system.

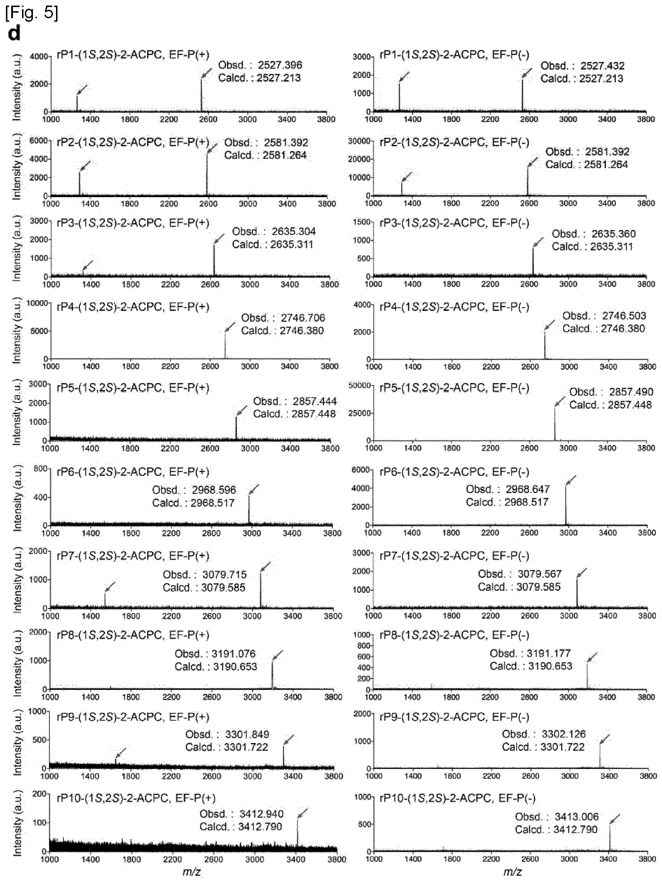

Fig. 5 shows the MALDI-TOF MS spectra of peptides containing (1S,2S)-2-ACPC. The concentration of EF-P in the translation in the presence of EF-P was 5 µm. The arrow indicates the peak of a desired peptide. The arrow, "Calcd.", and "Obsd." in the spectra have the same meanings as those in Fig. 4. The "N.D." means "not detected".

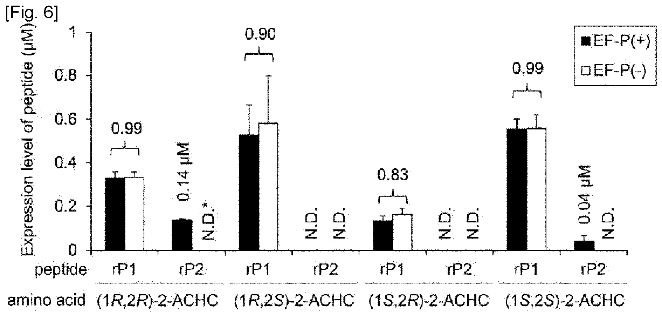

Fig. 6 shows the results of the expression levels of peptides when (1R,2R)-2-ACHC, (1R,2S)-2-ACHC, (1S,2R)-2-ACHC, and (1S,2S)-2-ACHC were introduced into a peptide rP1 or rP2. The 2-ACHC was assigned to a CCG codon by using pre-charged 2-ACHC-tRNA^{Pro1E2}_{CGG}. The black bar indicates the results of the translation performed in the presence of EF-P (EF-P(+)) and the white bar indicates the results of the translation performed in the absence of EF-P (EF-P(-)). The numeral on the bar graph is a ratio (EF-P(+)/EF-P(-)) of the expression level in the presence of EF-P to that in the absence of EF-P. The concentration of EF-P in the translation in the presence of EF-P was 5 µM. The reaction time was set at 30 minutes. The "N.D." means "not detected".

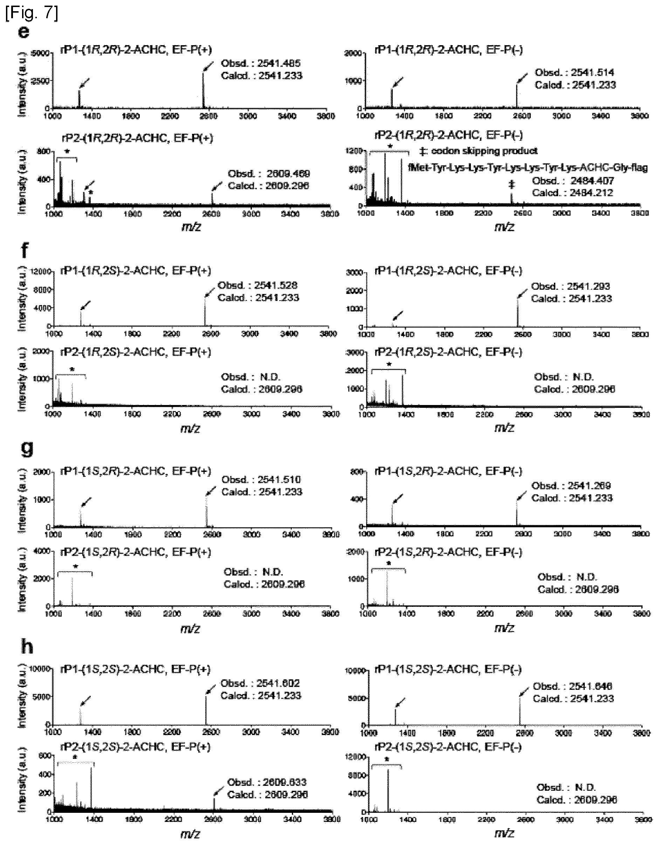

Figs. 7e, 7f, 7g, and 7h respectively show the MALDI-TOF MS spectra of peptides containing (1R,2R)-2-ACHC, (1R,2S)-2-ACHC, (1S,2R)-2-ACPC, and (1S,2S)-2-ACHC. The arrow, "Calcd.", "Obsd.", and * in the spectra have the same meanings as those in Fig. 4.

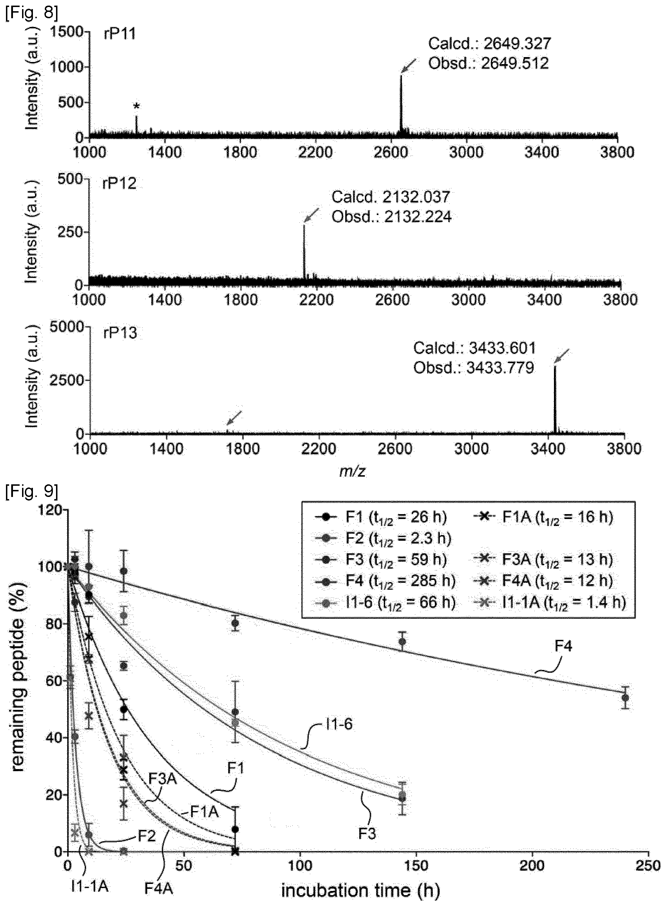

Fig. 8 shows the MALDI-TOF MS spectra of peptides rP11, rP12, and rP13. The arrow, "Calcd.", "Obsd.", "N.D." and * in the spectra have the same meanings as those in Fig. 4.
Fig. 9 shows the serum stability assay results of peptides bound to FXIIa and IFNGR1. Each of F1, F2, F3, F4, 11-6, F1A, F3A, F4A, and I1-1A was co-incubated with an internal standard peptide in the human serum at 37°C. The relative intensity of each peptide to the standard peptide was estimated by LC/MS at 0, 1, 3, 9, 24, 72, 144, and 240 hours. The relative intensity at 0 hour was defined as 100%.

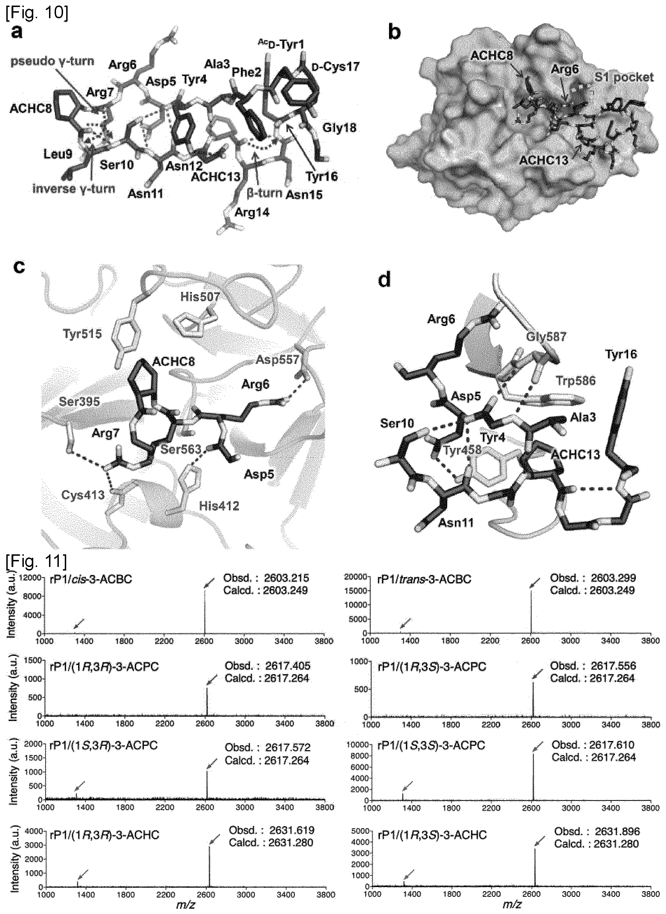

Fig. 10 shows the X-ray crystallographic analysis results of the structure of F3 bound to FXIIa, in which Fig. 10a is an overall structure of F3 shown by a stick model. Hydrogen bonding is indicated by a broken line and a local turn structure is present at the place indicated by an arrow. Fig. 10b is an overall structure of a FXIIa-F3 complex. The S1 pocket for housing an Arg residue (Arg6) of the substrate therein is indicated by a dotted line. Fig. 10c shows the binding of an Asp5-ACHC8 segment to FXIIa. Fig. 10d shows the binding of the molecular center portion of F3 to FXIIa.
Fig. 11 shows the MALDI-TOF MS spectra of peptides containing cis-3-ACBC, trans-3-ACBC, (1R,3S)-3-ACPC, (1R,3S)-3-ACPC, (1S,3R)-3-ACPC), (1S,3S)-3-ACPC, (1R,3R)-3-ACHC, and (1R,3S)-3-ACHC. The arrow, "Calcd.", "Obsd.", and * in the spectra have the same meanings as those in Fig. 4.

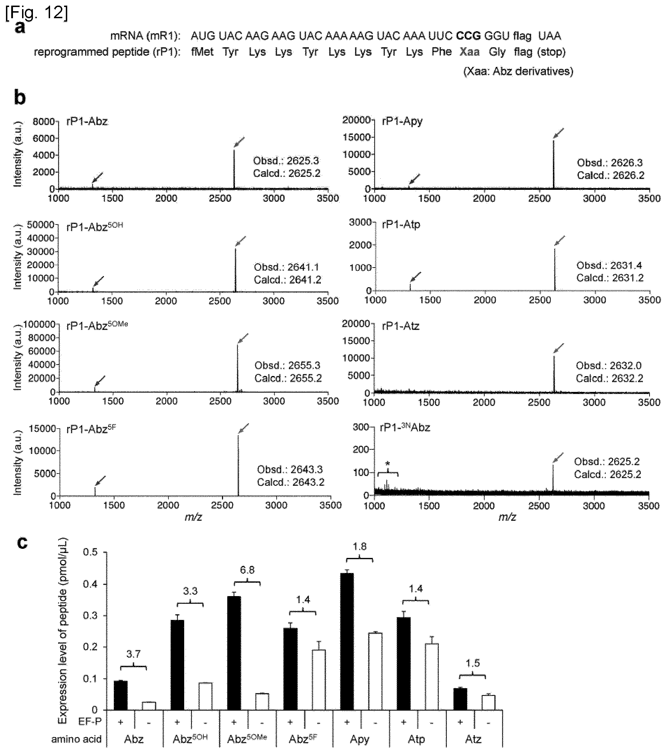

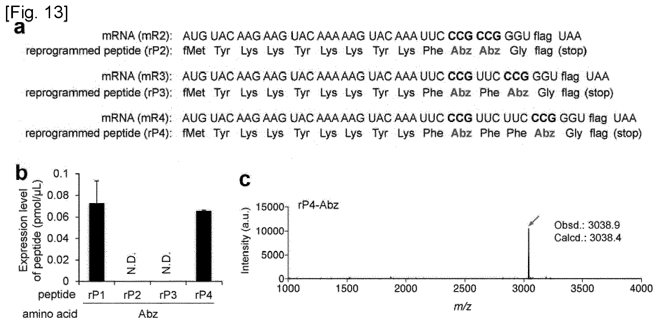

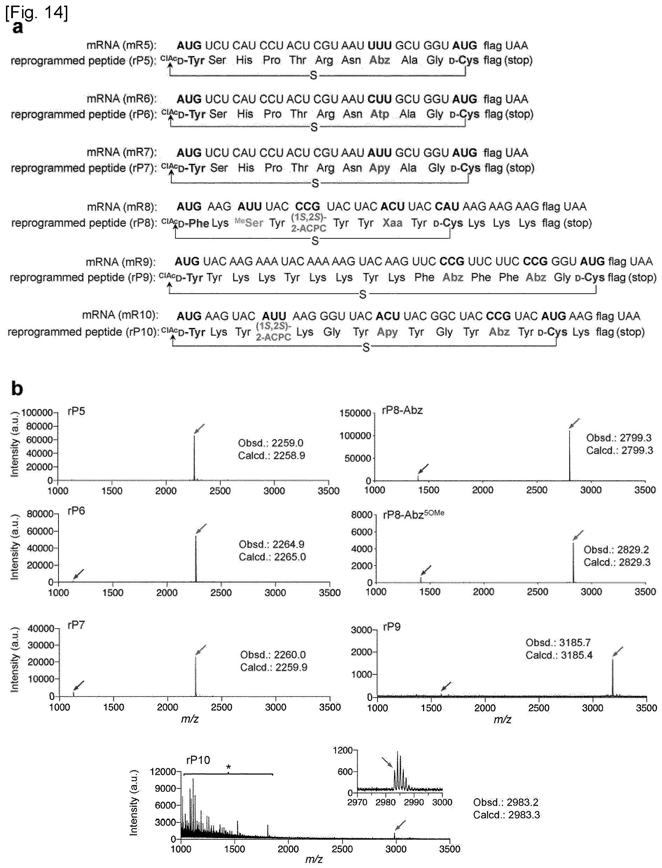

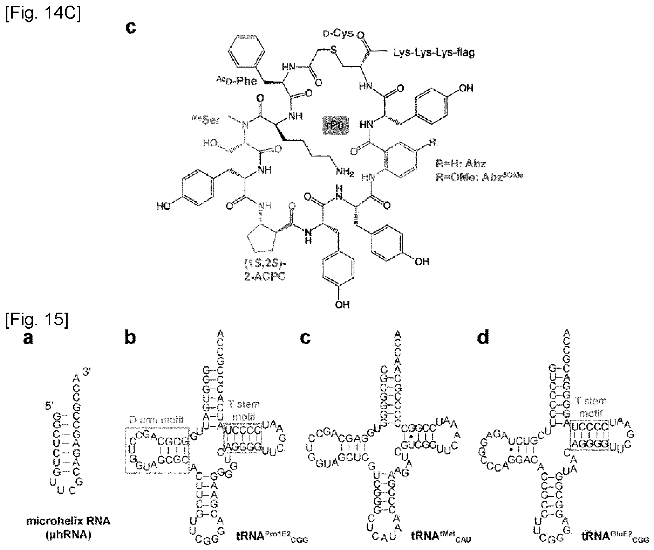

Fig. 12 shows the ribosomal incorporation of an Abz derivative into a peptide rP1. Fig. 12a shows the sequence of the peptide (rP1) corresponding to mRNA (mR1). The Abz derivative is assigned to a CCG codon by using a pre-charged aminoacyl-tRNA^{Pro1E2}_{CGG}. The flag is GACUACAAGGACGACGACGACAAG in mR1 and Asp-Tyr-Lys-Asp-Asp-Asp-Asp-Lys in rP1 (this also applies to Fig. 13 and Fig. 14). Fig. 12b shows the MALDI-TOF MS spectra of peptides containing an Abz derivative. The arrow, "Calcd.", "Obsd.", and * in the spectra have the same meanings as those in Fig. 4. Fig. 12c shows the expression level of the rP1 peptides estimated by autoradiography. The black bar indicates the translation performed in the presence of EF-P (EF-P(+)) and the white bar indicates the translation performed in the absence of EF-P (EF-P(-)). The numeral on the bar graph indicates a ratio (EF-P(+)/EF-P(-)) of the expression level in the presence of EF-P to that in the absence of EF-P.
Fig. 13 shows the ribosomal incorporation of a plurality of Abzs into peptides (rP2 to rP4). Fig. 13a shows the sequences of the peptides (rP2 to rP4) corresponding to mRNAs (mR2 to mR4). Fig. 13b shows the expression levels of the peptides (rP1 to rP4) estimated by autoradiography. The term "N.D." means "not detected". Fig. 13c shows the MALDI-TOF MS spectrum of the Abz-containing rP4 peptide. The arrow indicates a monovalent ion of a desired peptide. The terms "Calcd." and "Obsd." have the same meanings as those in Fig. 4.
Fig. 14 shows the ribosomal incorporation of an Abz derivative into cyclic peptides (rP5 to rP10). Fig. 14a shows the sequence of the cyclic peptides (rP5 to rP10) corresponding to mRNAs (mR5 to mR10). The thiol group of D-Cys forms a thioether bond with an N-terminal chloroacetyl group. Fig. 14b shows the MALDI-TOF MS spectra of the peptides (rP5 to rP10). The arrow, "Calcd.", "Obsd.", and * in the spectra have the same meanings as those in Fig. 4.
Fig. 14C shows the structure of rP8 peptide containing Abz or Abz^{5OMe}.
Fig. 15 shows the secondary structure of a µhRNA and tRNAs used in the incorporation of a cAA in Examples. Fig. 15a is the secondary structure of µhRNA. Fig. 15b is the secondary structure of tRNA^{Pro1E2}_{CGG}. The D-arm structure (D-arm motif) for EF-P binding consists of a D-loop of 9 bases closed by a stable 4 base pair stem. It also has a T-stem (T-stem motif) for EF-Tu binding. The sequence of an anticodon loop is changed as needed in order to decode another codon as described in Table 14. Fig. 15c shows tRNA^{fMet}_{CAU} used for the incorporation of ^{ClAc}D-Tyr and ^{ClAc}D-Phe by an initiation codon AUG. Fig. 15d shows tRNA^{GluE2}CGG used for the incorporation of (1S,2S)-2-ACPC by a CCG codon.

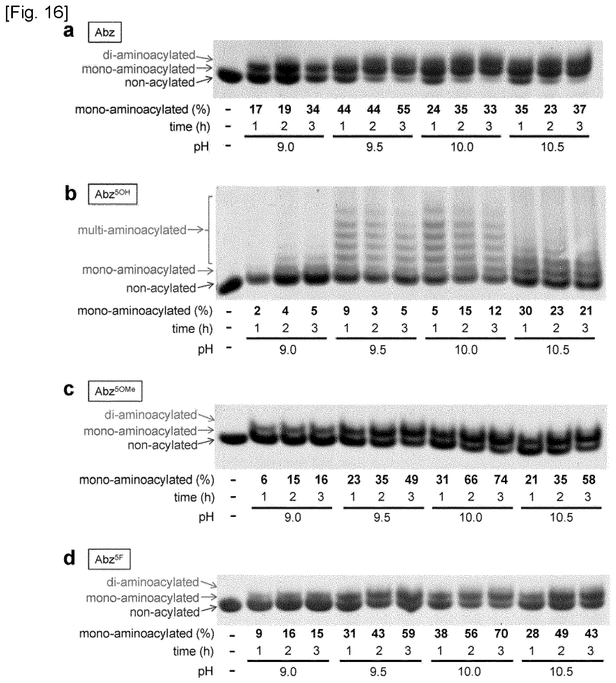

Figs. 16a, b, c, and d respectively show the analysis results of acid modification PAGE when µhRNA was aminoacylated using Abz, Abz^{5OH}, Abz^{5OMe}, and Abz^{5F} anhydrides. The bands were each detected by ethidium bromide staining. The center arrow indicates the position of desired mono-aminoacyl-µhRNA, while the upper and lower arrows respectively indicate the positions of a multiaminoacyl-µhRNA and a non-acylated µhRNA.

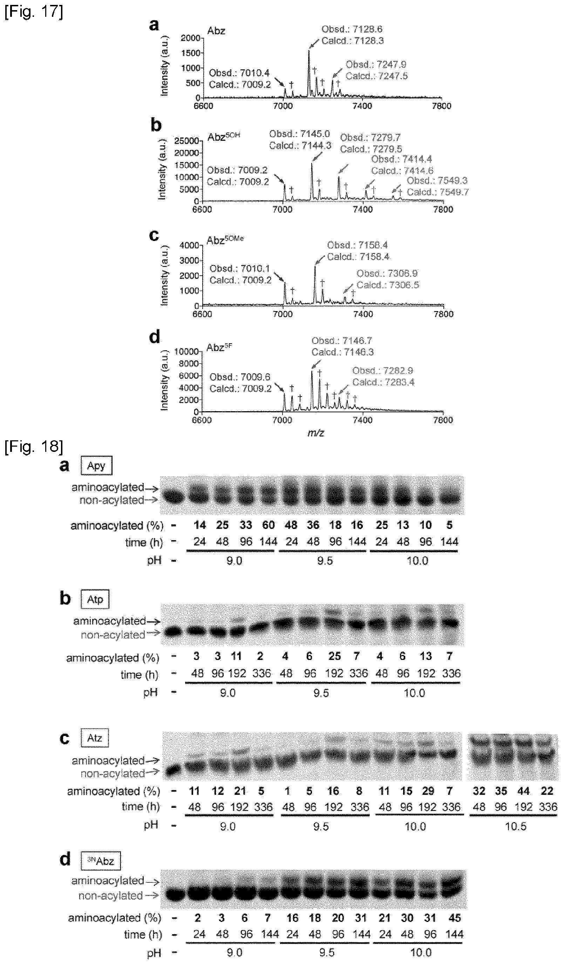

Figs. 17a, b, c, and d respectively show the MALDI-TOF MS spectra of aminoacyl-µhRNAs obtained by the aminoacylation using Abz, Abz^{5OH}, Abz^{5OMe}, and Abz^{5F}. The arrow indicating the largest peak which is the second from the left is a peak of a desired monoaminoacyl-µhRNA. The arrow on a higher molecular weight side (higher m/z side) indicates a multiaminoacyl-µhRNA, while the arrow on a lower molecular weight side (lower m/z side) indicates a non-acylated µhRNA. The terms "Calcd." and "Obsd." have the same meanings as those in Fig. 4, while † indicates a potassium adduct.
Figs. 18a, b, c, and d respectively show the analysis results of acid modification PAGE when µhRNA was aminoacylated using cyanomethyl esters (CME) of Apy, Atp, Atz, and ^{3N}Abz, and flexizyme (eFx). The upper and lower arrows respectively show the positions of a desired aminoacyl-µhRNA and a non-acylated µhRNA.

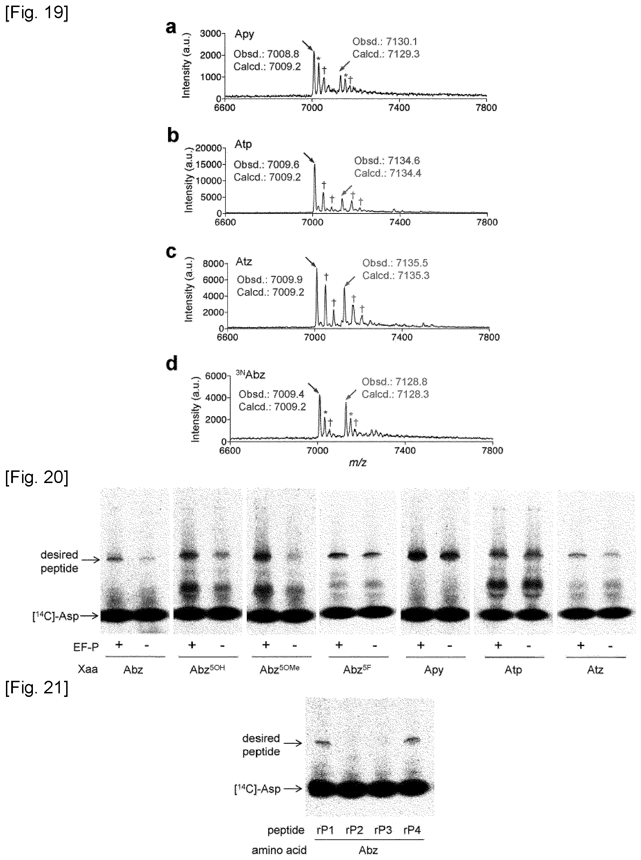

Figs. 19a, b, c, and d respectively show the MALDI-TOF MS spectra of aminoacyl-µhRNAs obtained by the aminoacylation using Apy, Atp, Atz, and ^{3N}Abz. The arrow on the high molecular weight side (high m/z side) indicates a desired monoacyl-µhRNA and the arrow on the low molecular weight side (low m/z side) indicates a non-acylated µhRNA. The terms "Calcd." and "Obsd." have the same meanings as those in Fig. 4 and * and † indicate a sodium adduct and a potassium adduct, respectively.
Fig. 20 shows the tricine SDS-PAGE analysis results of Abz derivative-containing rP1 peptides in Fig. 12c.
Fig. 21 shows the tricine SDS-PAGE analysis results of Abz-containing peptides (rP1 to rP4) in Fig. 13b.

### Description of Embodiments

Embodiments of the present invention will hereinafter be described in detail. The present invention is not limited by the following embodiments, but can be performed by modifying in various ways without departing from the gist of the invention.

### <Method of producing a library>

The present invention relates to a method of producing a library including two or more cyclic peptides.

At least one of the cyclic peptides included in the library of the present invention is a cyclic peptide having a cyclic structure composed of 4 to 30 amino acids or derivatives thereof and containing at least one selected from cyclic β-, γ-, and δ-amino acids (cAAs) in the cyclic structure.

One of the methods of producing a library according to the present invention includes a step of preparing an mRNA library encoding a peptide having a sequence represented by the following formula (1);

-(Xaa)ₙ₁- (1)

[in the formula (1),
Xaas are each an arbitrary amino acid or derivative thereof,
at least one of Xaas is one selected from cyclic β-, γ-, and δ-amino acids (cAAs), and
n1 is an integer of 2 to 28]; and
a step of using the mRNA library to express a peptide in a cell-free translation system and produce the library.

Another one of the methods of producing a library according to the present invention includes:
a step of preparing an mRNA library encoding a peptide having a sequence represented by the following formula (1);

-(Xaa)ₙ₁- (1)

[in the formula (1),
Xaas are each an arbitrary amino acid or derivative thereof,
at least one of Xaas is one selected from cyclic β-, γ-, and δ-amino acids (cAAs), and
n1 is an integer of 2 to 28];
a step of binding puromycin to the 3' end of each of mRNAs of the mRNA library to produce a puromycin-bound mRNA library; and
a step of using the puromycin-bound mRNA library to express the precursor peptide in a cell-free translation system and produce a peptide-mRNA complex library.

When a peptide chain has a β-amino acid residue or γ-amino acid residue therein, their stable helical propensities make it possible to form unique rigid folding structures. Further, a β-amino acid can induce a turn structure of a peptide such as γ-turn or β-turn. In particular, cyclic β- and γ-amino acids (may also be called cβAA and cyAA) act as a particularly strong helix/turn inducer. If cyclic β- or γ-amino acid is introduced into a cyclic peptide, there is a possibility of causing the peptide to express rigidity. By the method of producing a library according to the present invention, a cyclic peptide containing a cyclic amino acid such as cyclic β- or γ-amino acid can be obtained.

The present inventors have newly found that ribosomal synthesis can be applied even to a cyclic amino acid which is a non-natural β- or γ-amino acid and at the same time, has a bulky and rigid structure.

The term "cyclic peptide" as used herein means a peptide at least having, in the molecule thereof, a cyclic structure composed of four or more amino acids. The cyclic peptide may have, as its molecular structure, a chain structure in which amino acids are linked to each other via a peptide bond, as well as the cyclic structure. It may also have a structure other than a peptide structure.

The term "cyclic structure" as used herein means a closed ring structure of a linear peptide formed in the molecule by two amino acids separated by 2 or more amino acid residues.

The term "separated by 2 or more amino acid residues" as used herein means that at least 2 amino acid residues are present between the two amino acids.

The term "library" as used herein means a group of cyclic peptides including two or more cyclic peptides.

The cyclic peptide in the present invention contains at least one of cAAs, more specifically, cyclic β-amino acids (cβAAs), cyclic γ-amino acids (cyAAs), and cyclic δ-amino acids (cδAAs). The cyclic peptide in the present invention may further contain, in addition to a natural amino acid (which may also be called "amino acid", simply), an artificial amino acid variant and/or derivative thereof (which may also be called "derivative of an amino acid").

Examples of the amino acid constituting the cyclic peptide in the present invention include natural proteinogenic L-amino acids, non-natural amino acids, and chemically synthesized compounds having properties which are characteristics of an amino acid and known in the art.

The proteinogenic amino acids are, when expressed by a three-letter code known in the art, Arg, His, Lys, Asp, Glu, Ser, Thr, Asn, Gln, Cys, Gly, Pro, Ala, Ile, Leu, Met, Phe, Trp, Tyr, and Val. The proteinogenic amino acids are, when expressed by a one-letter code known in the art, R, H, K, D, E, S, T, N, Q, C, G, P, A, I, L, M, F, W, Y, and V.

The term "non-proteinogenic amino acids" means natural or non-natural amino acids other than the proteinogenic amino acids.

Examples of the non-natural amino acids include α,α-disubstituted amino acids (such as α-methylalanine), N-alkylamino acids, D-amino acids, β-amino acids (β-amino acids here are amino acids other than cβAA), and α-hydroxy acids, each having a main chain structure different from that of natural amino acids; amino acids (such as norleucine and homohistidine) having a side-chain structure different from that of natural amino acids; amino acids (such as "homo"amino acids, homophenylalanine, and homohistidine) having extra methylene in the side chain thereof; and amino acids (such as cysteic acid) obtained by substituting a carboxylic acid functional group in the side chain thereof by a sulfonic acid group. Specific examples of the non-natural amino acid include amino acids described in WO2015/030014.

Preferred examples of derivatives of amino acids in the present invention include N-alkyl-α-amino acids, which are amino acids having an alkyl group bound to the amino group at α-position.

The number of the amino acid residues forming the cyclic structure is not particularly limited as long as it is 4 or more and the number may be, for example, 5 or more, 6 or more, 7 or more, 8 or more, 9 or more, 10 or more, 11 or more, or 12 or more. The number of the amino acid residues forming the cyclic structure is not particularly limited as long as it is 30 or less and the number may be 25 or less, 20 or less, 18 or less, 17 or less, 16 or less, or 15 or less.

The number of amino acids forming the cyclic structure is usually 5 or more and 30 or less. Within a range of 5 or more and 30 or less, the number of amino acids forming the cyclic structure may be limited to 6 or more, 8 or more, 10 or more, 11 or more, or 12 or more and 30 or less, 25 or less, 20 or less, 18 or less, 17 or less, or 15 or less. By adjusting the number of amino acids forming the cyclic structure to a range of 5 or more and 30 or less, the diversity of the cyclic peptides constituting a library tends to be secured.

The number of amino acids forming the cyclic structure may be 8 or more and 20 or less, 8 or more and 17 or less, 9 or more and 17 or less, 10 or more and 15 or less, or 10 or more and 13 or less.

The number of amino acids forming the cyclic structure is preferably 9 or more and 25 or less, more preferably 10 or more and 20 or less, still more preferably 10 or more and 18 or less from the standpoint of functionality such as expression of a folded structure of the cyclic peptide.

The number of amino acids forming the cyclic structure may be 11 or more and 17 or less, 12 or more and 18 or less, 12 or more and 18 or less, or 11 or more and 16 or less.

In the present invention, the cyclic peptide may be a modified one such as phosphorylated, methylated, acetylated, adenylated, ADP-ribosylated, glycosylated, or polyethylene glycol-added one or it may be a fused one with another peptide and/or protein. Further, the cyclic peptide may be a biotinylated or labeled one via an appropriate linker.

In the present invention, the cyclic peptide may also be a dimer having in the molecule thereof two cyclic structures and obtained by binding two cyclic peptides, each having one cyclic structure, to each other via a linker structure or it may have an intramolecular lactam bridge structure obtained by intramolecularly forming a lactam structure.

The linker structure that links two cyclic peptides to each other is not particularly limited and a structure known in a peptide synthesis field as a linker that links peptides to each other may be used.

The intramolecular lactam bridge structure may be formed by binding the respective side chains of amino acids constituting the cyclic peptide. For example, an intramolecular lactam structure is formed by binding the side chain amino group of Lys to the side-chain carboxyl group of Asp or Glu to form a peptide bond and thereby form an intramolecular lactam structure. The cyclic peptide has, in the molecule thereof, another cyclic structure as a bridge structure. Instead of Lys, for example, DAP, DAB, and Orn may be bound to Asp or Glu.

The cAA in the present invention may be represented, for example, by the following formula (I).

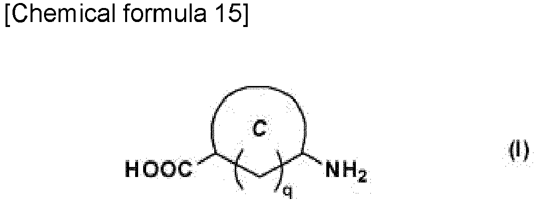

In the formula (I), the ring C may be a saturated or unsaturated alicyclic hydrocarbon ring or a heterocyclic ring. The ring C may also be an aromatic ring. Further, the ring C may have a crosslinked structure.

In the formula, q is any integer of 0, 1, or 2. When q is 0, the carbon to which the carboxylic acid group is bound and the carbon to which the amino group is bound form a single bond.

In order to perform smooth introduction into a cyclic peptide by ribosomal synthesis, the ring C is preferably a saturated or unsaturated alicyclic hydrocarbon ring or an aromatic ring, more preferably a saturated or unsaturated alicyclic hydrocarbon ring.

The cβAA in the present invention is preferably represented by the following formula (I-1).

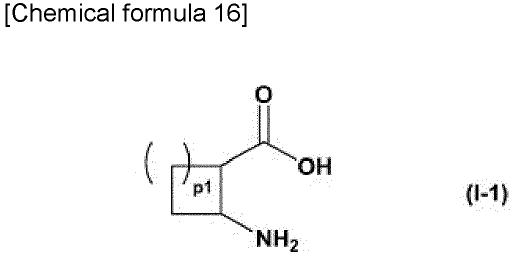

In the formula (I-1), p1 is any integer of 1 to 4, preferably any integer of 1 to 3.

In the present invention, cγAA is preferably represented by the following formula (I-2).

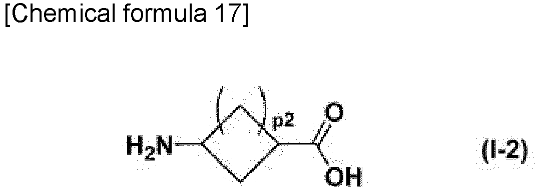

In the formula (I-2), p2 is any integer of 1 to 4, preferably any integer of 1 to 3.

The cδAA in the present invention is preferably represented by the following formula (I-3).

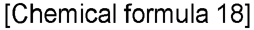

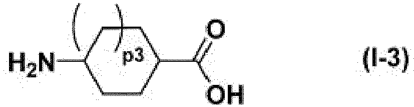

In the formula (I-3), p3 is an integer of 1 or 2, preferably 1.

The cAA such as cβAA, cγAA, or cδAA in the present invention is preferably represented by the following formula (I-4).

H₂N-Ar-COOH (I-4)

In the formula (I-4), Ar is a divalent aromatic group and the aromatic ring in the aromatic group may be a substituted one with one or more substituents.

Here, the term "Ar is a divalent aromatic group" means that the ring C is an aromatic ring and one of the two binding hands of the aromatic ring binds to an amino group (NH₂) and the other one of the two binding hands binds to a carboxyl group (COOH).

The two binding hands of the aromatic ring bind to at least to NH₂ and COOH, but the remaining binding hands may bind to a hydrogen atom or may bind to one or more substituents.

The aromatic ring may be benzene, naphthalene, or the like, or may be an aromatic heterocyclic ring composed of one or more nitrogen atoms, oxygen atoms, and/or sulfur atoms and carbon atoms.

When the aromatic ring is an aromatic heterocyclic ring, it may be a 5 or 6-membered aromatic heterocyclic compound containing one or more nitrogen atoms, oxygen atoms, and/or sulfur atoms, such as pyridine, pyrimidine, imidazole, oxazole, thiazole, furan, pyran, and thiophene. Alternatively, it may be a fused ring compound such as indole, quinoline, isoquinoline, benzothiophene, or benzofuran.

When the aromatic ring has one or more substituents, the number of the substituents may be 1, 2, 3, or 4, may be 1 to 3, may be 1 or 2, or may be 1.

The substituent is not particularly limited, and examples thereof include linear or branched alkyl groups, alkenyl groups or alkynyl groups, each having 1 to 6 carbon atoms, a hydroxyl group, an amino group, a halogen (fluorine, chlorine, bromine, or iodine), and linear or branched alkoxy groups, alkenyl groups, or alkynyl groups, each having 1 to 6 carbon atoms.

The aromatic ring is not particularly limited and it may be a 5-membered or 6-membered ring.

When the aromatic ring is a 6-membered ring, that having NH₂ and COOH at the ortho position is cβAA, that having them at the meta position is cyAA, and that having them at the para position is cδAA. In other words, when the aromatic ring is a 6-membered ring, that having NH₂ and COOH at position 1 and position 2, it is cβAA, that having them at position 1 and position 3 is cyAA, and that having them at position 1 and position 4 is cδAA.

When the aromatic ring is a 5-membered ring, that having NH₂ and COOH at position 1 and position 2, it is cβAA and that having them at position 1 and position 3 is cγAA.

The ribosomal incorporation of 2-aminobenzoic acid (Abz) which is a kind of an aromatic amino acid has been achieved only at the N terminal of a peptide, but the present invention makes it possible to achieve ribosomal elongation even if an aromatic amino acid is incorporated in a non-N-terminal site. In other words, the present invention makes it possible to incorporate, as a structural unit during the elongation, an aromatic amino acid into a peptide chain.

The cyclic peptide in the production method according to the present invention contains a sequence represented by the following formula (1);

-(Xaa)ₙ₁- (1).

In the formula (1),
Xaas are each an arbitrary amino acid or derivative thereof,
at least one of Xaas is one selected from cyclic β-, γ-, and δ-amino acids (cAAs), and
n1 is an integer of 2 to 28.

The cAA in the present invention is more preferably selected from the following structures.

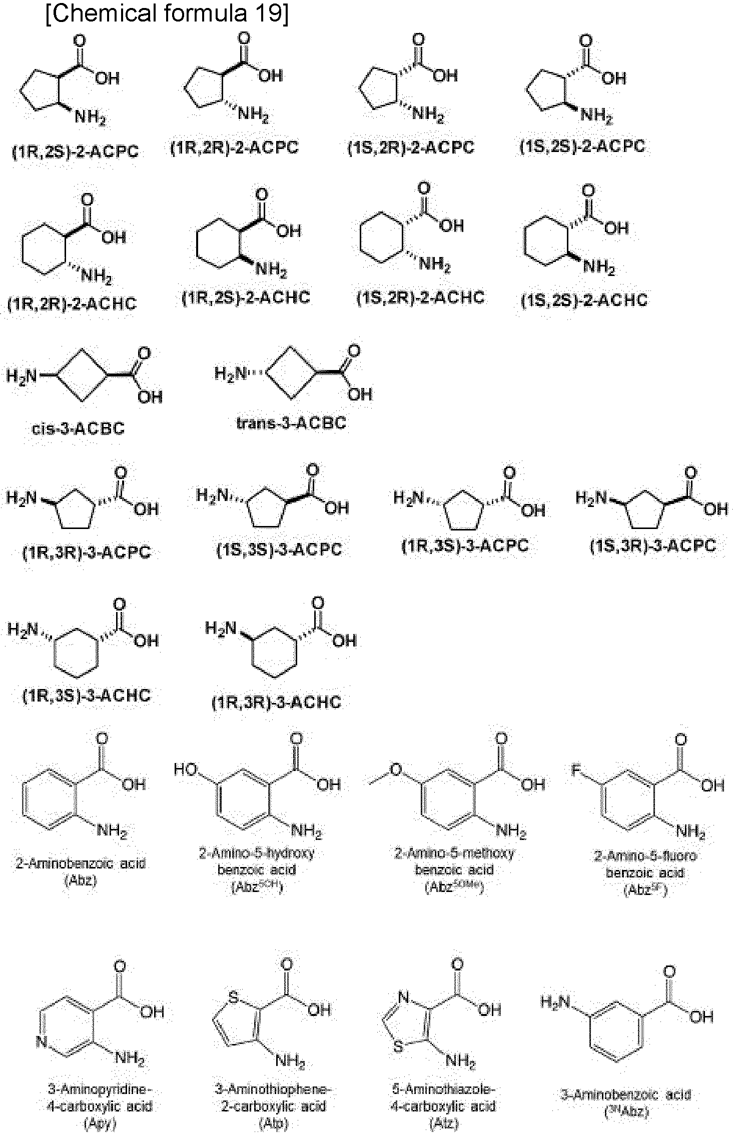

Although the closed ring structure of the cyclic peptide in the present invention is not particularly limited, it is formed by the covalent bonding of two amino acids.

Examples of the covalent bonding between two amino acids include disulfide bonding, peptide bonding, alkyl bonding, alkenyl bonding, ester bonding, thioester bonding, ether bonding, thioether bonding, phosphonate ether bonding, azo bonding, C-S-C bonding, C-N-C bonding, C=N-C bonding, amide bonding, lactam bridging, carbamoyl bonding, urea bonding, thiourea bonding, amine bonding, and thioamide bonding.

When two amino acids bind to each other in their main chain, a closed ring structure is formed by peptide bonding. A covalent bond between two amino acids may be formed by bonding between the respective side chains of the two amino acids, bonding between the side chain and the main chain of them, or the like.

The cyclic structure is not limited to that formed by bonding between the N-terminal and C-terminal amino acids of a linear peptide, but it may be formed by bonding between a terminal amino acid and a non-terminal amino acid or bonding between non-terminal amino acids. When one of the amino acids bound for the formation of a cyclic structure is a terminal amino acid and the other one is a non-terminal amino acid, the resulting cyclic peptide has a cyclic structure having a linear peptide attached thereto like a tail.

As the closed ring structure, a peptide translationally synthesized can be cyclized by a spontaneous reaction, for example, by incorporating an amino acid having the following Functional group 1 and Functional group 2 corresponding thereto as ring-forming amino acids. Either Functional group 1 or Functional group 2 may be placed on the N terminal side or they may be placed at the N terminal and C terminal, respectively. As to the position of Functional group 1 and Functional group 2, one of them may be placed as a terminal amino acid and the other one may be placed as a non-terminal amino acid, or both may be placed as a non-terminal amino acid.

**[Table 1]**

| | Functional group1 | Functional group2 |
|---|---|---|
| (A) | 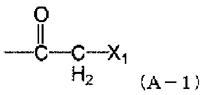 | HS- (A-2) |
| (B) | -C≡C-H (B-1) | N₃- (B-2) |
| (C) | -Ar-CH₂NH₂ (C-1) | 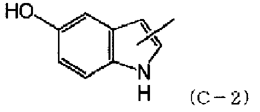 |
| (D) | -C**≡**C-CH₂-X₁ (D-1) | HS- (D-2) |
| (E) | -Ar-CH₂-X₁ (E-1) | HS- (E-2) |

The cyclic peptide in the present invention is preferably represented by the following formula (2). In the production method according to the present invention, it is therefore preferred to use an mRNA library encoding the peptide represented by the formula (2).

Xaa1-(Xaa)ₙ₁-Xaa2-(Xaaₓ)ₘ (2)

In the formula (2),
Xaas are each an arbitrary amino acid or derivative thereof,
at least one of Xaas is one selected from cyclic β-, γ-, and δ-amino acids (cAAs),
Xaa1 and Xaa2 are each an amino acid or derivative thereof which forms the ring of a cyclic peptide,
Xaaₓ is an arbitrary amino acid or derivative thereof,
n1 is an integer of 2 to 28, and
m is an integer of 0 to 10.

Between Xaa1 and Xaa2, it is preferred to form the aforesaid covalent bond such as disulfide bond, peptide bond, alkyl bond, alkenyl bond, ester bond, thioester bond, ether bond, thioether bond, phosphonate ester bond, azo bond, C-S-C bond, C-N-C bond, C=N-C bond, amide bond, lactam bridge, carbamoyl bond, urea bond, thiourea bond, amine bond, and thioamide bond. Of these bonds, thioether bond is more preferred.

When a thioether bond is formed between Xaa1 and Xaa2, Xaa1 is preferably N-chloroacetylphenylalanine (^{ClAc}Phe) or N-chloroacetyltyrosine (^{ClAc}Tyr), more preferably N-chloroacetyl-D-phenylalanine (^{ClAc}D-Phe) or N-chloroacetyl-D-tyrosine (^{ClAc}D-Tyr) and Xaa2 is preferably cysteine, more preferably D-cysteine (D-Cys).

When a thioether bond is formed between Xaa1 and Xaa2, the cyclic peptide in the present invention is preferably represented by the following formula (2').

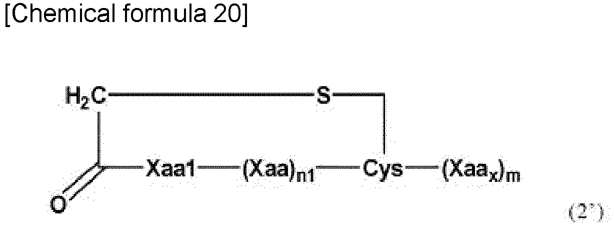

In the formula (2'),
Xaas are each an arbitrary amino acid or derivative thereof and at least one of Xaas is one selected from cyclic β-, γ-, and δ-amino acids (cAAs),
Xaa1 is preferably phenylalanine or tyrosine,
Xaaₓ is an arbitrary amino acid or derivative thereof,
n1 is an integer of 2 to 28, and
m is an integer of 0 to 10.

In the production method of the present invention, the peptide having a sequence represented by the formula (1) is expressed from an mRNA library encoding peptides having the sequence represented by the formula (1) by using a cell-free translation system. The mRNA library may be obtained from a commercially available product or may be prepared. For example, when the mRNA library is prepared, it may be prepared by obtaining a DNA library in accordance with the method described in Chemistry & Biology 18, 1562-1570 (2011) and/or Chemistry & Biology 21, 766-774 (2014) and transcribing the DNA library in vitro.

The mRNA library includes an mRNA having a plurality of N1N2N3s as a codon.

The term "N1N2N3" as used herein means a codon designating an arbitrary amino acid and for example, N1, N2, and N3 are independently selected from adenine (A), guanine (G), cytosine (C), and uracil (U). One mRNA contains a plurality of N1N2N3s and N1, N2, and N3 are each independently selected from them. For example, when mRNA contains -N1N2N3-N1N2N3-, therefore, these two N1s, N2s, or N3s may be the same or different from each other.

In the present invention, since the mRNA library encoding the peptide having the sequence represented by the formula (1): -(Xaa)ₙ₁- is used, it preferably contains at least an mRNA represented by -(N1N2N3)ₙ₁-.

In the present invention, an arbitrary amino acid is reassigned to N1N2N3, but a cAA is reassigned to at least one N1N2N3. In the reassignment, an amino acid different from an amino acid having a codon-amino acid relation in a natural genetic code table may be assigned or an amino acid having the same relation may be assigned. The term "natural genetic code table" as used herein means a table showing amino acids represented by genetic codes composed of an mRNA triplet, respectively, in a living body. In the natural genetic code table, N1N2N3 represents the following amino acids.

**[Table 2]**

| | U | C | A | G | |
|---|---|---|---|---|---|
| U | Phe | Ser | Tyr | Cys | U |
| | Phe | Ser | Tyr | Cys | C |
| | Leu | Ser | STOP | STOP | A |
| | Leu | Ser | STOP | Trp | G |
| C | Leu | Pro | His | Arg | U |
| | Leu | Pro | His | Arg | C |
| | Leu | Pro | Gln | Arg | A |
| | Leu | Pro | Gln | Arg | G |
| A | Ile | Thr | Asn | Ser | U |
| | Ile | Thr | Asn | Ser | C |
| | Ile | Thr | Lys | Arg | A |
| | Met | Thr | Lys | Arg | G |
| G | Val | Ala | Asp | Gly | U |
| | Val | Ala | Asp | Gly | C |
| | Val | Ala | Glu | Gly | A |
| | Val | Ala | Glu | Gly | G |

The mRNA library may include an mRNA containing, as a plurality of N1N2N3s, for example, any of a plurality of N1N2Ks, a plurality of N1N2Ss, a plurality of N1N2Ms, a plurality of N1N2Ws, a plurality of N1N2As, a plurality of N1N2Us, a plurality of N1N2Cs, and a plurality of N1N2Gs. The respective terms "N1" and "N2" as used herein have the same meanings as described above and Ks are independently either of uracil (U) or guanine (G), Ss are independently either of cytosine (C) or guanine (G), Ms are independently either of adenine (A) or cytosine (C), and Ws are independently either of adenine (A) or uracil (U).

For convenience' sake, the present invention will hereinafter be described using, as an example, the case where the mRNA library includes an mRNA containing a plurality of N1N2Ks, that is, the case where the mRNA library includes an mRNA containing, as N1N2N3, a plurality of N1N2Ks as a codon. Even if another mRNA library is used, it is possible to carry out the invention similarly insofar as a peptide contained in the translated peptide library is prenylated. In the natural genetic code table, N1N2K corresponds to 20 amino acids when the code in the right column in the above table is G or U.

In the present specification, for example, Leu is assigned to UUG as shown in the natural genetic code table or an amino acid other than Leu may be assigned by the reassignment of a codon. Any amino acid may be assigned to a "N1N2K" codon. The term "assigning an amino acid to a codon" means that the genetic code table is rewritten so that a certain codon encodes the amino acid. The term "assigning an amino acid to a codon" as used herein has the same meaning as "reassigning a codon".

Assignment, to each codon, of an amino acid different from that in the natural genetic code table is achieved, for example, by codon reassignment making use of artificial aminoacylated RNA catalyst flexizyme (Flexizyme). By Flexizyme, a desired amino acid can be bound to a tRNA having an arbitrary anticodon, so that an arbitrary amino acid can be assigned to an arbitrary codon. Flexizyme will be described later. In the present specification, "binding an amino acid to tRNA" may also be expressed as "charging tRNA with an amino acid", "aminoacylating tRNA", or "acylating tRNA with an amino acid".

In the present invention, cAA is assigned to "N1N2K" or a nonproteinogenic amino acid other than a cAA may be assigned to it. When mRNA contains two or more "N1N2Ks", all of them may be assigned to a cAA or nonproteinogenic amino acid or some of them may be assigned to a cAA or nonproteinogenic amino acid.

The term "cell-free translation system" as used herein means a translation system not containing cells. More specifically, it is a system of synthesizing an intended peptide or protein in a test tube by making use of the synthesizing function of a protein extracted from cells. As the cell-free translation system, for example, an Escherichia coli extract, a wheat germ extract, a rabbit reticulocyte extract, or an insect cell extract may be used. Alternatively, a reconstituted cell-free translation system may be used, which is obtained by reconstituting a purified ribosome protein, aminoacyl-tRNA synthase (aaRS), ribosomal RNA, amino acid, rRNA, GTP, ATP, translation initiation factor (IF), elongation factor (EF), release factor (RF), and ribosome recycling factor (RRF), and another factor necessary for translation.

The system may contain RNA polymerase for simultaneously performing transcription from DNA. Examples of a commercially available cell-free translation system include Escherichia-coli derived systems such as "RTS-100" (trade mark) of Roche Diagnostics, reconstituted translation systems such as "PURESYSTEM" (trade mark) of PGI and "PURExpressR In Vitro Protein Synthesis Kit" of New England BioLabs, and systems using a wheat germ extract such as those of ZOEGENE Corporation or CellFree Sciences.

As a system using a ribosome of Escherichia coli, for example, a technology described in the following documents is known: H. F. Kung et al., 1977. The Journal of Biological Chemistry Vol. 252, No. 19, 6889-6894; M. C. Gonza et al., 1985, Proceeding of National Academy of Sciences of the United States of America Vol. 82, 1648-1652; M. Y. Pavlov and M. Ehrenberg, 1996, Archives of Biochemistry and Biophysics Vol. 328, No. 1, 9-16; Y. Shimizu et al., 2001, Nature Biotechnology Vol. 19, No. 8, 751-755; and H. Ohashi et al., 2007, Biochemical and Biophysical Research Communications Vol. 352, No. 1, 270-276. By using the cell-free translation system, a high-purity expression product can be obtained without purifying. The cell-free translation system of the present invention may be used not only for translation, but also for transcription after adding a factor necessary for transcription.

In the present invention, peptide expression by the cell-free translation system may be performed using Flexible In vitro Translation system (FIT system), for example, in accordance with the method described in Goto, Y., Katoh, T. & Suga, H. Flexizymes for genetic code reprogramming. Nat Protoc 6, 779-790, (2011).

The cell-free translation system in the present invention preferably contains a tRNA charged with a cAA to obtain a cyclic peptide containing a cAA.

The present inventors studied a method of introducing a cAA into a cyclic peptide by ribosomal synthesis. As a result, it has been found that a cyclic peptide containing a cAA can be obtained efficiently by selecting a tRNA to be charged, depending on the kind of a cAA, and using the tRNA charged with the cAA.

As a tRNA to be charged with a cAA, a tRNA having a D-arm structure interactive with EF-P, which is a translation factor, or a tRNA not having a D-arm structure interactive with EF-P can be used. As a tRNA to be charged with a cAA, either of the aforesaid tRNAs may be used but it may be selected as needed depending on the kind of a cAA. The cell-free translation system in the present invention therefore preferably contains a tRNA charged with one selected from cyclic β-, γ-, and δ-amino acids (cAAs) and having a D-arm structure interactive with EF-P or a tRNA similarly charged and not having a D-arm structure interactive with EF-P.

As the tRNA having a D-arm structure interactive with EF-P, for example, the tRNA described in WO2019/077887 may be used.

Specific examples of the tRNA having a D-arm structure interactive with EF-P include a tRNA having a base sequence of N₁N₂GCN₃N₄N₅N₆N₇N₈N₉N₁₀N₁₁GCN₁₂N₁₃ (SEQ ID NO: 1), in which N1 to N13 are each an arbitrary base, N₃ to N₁₁ form a D-loop, and N₁N₂GC forms a base pair with N13N12CG. Any base can be selected insofar as N₁N₂ and N₁₂N₁₃ form a base pair, but it is preferred that N₁ is G and N₁₃ is C.

In addition, it is preferred that N₂ is C and N₁₂ is G.

The base sequence represented by SEQ ID NO: 1 is a D-arm comprised of a D-Ioop and a D-stem and the introduction of the base sequence represented by SEQ ID NO: 1 into a tRNA tends to promote peptidyl transfer by EF-P peptide.

Another structure of the tRNA having the base sequence represented by SEQ ID NO: 1, that is, the base sequence of an acceptor stem, an anticodon stem, an anticodon loop, a variable loop, or a T-arm is arbitrary. The anticodon loop may have a base sequence corresponding to a codon to which a cAA is assigned. The 3' end of the tRNA has a CCA sequence and binds to a cAA.

The base sequence represented by SEQ ID NO: 1 is preferably a base sequence represented by SEQ ID NO: 3.
GCGCN₃N₄N₅N₆N₇N₈N₉N₁₀N₁₁GCGC (SEQ ID NO: 3)

In SEQ ID NO: 3, N₃ to N₁₁ have the same meanings as described above and GCGC forms a base pair with CGCG.

The base sequence of N₃ to N₁₁ in SEQ ID NO: 1 and SEQ ID NO: 3 is preferably a base sequence represented by SEQ ID NO: 4.
AGCCUGGUA (SEQ ID NO: 4)

The base sequence represented by SEQ ID NO: 4 forms a D-Ioop in the tRNA.

In SEQ ID NO: 4, one or a plurality of bases may be substituted.

The term "a plurality of bases may be substituted" means that in 9 bases forming a D-loop, 2, 3, 4, 5, 6, 7, 8, or 9 bases may be substituted, 1 to 4 bases may be substituted, 1 to 3 bases may be substituted, 1 to 2 bases may be substituted, or 1 base may be substituted.

The base sequence represented by SEQ ID NO: 1 is preferably a base sequence represented by SEQ ID NO: 5 and is preferably a base sequence represented by SEQ ID NO: 6.
N₁N₂GCGCAGCCUGGUAGCGCN₁₂N₁₃(SEQ ID NO: 5)

In SEQ ID NO: 5, N₁, N₂, N₁₂, and N₁₃ have the same meanings as described above and N₁N₂GC forms a base pair with N13N12CG.
GCGCGCAGCCUGGUAGCGCGC (SEQ ID NO: 6)

In SEQ ID NO: 5 and SEQ ID NO: 6, one or a plurality of bases may be substituted. The term "in SEQ ID NO: 5 and SEQ ID NO: 6, a plurality of bases may be substituted" means that in SEQ ID NO: 4 which indicates 9 bases forming a D-loop, 2, 3, 4, 5, 6, 7, 8, or 9 bases may be substituted, 1 to 4 bases may be substituted, 1 to 3 bases may be substituted, 1 to 2 bases may be substituted, or 1 base may be substituted.

The tRNA having a D-arm structure interactive with EF-P and the tRNA not having a D-arm structure interactive with EF-P may both have a base sequence represented by SEQ ID NO: 2.
AGGGG(N₁₄)ₘCCCCU (SEQ ID NO: 2)

In SEQ ID NO: 2, N₁₄ is an arbitrary base, m is an integer of 1 or more, (N₁₄)ₘ forms a T-loop, and AGGGG forms a base pair with UCCCC.

The T-stem of the aminoacyl-tRNA controls the interaction with EF-Tu protein and reinforces the incorporation of a nonproteinogenic amino acid. The base sequence represented by SEQ ID NO: 2 is a T-arm comprised of a T-loop and a T-stem and introduction of the base sequence represented by SEQ ID NO: 2 into a tRNA tends to promote accommodation by EF-Tu protein in introducing a cAA into a cyclic peptide.

Although m is not particularly limited insofar as (N₁₄)ₘ forms a T-loop, it may be an integer of 2, 3, 4, 5, 6, 7, 8, 9, or 10, preferably an integer of 6 to 8.

(N₁₄)ₘ is preferably a base sequence derived from the T-loop of tRNA^{GluE2} and represented by SEQ ID NO: 7.
UUCGAAU (SEQ ID NO: 7)

In SEQ ID NO: 7, 1 or a plurality of bases may be substituted. The term "a plurality of bases may be substituted" means that in 7 bases forming a T-loop, 2, 3, 4, 5, 6, or 7 bases may be substituted, 1 to 3 bases may be substituted, 1 to 2 bases may be sub substituted, or 1 base may be substituted.

In SEQ ID NO: 2, AGGGG and UCCCC forming a base pair form a T-stem. In the base sequence forming the T-stem, one or a plurality of bases may be substituted insofar as they form a base pair.

The term "a plurality of bases may be substituted" in the T-stem of SEQ ID NO: 2 means that in 10 bases forming the T-stem, 2, 3, 4, 5, 6, 7, 8, 9, or 10 bases may be substituted, 2 bases may be substituted, 4 bases may be substituted, or 6 bases may be substituted. Insofar as they form a base pair, 1, 3, or 5 bases may be substituted.

When the tRNA having a D-arm structure interactive with EF-P is used in the present invention, the tRNA preferably contains both the base sequence represented by SEQ ID NO: 1 and the base sequence represented by SEQ ID NO: 2 in order to allow an EF-P protein to exert a peptidyl transfer promotion effect and to allow an EF-Tu protein to exert an accommodation promotion effect.

The tRNA having a D-arm structure interactive with EF-P is preferably a chimera of tRNA^{Pro1} and tRNA^{GluE2} and such a tRNA will hereinafter be called "tRNA^{Pro1E2}".

More specifically, the tRNA having a D-arm structure interactive with EF-P preferably has the T-stem of tRNA^{GluE2} introduced into tRNA^{Pro1}.

When the tRNA not having a D-arm structure interactive with EF-P is used, the tRNA preferably has the base sequence represented by SEQ ID NO: 2 in order to allow an EF-Tu protein to exert an accommodation promotion effect.

The tRNA not having a D-arm structure interactive with EF-P is preferably tRNA^{GluE2}.

Therefore, in one of preferred modes of a cell-free translation system in the method of producing a library according to the present invention, the cell-free system contains a tRNA charged with one selected from cyclic β-, γ-, and δ-amino acids (cAAs) and the tRNA is at least one selected from tRNA^{Pro1E2} and tRNA^{GluE2}. When the cAA is an aromatic amino acid, the tRNA is tRNA^{Pro1E2} in a preferred mode.

In the tRNA having a D-arm structure interactive with EF-P and the tRNA not having a D-arm structure interactive with EF-P, a base sequence other than SEQ ID NO: 1 and/or SEQ ID NO: 2 may be derived from a wild-type tRNA, may be derived from an Escherichia coli-derived wild type tRNA, or an artificial tRNA prepared by the in vitro transcription.

The sequence represented by the formula (1) in the method of producing a library according to the present invention may contain at least one cAA selected from cyclic β-, γ-, and δ-amino acids.

When two or more cAAs are contained, two or more cAAs may be contained at random in the sequence represented by the formula (1) or two or more consecutive cAAs may be contained in the sequence represented by the formula (1). The term "two or more cAAs may be contained at random in the sequence represented by the formula (1)" as used herein means a mode in which at least two cAAs are contained at any position of the sequence of the formula (1) and these two or more cAAs are not adjacent to each other. Therefore, when two or more cAAs are contained at random, an amino acid or derivative thereof other than a cAA adjacently binds to each of these two or more cAAs.

When at least one cAA is contained in the sequence represented by the formula (1), the kind of the cAA is not particularly limited and a cAA represented by any of the formulas (I-1), (I-2), (I-3), and (I-4) is preferred, a cAA represented by either the formula (I-1) or (I-2) is more preferred, a cAA represented by the formula (I-4) is more preferred, and a cAA represented by the following structure is still more preferred. When two or more cAAs are contained at random in the sequence represented by the formula (1), the kind of the cAAs is not particularly limited and a cAA represented by any of the formulas (I-1), (I-2), (I-3), and (I-4) is preferred, a cAA represented by any of the formulas (I-1), (I-2), and (I-4) is more preferred, and a cAA represented by the following structure is still more preferred.

When two or more cAAs are contained at random, they may be any combination of the cAAs represented by the formulas (I-1), (I-2), (I-3), and (I-4). The combination may be that of two or more cAAs represented only by the formula (I-1), that of two or more cAAs represented only by the formula (I-2), that of two or more cAAs represented only by the formula (I-3), or that of two or more cAAs represented only by the formula (I-4).

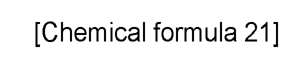

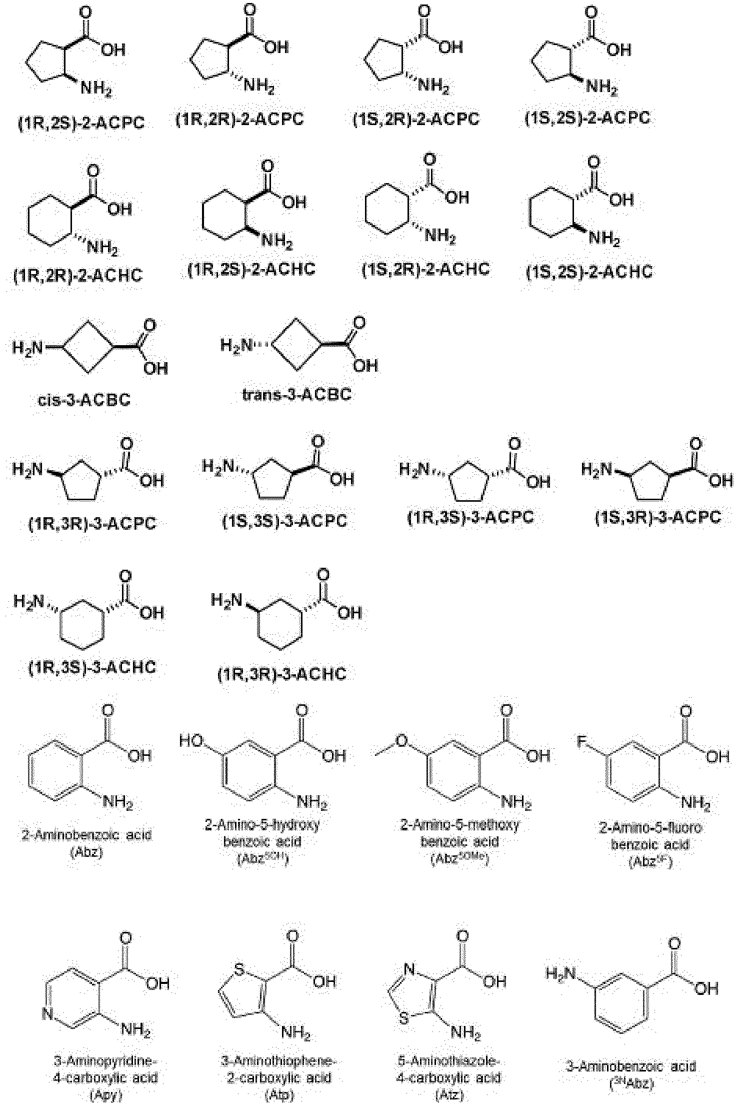

When 2 or more consecutive cAAs are contained in the sequence represented by the formula (1), the kind of the two consecutive cAAs is not particularly limited and they are preferably any of the cAAs represented by the formula (I-1), (I-2), (I-3), and (I-4), more preferably any of the cAAs represented by the formula (I-1), (I-2), and (I-4), still more preferably the cAA represented by the formula (I-1), also still more preferably the cAA represented by the formula (I-4), and further more preferably the cAA represented by the following structure.

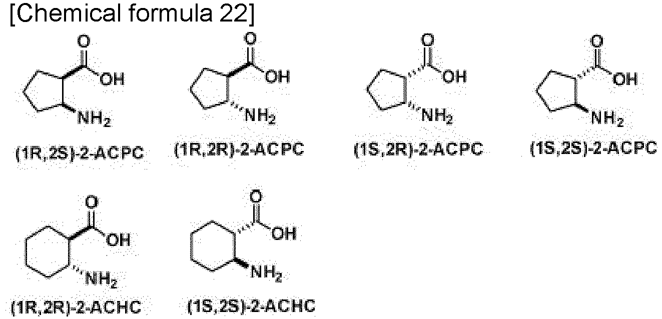

When two or more consecutive cAAs are contained in the sequence represented by the formula (1) as described above, it is only necessary that at least one unit represented by -cAA-cAA- may be contained in the sequence represented by the formula (1) and another cAA may be contained. The other cAA contained in the sequence is not particularly limited and a preferred mode is the same as that in the case where two or more cAAs are contained at random in the sequence represented by the formula (1).

When two or more consecutive cAAs are contained in the sequence represented by the formula (1), the number of the consecutive cAAs is preferably 2.

When three or more consecutive cAAs are contained in the sequence represented by the formula (1), the cAAs are preferably a cAA represented by the formula (I-1), more preferably (1S,2S)-2-ACPC. By using (1S,2S)-2-ACPC as the cAAs, these cAAs tend to be consecutively incorporated in a peptide more efficiently. Since the folding of (1S,2S)-2-ACPC is expressed when the number of residues is 4 or so (refer to Kwon, S., Jeon, A., Yoo, S. H., Chung, I. S. & Lee, H. S. Unprecedented molecular architectures by the controlled self-assembly of a β-peptide foldamer. Angew. Chem. Int. Ed. Engl. 49, 8232-8236 (2010) and Kim, J. et al. Microtubes with rectangular cross-section by self-assembly of a short β-peptide foldamer. J. Am. Chem. Soc. 134, 20573-20576, (2012)), it is presumed that particularly a peptide containing 4 or more consecutive (1S,2S)-2-ACPCs is folded into a helical structure. As described above, according to the present invention, a folded molecule can be synthesized biochemically.

When 3 or more consecutive cAAs are contained in the sequence represented by the formula (1), it is only necessary that at least one unit represented by -cAA-cAA-cAA- is contained in the sequence represented by the formula (1) and another cAA may be contained in the sequence. The other cAA contained in the sequence is not particularly limited and a preferred mode is similar to that in the case where two or more cAAs are contained at random in the sequence represented by the formula (1).

The (1S,2S)-2-ACPC is represented by the following formula.

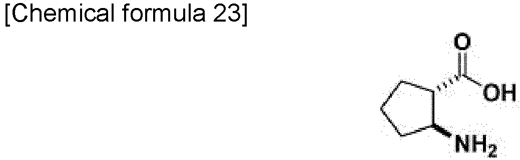

When three or more consecutive (1S,2S)-2-ACPCs are contained, the upper limit of the introduction number is not particularly limited and it is preferably 15 or less, more preferably 12 or less, still more preferably 10 or less.

As described above, a tRNA to be charged is preferably selected, depending on the kind of a cAA from the standpoint of efficiently introducing the cAA into a cyclic peptide.

The following cAA (which will hereinafter be called "cyclic β- or γ-amino acid (i)", collectively) is preferably charged onto the tRNA having a D-arm structure interactive with EF-P, more preferably charged onto tRNA^{Pro1E2}. In particular, the cAAs of the formula (I-4) (of these, particularly, Abz, Abz^{5OH}, Abz^{5OMe}, Abz^{5F}, Apy, Atp, or Atz) are preferably charged onto the tRNA having a D-arm structure interactive with EF-P, more preferably charged onto tRNA^{Pro1E2}.

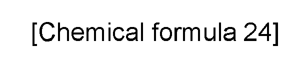

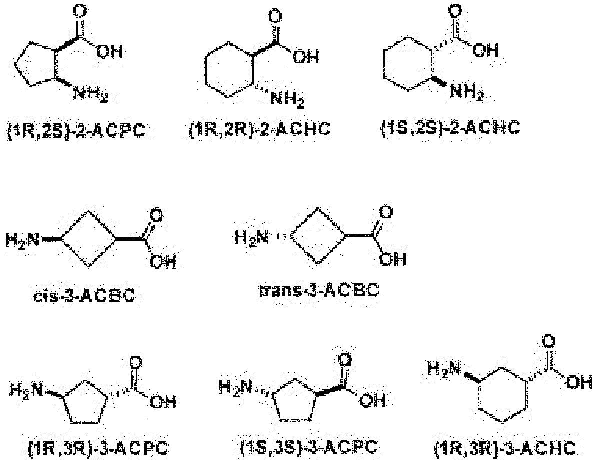

The following cAA (which will hereinafter be called "cyclic β- or γ-amino acid (ii)", collectively) is preferably charged onto the tRNA not having a D-arm structure interactive with EF-P, more preferably charged onto tRNA^{GluE2}.

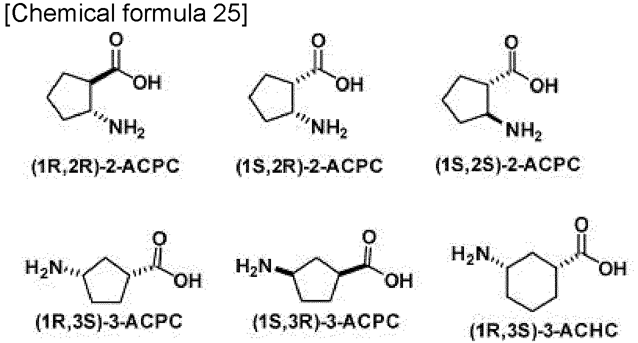

The kind or concentration of components constituting the cell-free translation system in the present invention may be adjusted as needed.

For example, when the tRNA having a D-arm structure interactive with EF-P is used, more specifically, when the tRNA charged with the cyclic β- or γ-amino acid (i) or the cAA of the formula (1-4) and having a D-arm structure interactive with EF-P is used, the concentration of EF-P may be controlled to be larger than that in the case where such a tRNA is not used.

For example, when tRNA^{Pro1E2} or tRNA^{GluE2} is used, more specifically, when tRNA^{Pro1E2} charged with the cyclic β- or γ-amino acid (i) or tRNA^{GluE2} charged with the cyclic β- or γ-amino acid (ii) is used, the concentration of EF-Tu may be controlled to be larger than that in the case where such a tRNA is not used. When tRNA^{Pro1E2} or tRNA^{GluE2} is used, more specifically, when tRNA^{Pro1E2} charged with the cyclic β- or γ-amino acid (i) or tRNA^{GluE2} charged with the cyclic β- or γ-amino acid (ii) is used, the concentration of EF-G may be controlled to be smaller than that in the case where such a tRNA is not used. Controlling the respective concentrations of EF-Tu and EF-G as described above tends to make it possible to prevent the drop-off of a peptidyl-tRNA from a ribosome during translation.

In the aforesaid preferred combination of the tRNA and the amino acid to be charged with the tRNA, the tRNA charged with the amino acid can suitably be used for the synthesis of a peptide containing the amino acid. The peptide containing the amino acid can be produced suitably by using the tRNA charged with the amino acid.

The method of producing a library according to the present invention can be used for obtaining the cyclic peptide of the present invention which will hereinafter be described in detail. The sequence represented by the above formula (1) may be a sequence represented by the formula (3) or the formula (4) which will be described later (cAA is not limited to cβAA and it may be cγAA or cδAA. It is preferably cβAA and/or cγAA, more preferably cβAA).

### <Cyclic peptide>

The present invention relates to a cyclic peptide having a cyclic structure composed of 4 to 30 amino acids or derivatives thereof, or a pharmaceutically acceptable salt of the cyclic peptide, wherein two amino acids or derivatives thereof, of the 4 to 30 amino acids or derivatives thereof of the cyclic structure are Xaa1 and Xaa2; Xaa1 and Xaa contain a structure for forming the cyclic structure, Xaa1 and Xaa2 have a linked structure via an amino acid sequence composed of 2 to 28 amino acids or derivatives thereof; and the amino acid sequence composed of the 2 to 28 amino acids or derivatives thereof is composed of at least one selected from cyclic β-, γ-, and δ-amino acids (cAAs) and an arbitrary amino acid or derivative thereof.

In the present specification, a cyclic peptide and a pharmaceutically acceptable salt thereof may hereinafter be called "cyclic peptide" collectively and simply.

The cyclic peptide of the present invention containing a cAA can form a folded structure and has possibility of allowing a cyclic amino acid to express rigidity. In addition, the peptide of the present invention is highly stable to serum or the like, and in vivo degradation of it is suppressed. The degradation of the peptide of the present invention before it acts on a target protein is therefore presumed to be suppressed.

The cyclic peptide of the present invention can be obtained by the aforesaid <method of producing a library>. A cyclic peptide selected from the library and specified can be produced in an ordinary solid-phase synthesis method.

The definition, example, and mode of the "amino acid or derivative thereof", "Xaa1", "Xaa2", "cyclic β-, γ-, and δ-amino acids (cAAs)" in the cyclic peptide of the present invention are the same as the definition, example, and mode described in the aforesaid <method of producing a library> and a preferred mode is also the same as the aforesaid one.

### <Cyclic peptide having affinity for FXIIa>

One mode of the cyclic peptide of the present invention is a cyclic peptide represented by the following formula (3).

Xaa1-(Xaa)ₙ₁-Xaa2-(Xaaₓ)ₘ (3)

In the formula (3),
Xaas are each an arbitrary amino acid or derivative thereof,
at least one of Xaas is a cyclic β-amino acid (cβAA),
Xaa1 and Xaa2 are amino acids or derivatives thereof forming the ring of the cyclic peptide,
n1 is an integer of 10 to 16,
Xaaₓ is an arbitrary amino acid or derivative thereof, and
m is an integer of 0 to 10.

In the above formula, supposing that Xaa1 is the first amino acid, at least one cAA is present as the fifth to ninth one.

The cyclic peptide represented by the formula (3) has affinity for activated blood coagulation factor XII (which may also be called "FXIIa"). One provided by the present invention is a binding agent for FXIIa containing the cyclic peptide represented by the formula (3) or pharmaceutically acceptable salt thereof. The FXIIa is serine protease involved in the kallikrein-kinin system which contributes to coagulation and inflammation. An inhibitor against FXIIa is useful as an antithrombotic drug.

The cβAA in the formula (3) is preferably represented by the formula (I-1), more preferably (1S,2S)-2-ACHC and/or (1R,2R)-2-ACPC.

At least one of the Xaas in the formula (3) is preferably a basic amino acid or derivative thereof. The basic amino acid or derivative thereof described above is not particular limited insofar as it has an amino group on the side chain thereof. Examples include lysine, arginine, and histidine, and derivatives thereof. When at least one of the Xaas in the formula (3) is a basic amino acid or derivative thereof, an interaction of the cyclic peptide of the present invention to a binding site in FXIIa can be enhanced further and a stronger bond tends to be formed. This is presumed to occur because the basic amino acid or derivative thereof fits in an S1 pocket (refer to Fig. 10b) in FXIIa.

The (Xaa)ₙ₁ in the formula (3) is preferably any one selected from the following f1 to f4.

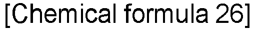

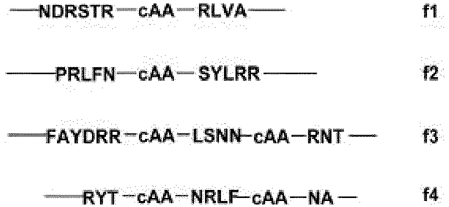

In the formulas f1 to f4, the cAA is a cyclic β-amino acid.

The cyclic peptide represented by the formula (3) is preferably represented by the formula (3-1).

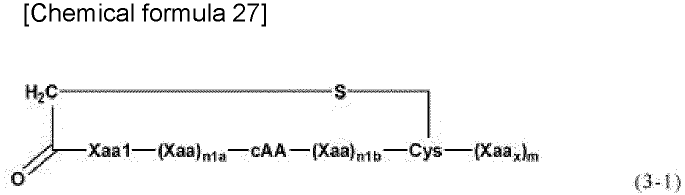

In the formula (3-1),
Xaas are each an arbitrary amino acid or derivative thereof,
cAA is a cyclic β-amino acid,
n1a is an integer of 3 to 7 and n1b is an integer of 2 to 8 (with the proviso that the sum of n1a and n1b is an integer of 9 to 15),
Xaaₓ is an arbitrary amino acid or derivative thereof,
Xaa1 is Phe or Tyr, and
m is an integer of 0 to 10, with the proviso that (Xaa)ₙ₁ₐ does not contain a cAA.

A difference (absolute value) between n1a and n1b is preferably 4 or less.

The n1a may be an integer of 3 to 6 and n1b may be an integer of 4 to 8 (with the proviso that in this case, the sum of n1a and n1b is an integer of 9 to 14).

The cyclic peptide represented by the formula (3-1) is preferably any of the following F1 to F4.

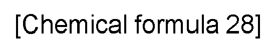

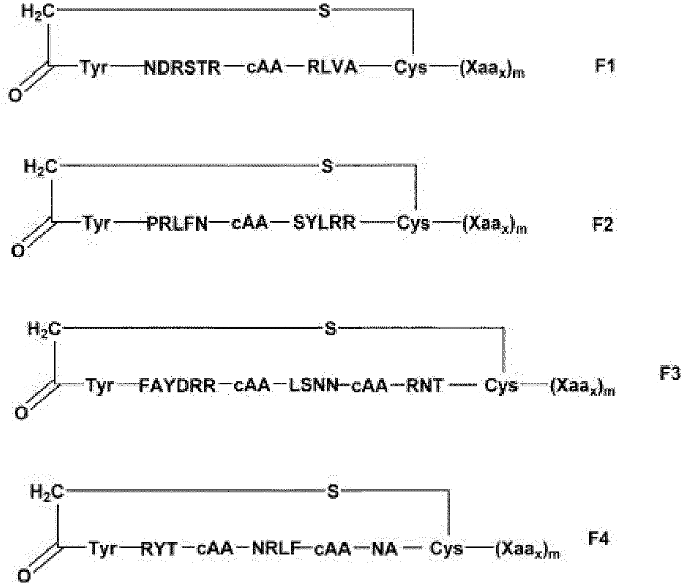

In the formulas F1 to F4, cAA, Xaaₓ, and m have the same meanings as cAA, Xaaₓ, and m in the formula (3-1).

### <Cyclic peptide having affinity for IFNGR1>

The cyclic peptide in one mode of the present invention is a cyclic peptide represented by the formula (4).

Xaa1-(Xaa)ₙ₁-Xaa2-(Xaaₓ)ₘ (4)

In the formula (4),
Xaas are each an arbitrary amino acid or derivative thereof,
at least one of the Xaas is a cyclic β-amino acid (cβAA),
Xaa1 and Xaa2 are amino acids or derivatives thereof forming the ring of the cyclic peptide,
n1 is an integer of 9 to 15,
Xaaₓ is an arbitrary amino acid or derivative thereof, and
m is an integer of 0 to 10.

In the above formula, supposing that the Xaa1 is a first amino acid, at least one cAA is present as the fourth to eighth one.

The cyclic peptide represented by the formula (4) has binding affinity for type II interferon receptor complex (which may also be called "IFNGR1"). The IFNGR1 is a receptor of an interferon-γ(IFN-γ) which is a proinflammatory cytokine having an important role in inflammation and autoimmune diseases. An inhibitor against IFNGR1 is useful for the treatment of autoimmune diseases.

The cβAA in the formula (4) is preferably represented by the formula (I-1), more preferably any one or more selected from (1S,2S)-2-ACHC, (1R,2R)-2-ACPC, and (1S,2S)-2-ACPC.

The (Xaa)ₙ₁ in the formula (4) is preferably any one selected from the following i1-1 to i1-6.

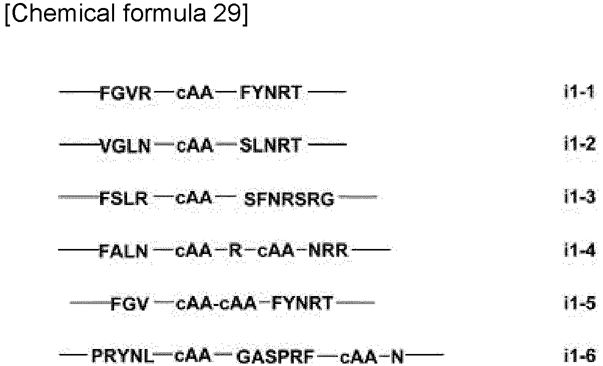

In the formulas i1-1 to i1-6, the cAA is a cyclic β-amino acid.

The cyclic peptide represented by the formula (4) is preferably represented by the following formula (4-1).

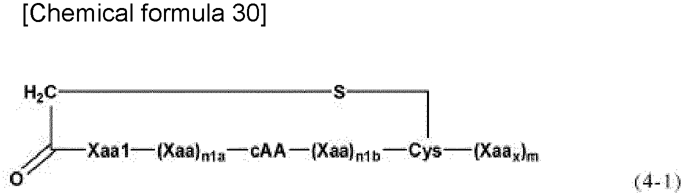

In the formula (4-1), Xaas are each an arbitrary amino acid or derivative thereof,
cAA is a cyclic β-amino acid,
n1a is an integer of 3 to 7 and n1b is an integer of 5 to 7 (with the proviso that the sum of n1a and n1b is an integer of 8 to 14),
Xaaₓ is an arbitrary amino acid or derivative thereof,
Xaa1 is Phe or Tyr, and
m is an integer of 0 to 10, with the proviso that (Xaa)ₙ₁ₐ does not contain cAA.

A difference (absolute value) between n1a and n1b is preferably 4 or less.

n1a may be an integer of 3 to 5 and n1b may be an integer of 5 to 8 (with the proviso that in this case, the sum of n1a and n1b is an integer of 8 to 13).

The cyclic peptide represented by the formula (4-1) is preferably any of the following I1-1 to 11-6.

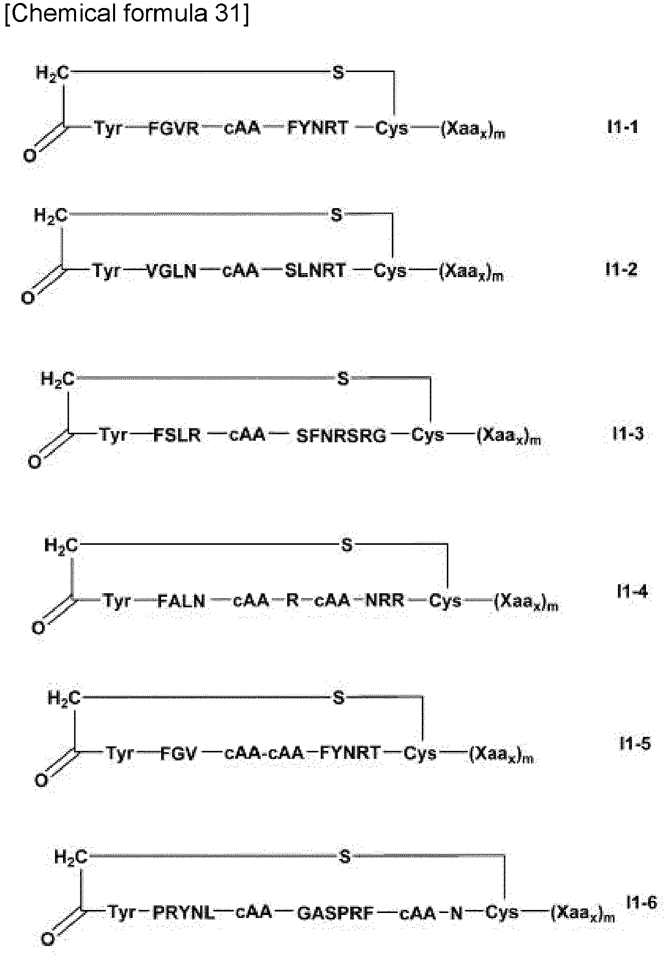

In the formulas I1-1 to 11-6, cAA, Xaaₓ, and m have the same meanings as cAA, Xaaₓ, and m in the formula (4-1).

The cyclic peptide of the present invention binds to FXIIa or IFNGR1 and inhibits its action. One provided by the present invention, therefore, is an FXIIa or IFNGR1 inhibitor containing the cyclic peptide of the present invention or pharmaceutically acceptable salt thereof. More specifically, one provided by the present invention is an FXIIa inhibitor containing the compound represented by the formula (3) or pharmaceutically acceptable salt thereof. In addition, one provided by the present invention is an IFNGR1 inhibitor containing the compound represented by the formula (4) or pharmaceutically acceptable salt thereof.

The binder or inhibitor of the present invention can be used as a pharmaceutical composition containing it.

The pharmaceutical composition of the present invention containing the FXIIa inhibitor can be used for the treatment or prevention of a disease related to blood clots. The pharmaceutical composition of the present invention containing the IFNGR1 inhibitor can be used for the treatment or prevention of a disease related to an autoimmune disease.

The administration route of the pharmaceutical composition is not particularly limited, and the composition may be either orally administered or parenterally administered. Examples of the parental administration include administration by injection such as intramuscular injection, intravenous injection or subcutaneous injection, percutaneous administration, and transmucosal administration. Examples of the administration route by the transmucosal administration include transnasal, transocular, transpulmonary, transvaginal and transrectal ones.

The cyclic peptide in the pharmaceutical composition may be subjected to various modifications from the standpoint of pharmacokinetics such as metabolism and/or excretion. For example, polyethylene glycol (PEG) and/or glycoside is added to the cyclic peptide to prolong the blood residence time and thereby lower the antigenicity.

The cyclic peptide may be encapsulated in an emulsion, nanoparticles, nanospheres, or the like used as a sustained-release base, which is/are prepared from a biodegradable high-molecular compound such as polylactic acid/glycol (PLGA), porous hydroxyapatite, liposome, surface-modified liposome, or unsaturated fatty acid. When it is administered percutaneously, it can be penetrated through the stratum corneum by passing a weak electrical current through the skin surface (iontophoresis).

The pharmaceutical composition may contain, as active ingredient, the cyclic peptide as is or it may be formulated by adding thereto a pharmaceutically acceptable additive and the like.

Examples of the dosage form of the pharmaceutical formulation include solutions (for example, injections), dispersions, suspensions, tablets, pills, powdered drug, suppositories, powders, fine granules, granules, capsules, syrups, troches, inhalants, ointments, ophthalmic formulations, nasal formulations, ear formulations, and cataplasms.

The pharmaceutical composition may be formulated in a conventional manner by using, for example, an excipient, a binder, a disintegrant, a lubricant, a dissolving agent, a solubilizing agent, a colorant, a taste/odor corrigent, a stabilizer, an emulsifier, an absorption promoter, a surfactant, a pH regulator, an antiseptic, a humectant, a dispersing agent, or an antioxidant as needed.

The additive used for the formulation is not particularly limited and examples include purified water, saline, phosphate buffer, pharmaceutically acceptable organic solvents such as dextrose, glycerol, and ethanol, animal or vegetable oils, lactose, mannitol, glucose, sorbitol, crystalline cellulose, hydroxypropyl cellulose, starch, corn starch, silicic anhydride, magnesium aluminum silicate, collagen, polyvinyl alcohol, polyvinylpyrrolidone, carboxy vinyl polymer, carboxymethylcellulose sodium, sodium polyacrylate, sodium alginate, water-soluble dextran, carboxymethyl starch sodium, pectin, methyl cellulose, ethyl cellulose, xanthan gum, gum arabic, tragacanth, casein, agar, polyethylene glycol, diglycerin, glycerin, polypropylene glycol, petrolatum, paraffin, octyl dodecyl myristate, isopropyl myristate, higher alcohol, stearyl alcohol, stearic acid, and human serum albumin.

As an absorption promoter in transmucosal absorption, surfactants such as polyoxyethylene lauryl ethers, sodium lauryl sulfate, and saponin; bile salts such as glycocholate, deoxycholate, and taurocholate; chelating agents such as EDTA and salicylic acid; fatty acids such as caproic acid, capric acid, lauric acid, oleic acid, linoleic acid, and mixed micelle; enamine derivatives, N-acylcollagen peptide, N-acylaminoic acid, cyclodextrines, chitosans, and nitric oxide donors may be used.

The tablets or pills may be sugar-, gastric-, or enteric-coated.

The solutions may contain distilled water for injection, physiological saline, propylene glycol, polyethylene glycol, a vegetable oil, an alcohol, or the like. The solutions may further contain a humectant, an emulsifier, a dispersant, a stabilizer, a dissolving agent, a solubilizing agent, an antiseptic, or the like.

The present invention also provides a method of administering the FXIIa inhibitor or IFNGR1 inhibitor to a patient who requires the inhibitor and thereby treating or preventing the disease of the patient.

The dose of the HGF inhibitor of the present invention is determined as needed by those skilled in the art, depending on the symptom, age, sex, weight, and difference in sensitivity of patients who require the inhibitor, the administration method, administration interval, and the type of the formulation.

The patient is a mammal, preferably a human being.

### Examples

The tRNAs used in the present Examples were prepared by the following method.

### (Preparation of tRNA)

Flexizymes (dFx and eFx) and tRNAs to be charged with 2-ACPC, 2-ACHC, ^{ClAc}D-Phe, ^{ClAc}D-Tyr, and D-Cys were prepared by the in vitro transcription using T7 RNA polymerase. Template DNAs for the transcription were prepared by the extension using a forward and reverse extension primer pair and the subsequent PCR using a forward and reverse PCR primer pair. Refer to Table 3 about the sequence of the primer.

The PCR products thus obtained were purified by phenol/chloroform extraction and ethanol precipitation. The template DNAs are composed of a T7 promoter at 5'-end and a downstream tRNA or flexizyme sequence. Refer to Table 3 about the sequence.

The transcription of RNA from the PCR product was performed at 37°C for 16 hours in a 500 µL reaction mixture (a mixture of 40 mM Tris-HCI (pH 8.0), 22.5 mM MgCl₂, 1 mM DTT, 1 mM spermidine, 0.01% Triton X-100, 0.12 µM T7 RNA polymerase, 0.04 U/µL RNasin RNase inhibitor (Promega), and 3.75 mM NTP). For the transcription of the tRNA, 5 mM GMP or CMP was added to the above solution to start the transcription from G or C. The RNA transcripts thus obtained were subjected to DNase treatment at 37°C for 30 minutes with RQ1 DNase (Promega) and then, purified using an 8% (in the case of the tRNA) or 12% (in the case of the flexizyme) polyacrylamide gel containing 6M urea.

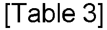

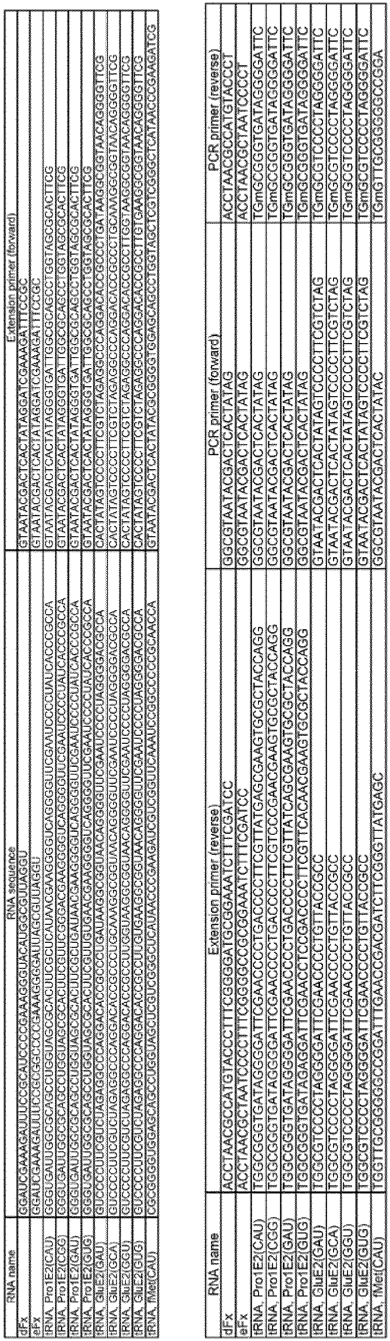

### (Aminoacylation of tRNA)

Based on the previously reported methods (refer to Murakami, H., Ohta, A., Ashigai, H. & Suga, H. A highly flexible tRNA acylation method for non-natural polypeptide synthesis. Nat. Methods 3, 357-359 (2006) and Saito, H., Kourouklis, D. & Suga, H. An in vitro evolved precursor tRNA with aminoacylation activity. EMBO J. 20, 1797-1806 (2001)), 2-ACPC, 2-ACHC, and D-Cys were pre-activated in the form of a 3,5-dinitrobenzyl ester (DBE), and ^{ClAc}D-Phe and ^{ClAc}D-Tyr were activated in the form of a cyanomethyl ester (CME). Those activated amino acids were charged onto the tRNA by using flexizymes (dFx for 2-ACPC, 2-ACHC, and D-Cys-DBE, or eFx for ^{ClAc}D-Phe-CME and ^{ClAc}D-Tyr). Aminoacylation using flexizymes was carried out at 0°C in a 50 mM buffer (Bicine-KOH (pH 8.7) for 2-ACPC and 2-ACHC, D-Cys, and HEPES-KOH (pH 7.5) for ^{ClAc}D-Phe and ^{ClAc}D-Tyr), 600 mM MgCl₂, 20% DMSO, 25 µM dFx or eFx, 25 µM tRNA, and 5 mM activated amino acid. Then, the aminoacyl-tRNAs thus obtained were subjected to ethanol precipitation to remove the activated amino acid and then, the pellet was washed twice with 70% ethanol containing 0.1M sodium acetate (pH 5.2).

### [Example 1: Incorporation test of 2-ACPC into peptide]

To verify the ability of ribosome to incorporate one or a plurality of cβAAs into a peptide chain, the elongation of a peptide sequence when four 2-ACPC isomers, that is, (1R,2R)-2-ACPC, (1R,2S)-2-ACPC, (1S,2R)-2-ACPC, and (1S,2S)-2-ACPC were used was studied first.

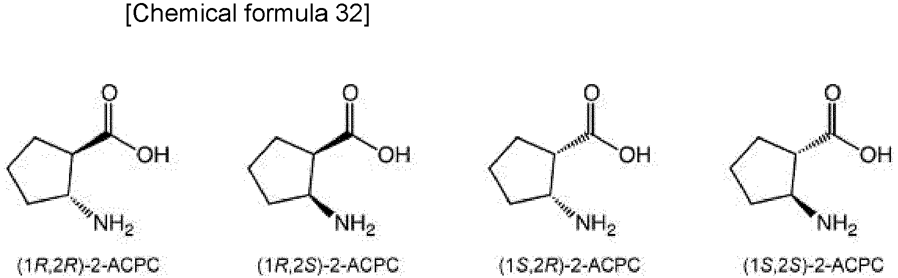

Based on the method described in Murakami, H., Ohta, A., Ashigai, H. & Suga, H. A highly flexible tRNA acylation method for non-natural polypeptide synthesis. Nat. Methods 3, 357-359 (2006) and Goto, Y., Katoh, T. & Suga, H. Flexizymes for genetic code reprogramming. Nat Protoc 6, 779-790, (2011), those amino acids were charged onto tRNA^{Pro1E2}_{CGG} that has a specific D-arm motif for EF-P binding and an engineered T-stem motif by using flexizymes (flexible tRNAacylation ribozymes). The tRNA^{Pro1E2}_{CGG} is shown in Fig. 1.

Then, the respective pre-charged tRNAs were added to a FIT (Flexible In vitro Translation) system, which was a reconstituted E. coli translation system, to express peptides rP1 to rP10. The peptides rP1 to rP10 are shown in Table 4. In their sequences, the flag in mR1 to mR13 was GACUACAAGGACGACGACGACAAG and the flag in rP1 to rP13 was Asp-Tyr-Lys-Asp-Asp-Asp-Asp-Lys.

**[Table 4]**

| | | |
|---|---|---|
| mRNA(mR1): | AUG UAC AAG AAG UAC AAA AAG UAC AAA CCG GGU GGU flag UAA | |
| reprogrammed peptide (rP1): | fMet Tyr Lys Lys Tyr Lys Lys Tyr Lys cβAA Gly Gly flag (stop) | |
| mRNA(mR2): | AUG UAC AAG AAG UAC AAA AAG UAC AAA CCG CCG GGU flag UAA | |
| reprogrammed peptide (rP2): | fMet Tyr Lys Lys Tyr Lys Lys Tyr Lys cβAA cβAA Gly flag (stop) | |
| mRNA(mR3-mR10): | AUG UAC AAG AAG UAC AAA AAG UAC AAA (CCG)ₙ flag UAA | n=3-10 |
| reprogrammed peptide (rP3-rP10): | fMet Tyr Lys Lys Tyr Lys Lys Tyr Lys (cβAA)ₙ flag (stop) | |

The translation of the peptides was performed in the following procedure.

The FIT system used in the present example contained only 5 µM EF-P, 3 µM IF2, 20 µM EF-Tu, and 0.1 µM EF-G, and the minimum number of amino acids and aminoacyl-tRNA synthetase (ARS), that is, Met, Lys, Gly, Tyr, and Asp, and ARS corresponding thereto.

In order to improve the affinity between cβAA and tRNA, the EF-Tu concentration in the conventional FIT system (refer to Katoh, T. & Suga, H. Ribosomal incorporation of consecutive β-amino Acids. J. Am. Chem. Soc. 140, 12159-12167 (2018), Katoh, T., Tajima, K. & Suga, H. Consecutive elongation of D-amino acids in translation. Cell Chem. Biol. 24, 1-9 (2017), and Katoh, T., Iwane, Y. & Suga, H. Logical engineering of D-arm and T-stem of tRNA that enhances D-amino acid incorporation. Nucleic Acids Res. 45, 12601-12610 (2017)) was increased from 10 µM to 20 µM. Similarly, the EF-G concentration was reduced from 0.26 µM to 0.1 µM to prevent the drop-off, from a ribosome, of the peptidyl-tRNA during translation.

In order to evaluate the effect of EF-P, translation was performed also in the absence of EF-P. The details of the composition of the above particular FIT system were as shown in the following Table 5.

**[Table 5]**

| |
|---|
| 50mM HEPES-KOH (pH 7.6) |
| 100mM Potassium acetate |
| 12.3mM Magnesium acetate |
| 2mM ATP |
| 2mM GTP |
| 1mM CTP |
| 1mM UTP |
| 20mM Phosphocreatine |
| 0.1mM 10-Formyl-5,6,7,8-tetrahydrofolic acid |
| 2mM Spermidine |
| 1mM DTT |
| 1.5mg/mL Escherichia coli E. coli total tRNA |
| 1.2µM Escherichia coli E. coli ribosome |
| 0.6µM Methionyl-tRNA formyl transferase |
| 2.7µM IF1 |
| 3µM IF2 |
| 1.5µM IF3 |
| 0.1µM EF-G |
| 20µM EF-Tu/Ts |
| 0.25µM RF2 |
| 0.17µM RF3 |
| 0.5µM RRF |
| 4µg/mL Creatine kinase |
| 3µg/mL Myokinase (mykinase) |
| 0.1µM Inorganic pyrophosphatase |
| 0.1µM Nucleotide diphosphate kinase |
| 0.1µM T7 RNA polymerase |
| 0.13µM AspRS |
| 0.11µM LysRS |
| 0.03µM MetRS |
| 0.02µM TyrRS |
| 0.09µM GlyRS |
| 0.05mM [¹⁴C]-Asp |
| 0.5mM Lys |
| 0.5mM Met |
| 0.5mM Tyr |
| 0.5mM Gly |
| 25µM Each pre-charged aminoacyl-tRNA |
| 0.5µM Template DNA |

The template DNAs for the peptide translation were prepared by the extension using a forward and reverse extension primer pair and the subsequent PCR using a forward and reverse PCR primer pair. Refer to Table 3 about the sequence of the primers. The PCR products thus obtained were purified by phenol/chloroform extraction and ethanol precipitation. The template DNAs were transcribed to mRNA by T7 RNA polymerase contained in the FIT system and translated into peptides. The peptides rP1 to rP11 and rP13 contained a Tyr-Lys-Lys-Tyr-Lys-Lys-Tyr-Lys sequence before a cβAA incorporation site in order to suppress the drop-off of the peptidyl-tRNA and promote ionization in MALDI-TOF MS. The translation reaction was performed at 37°C in a 2.5 µL solution, stopped by the addition of 2.5 µL of stop solution (0.9M Tris-HCI (pH 8.45), 8% SDS, 30% glycerol, and 0.001% xylene cyanol), and heated at 95°C for 3 minutes. Then, the samples were subjected to 15% tricine SDS-PAGE and analyzed by autoradiography using Typhoon FLA 7000 (GE Healthcare). The expression level of a radioisotopelabeled peptide was normalized by the intensity of [¹⁴C]-Asp band.

The MALDI-TOF MS of the translated peptide was performed in the following procedure.

Translation was performed for 45 minutes in the above reaction mixture containing 0.5 mM non-radioactive Asp instead of radioactive [¹⁴C]-Asp. Next, after addition of an equivalent amount of a 2xHBS buffer (100 mM HEPES-KOH (pH 7.6), 300 mM NaCl), the resulting mixture was mixed with 5 µL of ANTI-FLAG M2 affinity gel (Sigma) and incubated for 30 minutes at room temperature. The gel beads were washed once with 25 µL of an HBS buffer (50 mM HEPES-KOH (pH 7.6) and 150 mM NaCl) and 15 µL of 0.2% trifluoroacetic acid was added to elute the peptides from the beads. Then, the peptides were desalted using SPE C-TIP (Nikkyo Technos) and co-crystallized with α-cyano-4-hydroxycinnamic acid. MALDI-TOF MS was performed using UltrafleXtreme (Bruker Daltonics). A peptide calibration standard II (Bruker Daltonics) was used for external mass calibration.

The expression level of the peptides when four 2-ACPC isomers were used is shown in Fig. 2. The SDS-PAGE analysis results of the translation products obtained using the four 2-ACPC isomers are shown in Figs. 3a to 3d. The MALDI-TOF MS results of the translation products obtained using the four 2-ACPC isomers are shown in Figs. 4 and 5.

Although tRNA^{Pro1E2} generally improves the consecutive incorporation efficiency of D-amino acid and β³-amino acid, EF-P enhanced only the expression of rP2 when (1R,2S)-2-ACPC was used and showed a negative effect on the expression of peptides containing the other three steric isomers.

It was revealed that the efficiency of the double elongation of (1S,2S)-2-ACPC was on the same level with that of the single elongation. As a result of a test on the expression on longer peptides (rP3 to rP10), the consecutive elongation was prolonged up to 10 residues (refer to Fig. 2-IV).

### [Example 2: Incorporation test of 2-ACHC into peptide]

The possibility of the introduction of four 2-ACHC isomers: (1R,2R)-2-ACHC, (1R,2S)-2-ACHC, (1S,2R)-2-ACHC, and (1S,2S)-2-ACHC into rP1 and rP2 (refer to Table 4) was tested.

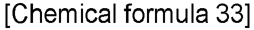

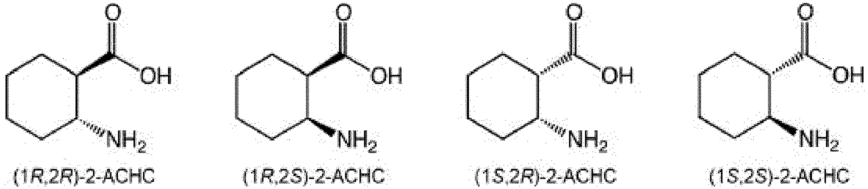

The expression level of the peptides using four 2-ACHC isomers is shown in Fig. 6. The SDS-PAGE analysis results of the translation products for which four 2-ACHC isomers were used are shown in Fig. 3e. The MALDI-TOF MS results of the translation products obtained using four 2-ACHC isomers are shown in Fig. 7.

Four 2-ACHCs were introduced by single incorporation into rP1 and no significant difference in expression level was found between in the presence and absence of EF-P.

When two consecutive 2-ACHCs were incorporated, the product of rP2 was not observed in the absence of EF-P. On the other hand, the expression of rP2 containing (1R,2R)-2-ACHC or (1S,2S)-2-ACHC was observed in the presence of EF-P, showing that EF-P can promote the consecutive incorporation of them.

### [Example 3: ribosomal synthesis of designed foldamer peptide]

The possibility of consecutive incorporation of many kinds of cβAAs was tested.

First, peptide rP11 containing two (1S,2S)-2-ACPCs assigned to AUU codon and one (1S,2S)-2-ACHC assigned to CAU codon was designed. The rP11 is shown in Table 6.

**[Table 6]**

| | |
|---|---|
| mRNA (mR11): | AUG UAC AAG AAG UAC AAA AAG UAC AAA AUU CAU AUU flag UAA fMet Tyr Lys Lys Tyr Lys Lys Tyr Lys (1S,2S)- (1S,2S)- (1S,2S)- flag (stop) 2-ACPC 2-ACHC 2-ACPC |
| reprogrammed peptide (rP11): | |
| mRNA (mR12): | AUG ACU ACU ACU ACU CAU AAG AAG AAG flag UAA ClAcD-Phe (1S,2S)- (1S,2S)- (1S,2S)- (1S,2S)- D-Cys Lys Lys Lys flag (stop) 2-ACPC 2-ACPC 2-ACPC 2-ACPC S |
| reprogrammed peptide (rP12): | |
| mRNA (mR13): | AUG UAC AAG AAG UAC AAA AAG UAC AAA fMet Tyr Lys Lys Tyr Lys Lys Tyr Lys CCG UAC UAU CCG UAU UAC CCG UAC UAU CCG flag UAA (1S,2S)- Tyr Tyr (1S,2S)- Tyr Tyr (1S,2S)- Tyr Tyr (1S,2S)- flag (stop) 2-ACPC 2-ACPC 2-ACPC 2-ACPC |
| reprogrammed peptide (rP13): | |
| | |

The finding suggesting that the presence of EF-P inhibited the incorporation of (1S,2S)-2-ACPC (in other words, a tRNA having a D-arm structure interactive with EF-P was not optimum for the introduction of (1S,2S)-2-ACPC) was obtained in Example 1, so that in order to incorporate (1S,2S)-2-ACPC, tRNA^{GluE2}_{GAU} whose T-stem recruited EF-Tu but whose D-arm was not interactive with EF-P was selected as a tRNA. In order to incorporate (1S,2S)-2-ACHC, on the other hand, tRNA^{Pro1E2}_{GUG} which recruited both EF-Tu and EF-P was selected.

The translation of rP11 in the presence of EF-P produced a desired peak of a full length rP11 in MALDI-TOF MS. Similarly, rP12 (refer to Table 6) having N-chloroacetyl-D-phenylalanine (^{ClAc}D-Phe) at the N terminal side of (1S,2S)-2-ACPC and D-cysteine (D-Cys) downstream of (1S,2S)-2-ACPC was expressed and it was confirmed in MALDI-TOF MS that a desired thioether cyclic skeleton was obtained. Further, rP13 (refer to Table 6) containing four (1S,2S)-2-ACPCs, each at every 3 amino acid residues, was expressed and it was confirmed that a desired peptide was obtained. The MALDI-TOF MS results of rP11, rP12, and rP13 are shown in Fig. 8.

This type of peptide containing (1S,2S)-2-ACPC is known to induce 10/11/11-helix (refer to Schmitt, M. A., Choi, S. H., Guzei, I. A. & Gellman, S. H. New helical foldamers: heterogeneous backbones with 1:2 and 2:1 α:β-amino acid residue patterns. J. Am. Chem. Soc. 128, 4538-4539 (2006)). These results have proved the possibility of ribosomal synthesis of a designed foldamer peptide having a helix.

### [Example 4-1: Preparation of library]

Based on the method of expressing a cyclic foldamer peptide containing a cβAA and with reference to Yamagishi, Y. et al. Natural product-like macrocyclic N-methyl-peptide inhibitors against a ubiquitin ligase uncovered from a ribosomeexpressed de novo library. Chem. Biol. 18, 1562-1570 (2011). and Passioura, T., Katoh, T., Goto, Y. & Suga, H. Selection-based discovery of druglike macrocyclic peptides. Annu. Rev. Biochem. 83, 727-752 (2014)., two targets, that is, human factor XIIa (FXIIa) and interferon γ receptor 1 (IFNGR1) were selected, and new discovery of a physiologically active molecule from a random sequence library was investigated by using a RaPID (Random Non-standard Peptides Integrated Discovery) system.

The cyclic peptide library was designed, as shown below, to have a repetition of 6 to 15 random residues (encoded by an NNU codon) including three cβAAs, (1S,2S)-2-ACHC, (1R,2R)-2-ACPC, and (1S,2S)-2-ACPC which was sandwiched between cyclized ^{ClAc}D-Tyr and D-Cys and was linked to the 3' end of mRNA via a puromycin linker. Assignment of an amino acid to an NNU codon is shown in Table 7.

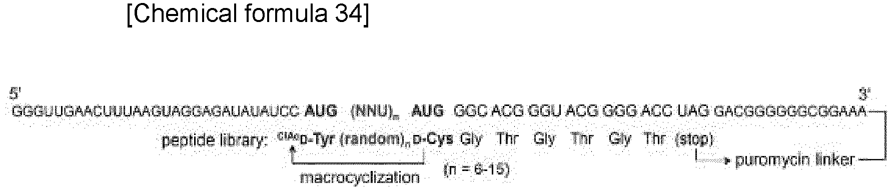

**[Table 7]**

| 1st | 2nd | | | | 3rd | | |
|---|---|---|---|---|---|---|---|
| | U | C | A | G | | | |
| U | Phe | Ser | Tyr | 3 | U | 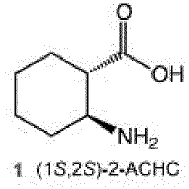 | |
| | | | | | C | | |
| | Leu | Ser | stop | | G | | |
| C | Leu | Pro | 2 | Arg | U | | |
| | | | | | C | | |
| | Leu | Pro | | Arg | G | 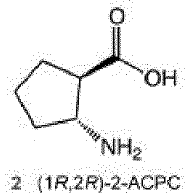 | 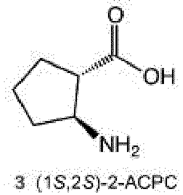 |
| A | 1 | Thr | Asn | Ser | U | | |
| | | | | | C | | |
| | ^{ClAc}_{D}-Tyr/_{D}-Cys | Thr | Lys | Arg | G | | |
| G | Val | Ala | Asp | Gly | U | | |
| | | | | | C | | |
| | Val | Ala | | Gly | G | | |

More specifically, the preparation of the library was performed in the following procedure;
The FIT system used in the preparation of the cyclic peptide library is as shown below in Table 8.

**[Table 8]**

| |
|---|
| 50mM HEPES-KOH (pH 7.6) |
| 100mM Potassium acetate |
| 12.3mM Magnesium acetate |
| 2mM ATP |
| 2mM GTP |
| 1mM CTP |
| 1mM UTP |
| 20mM Phosphocreatine |
| 2mM Spermidine |
| 1mM DTT |
| 1.5mg/mL Escherichia coli E. coli total tRNA |
| 1.2µM Escherichia coli E. coli ribosome |
| 0.6µM Methionyl-tRNA formyl transferase |
| 2.7µM IF1 |
| 3µM IF2 |
| 1.5µM IF3 |
| 0.1µM EF-G |
| 20µM EF-Tu/Ts |
| 0.25µM RF2 |
| 0.17µM RR3 |
| 0.5µM RRF |
| 4µg/-mL Creatine kinase |
| 3µg/mL Myokinase (mykinase) |
| 0.1µM Inorganic pyrophosphatase |
| 0.1µM Nucleotide diphosphate kinase |
| 0.1µM T7 RNA polymerase |
| 0.13µM AspRS, 0.11µM LysRS, 0.02 µM TyrRS, 0.09µM GlyRS, 0.73µM AlaRS, |
| 0.03µM ArgRS, 0.38µM AsnRS, 0.04µM LeuRS, 0.15µM PheRS, 0.16µM ProRS, |
| 0.04µM SerRS, 0.09µM ThrRS, 0.04µM VaIRS, |
| 0.5 mM each of Asp, Lys, Tyr, Gly, Ala, Arg, Asn, Leu, Phe, Pro, Ser, Thr, Val |
| 12.5µM ^{ClAc}D-Tyr-tRNA^{fMet}_{CAU} |
| 20µM D-Cys-tRNA^{Pro1E2}_{GAU} |
| 20µM (1S, 2S)-2-ACHC-tRNA^{Pro1E2}_{GAU} |
| 20µM (1R, 2R)-2-ACPC-tRNA^{GluE2}_{GUG} |
| 20µM (1S, 2S)-2-ACPC-tRNA^{GluE2}_{GCA} |
| 1.5 µM mRNA library bound to a puromycin linker |

The peptide library was translated at 37°C for 30 minutes in 150 µL (for the first selection), 10 µL (for the second to fourth selection), or 5 µL (for the fifth to seventh selection) FIT system.

Then, the reaction mixture was incubated at room temperature for 12 minutes and 0.04 time the amount of 500 mM EDTA (pH 8.0) was added. The resulting mixture was incubated at 37°C for 30 minutes to induce dissociation of ribosome from the mRNA-peptide complexes.

The reverse transcription was performed at 42°C for 15 minutes by using NNUAUG-GT3.R38 primer (5'-TTTCCGCCCCCCGTCCTAGGTCCCCGTACCCGTGCCCA-3') and M-MLV reverse transcriptase (Promega) lacking RNase H activity.

Then, the cDNA/mRNA/peptide complexes were mixed with Dynabeads streptavidin (Thermo Fisher) on which FXIIa was not immobilized or Dynabeads protein G (Thermo Fisher) on which IFNGR1 was not immobilized and the mixture was incubated three times at 4°C for 15 minutes as a negative selection. The negative selection was not performed in the first round of the selection.

The supernatant of the negative selection was collected and mixed at 4°C for 15 minutes with a 0.48x translation volume of FXIIa immobilized Dynabeads streptavidin or a 0.24x translation volume of IFNGR1 immobilized Dynabeads protein G. The beads were washed three times with 100 µL of a cold TBS-T buffer (100 mM Tris-HCI, pH7.5, 300 mM NaCl, 0.05% Tween20).

Recombinant biotinylated human FXIIa was purchased from Molecular Innovations. The recombinant human IgG1 Fc and Fc labeled human IFNGR1 were purchased from R&D systems and AcroBiosystems, respectively.

Next, 100 µL of a PCR buffer (10 mM Tris-HCI (pH 9.0), 50 mM KCI, 0.1% Triton X-100, 0.25 mM dNTP, 2.5 mM MgCl₂, 0.25 µM T7.F52 primer 5'-GGCGTAATACGACTCACTATAGGGTTGAACTTTAAGTAGGAGATATATCCAT-3', and 0.25 µM NUAUG-GT3.R38 primer) was added to the beads and cDNAs were eluted at 95°C for 5 minutes and amplified by PCR. The eluted solution (1 µL) was mixed with 19 µL of a PCR buffer containing SYBR Green I and Taq DNA polymerase and the amount of cDNA was determined by the Real-Time PCR.

### [Example 4-2: Novel discovery of cyclic foldamer peptide]

First, the library was subjected to the negative selection as described above in order to remove species bound to beads and then, as positive selection, was brought into contact with a target protein immobilized on magnetic beads.

Seven rounds of affinity selection were performed and the recovery rate of cDNA in each round was measured by qPCR. As a result, in the affinity selection of FXIIa and IFNGR1, a significant increase in the recovery rate was observed after the fourth round. Deep sequencing of cDNA has revealed that peptide sequences having one or more cβAAs are abundantly present in the library of the fourth round. The peptides present in the library are shown in Table 9 (for FXIIa) and Table 10 (for IFNGR1).

**[Table 9]**

| peptide sequence | read number | peptide name |
|---|---|---|
| yNDRSTR2RLVAc | 10796 | F1 |
| yPRLFN2SYLRRc | 10761 | F2 |
| yFAYDRR1LSNN1RNYc | 1601 | F3 |
| y1AFDRTFN3RT1TRYc | 777 | |
| ySDRSDYNRRVGc | 609 | |
| yRYRNNNYRRc | 267 | |
| yRYT1NRLF1NAc | 204 | F4 |
| vFLYP1PRYNR3FTNc | 161 | |
| yR3FSDLNSLYTSc | 75 | |
| yNDRSTR2RPVAc | 75 | |
| ySDRSTR2RLVAc | 63 | |
| yPRPFN2SYLRRc | 51 | |
| yPRLFS2SYLRRc | 51 | |
| yNDRSTRHRLVAc | 49 | |
| yPRLFK2SYLRRc | 41 | |
| yPRLFN2SYPRRc | 38 | |
| ySDRSTF2R1VLG1NSc | 34 | |
| yPRLSN2SYLRRc | 31 | |
| yNDRSTRRRLVAc | 30 | |
| yNDRSTR2RLAAc | 29 | |
| yFAYDRR1LSNNIRMYc | 29 | |
| yPRLLIIVIFVV | 25 | |
| y1AVDRSYS2ST1RYYc | 25 | |
| IPRLFN2SYLRRc | 22 | |
| yVDT1LRTL1NVc | 21 | |
| ySNVP3RYSSDS3RYSc | 21 | |
| yR3PADLNRLFQ | 21 | |
| yNDRSTR2RLVTc | 20 | |
| yPRLLN2SYLRRc | 19 | |
| y1AFDRTFNRRT1TRYc | 19 | |
| yPRLFN2SYLR2c | 18 | |
| yPRLFN2SYL3Rc | 18 | |
| yPRLFN2GYLRRc | 18 | |
| yNGRSTR2RLVAc | 18 | |
| yFAYDRR1LSSN1RNYc | 18 | |
| yRYT1NRLL1NAc | 17 | |
| yFAYDRR1LSNS1RNYc | 17 | |
| yPRLFN2SYLR3c | 16 | |
| yNDRST32RLVAc | 16 | |
| yRYRNDNYRRc | 15 | |
| yPRLFN2S2LRRc | 15 | |
| yPRLFM2SYL2Rc | 14 | |
| yMDRSTR23LVAc | 14 | |
| yTNT1LRLLSNc | 13 | |
| yNDRSTR22LVAc | 13 | |
| yNDRSAR2RLVAc | 13 | |
| yND2STR2RLVAc | 13 | |
| yPRLFNRSYLRRc | 12 | |
| y1ATNRV3VNRT13NYc | 12 | |
| yP2LFN2SYLRRc | 11 | |
| yNDRSTR2RL1Ac | 11 | |
| yGYL3FTc | 11 | |
| INDRSTR2RLVAc | 11 | |
| yFAYDRRILSNN1RNYc | 10 | |

**[Table 10]**

| peptide sequence | read number | peptide name | peptide sequence | ead number | peptide name | peptide sequence | read number | peptide name |
|---|---|---|---|---|---|---|---|---|
| yFGVR2FYNRTc | 15739 | I1-1 | yYDVR2R1YNc | 32 | | yYDLR2R1RTc | 16 | |
| y1GLS2YVNRSc | 8376 | I1-2 | yFGV32FYNRTc | 31 | I1-6 | yYGLS2FLSSc | 16 | |
| yVGLN2SLNRTc | 4401 | I1-3 | yVGLS2YVNRSc | 31 | | yFSLRHSFNRSRGc | 15 | |
| yFGLS2FYRRSc | 29 44 | | yYDLR2R1VRNc | 31 | | yAGLN2SLNRTc | 14 | |
| yFSLR2FNRSRGc | 211 8 | I1-4 | y1ALSRRRRSYRNGLc | 30 | | yFGLS2FYRRNc | 14 | |
| y1GLSHYVNRSc | 141 1 | | ySGVR2FYNRTc | 30 | | yFGVR2FYN3Tc | 14 | |
| yYGVN2SYNRSc | 11 61 | | yYGLS2FLSNc | 29 | | yFSVR2FYNRTc | 14 | |
| yYSVS2RFNRTc | 9 48 | | y1DLR2RLYDc | 28 | | yYGVR2FYNRTc | 14 | |
| yFDLR2RLANc | 90 6 | | y1G1YRRYLPRRSPc | 28 | | I1GLS2YVNRSc | 1 3 | |
| yFALN2R1NRRc | 77 9 | I1-5 | yTGLS2YVNRSc | 28 | | y1GLSHYVNRTc | 1 3 | |
| yYDLR2RLYRNc | 69 4 | | yFGLSHFYRRSc | 27 | | yFGLS2SYRRSc | 1 3 | |
| y1ALSRSRRTTRDNc. | 66 6 | | yFGVR2FYSRTc | 27 | | yFS1R2VFNRSc | 1 3 | |
| yFGLS21LSRc | 45 2 | | yYDVR2RLNPNc | 27 | | yYD1R2RLNAc | 3 | |
| yYDLR2RLSNc | 36 8 | | y1G1S3FSRNSVNRSc | 26 | | yYSVR2AINRVc | 1 2 | |
| yYDLR2RLYSc | 36 4 | | yFNL32FLSPc | 26 | | y1GLGHYVNRSc | 1 11 | |
| yYNVR2FVNRTc | 343 | | yYNLR2Y1NRSNc | 26 | | y1GLN2SLNRTc | 11 | |
| yFDVR2R1NSc | 240 | yFGVR2F2NRTc | yFGVR2F2NRTc | 25 | | yFG1R2FYNRTc | 11 | |
| yFGLS2FVSPc | 235 | | yFGVR2FYN2Tc | 25 | | yFGV22FYNRTc | 11 | |
| y1GLNRSLPPRLYNc | 177 | | yFSLR2SFNRSRSc | 24 | | yVGLN2SLNRSc | 11 | |
| yYDLR2A1NNc | 168 | | yYTR2GRSR1Nc | 24 | | IFGVR2FYNRTc | 10 | |
| y1GLS2YVNRTc | 140 | | y1GLS22VNRSc | 23 | | y1GLR2YVNRSc | 10 | |
| y1SLSRNRVPRLVLTc | 120 | | y1PVNRY1RRFNRc | 23 | | y1SLT2FVNRRc | 1 0 | |
| yYDVR2RVSNc | 107 | | yFGVR2SYNRTc | 23 | | yFGLN2FYRRSc | 1 0 | |
| yYSVN2Y1NRSNNc | 105 | | yYDLR2RFN32DTSP2c | 23 | | yFGVR2FYNRNc | 0 | |
| vlGLS2YVNRSc | 82 | | y1SVRRY31LN3NRSLc | 21 | | yIGLSHYVNRSc | 10 | |
| yRSSN1RPRLRY22Lc | 80 | | yFDVR2FYNRTc | 21 | | yVGLN2SPNRTc | 10 | |
| yYGLS2YVSNc | 74 | | yFGLG2FYRRSc | 21 | | yYALN2Y1SNc | 10 | |
| yFGVR2LYNRTc | 71 | | yYDVR2F1RTc | 21 | | yYDLR2R1Gc | 10 | |
| yFSVY2FFNRLc | 71 | | yYDVR2FLRTc | 21 | | yYNLR2A1NTNc | 10 | |
| y1GLG2YVNRSc | 69 | | y1ALN2A1NRSc | 20 | | | | |
| yYDVR2R1VNc | 64 | | y1GLN21LNRAc | 20 | | | | |
| yFGLT2YLNRNc | 63 | | y1GPS2YVNRSc | 20 | | | | |
| yLGVR2FYNRTc | 63 | | y1SPRR2TFNRTc | 20 | | | | |
| yPRYNL3GASPRF2Nc | 63 | I2 | yFGVR2FIIVL | 20 | | | | |
| y1GR2YFNRSc | 62 | | y1GLS2YANRSc | 19 | | | | |
| yYDLR2Y1RNc | 61 | | y1SLS2YVNRSc | 19 | | | | |
| yFGVRHFYNRTc | 60 | | yFGVR2F3NRTc | 19 | | | | |
| yFGAR2FYNRTc | 55 | | y1GLS2YVSRSc | 18 | | | | |
| yNSRVRLYLNPc | 54 | | y1GVR2FYNRTc | 18 | | | | |
| yYDL32R1SNc | 54 | | yFGLS2LYRRSc | 18 | | | | |
| yFGVR2FYNRAc | 52 | | yVGLNHSLNRTc | 18 | | | | |
| yFGR22FNRSc | 50 | | y1AVT2FVNRSNDc | 17 | | | | |
| y1GLSRSNPN3RNPc | 48 | | y1GLN2YVNRSc | 17 | | | | |
| yFALR2FFANc | 46 | y1GLSRYVNRSc | y1GLSRYVNRSc | 17 | | | | |
| yYGLS2YFRDc | 46 | | y3DVR2R1SNc | 17 | | | | |
| yYNLRPF1SNc | 46 | | yFGLS2YVNRSc | 17 | | | | |
| y1GLS2Y1NRSc | 45 | | yFGVRRFYNRTc | 17 | | | | |
| yFGVR2FYDRTc | 44 | | yYGLN2T1N3Gc | 17 | | | | |
| yYSVR2A1NRVc | 44 | | yYGVN2SYNRTc | 17 | | | | |
| yFGVR2FYNRSc | 42 | | y1DLS2YVNRSc | 16 | | | | |
| yYGLS21VSLc | 37 | | y1GLS7YVNRGc | 16 | | | | |
| y1GLS2YVNRNc | 36 | | yFDLR2Y13Nc | 16 | | | | |
| yFGLS2FYRRTc | 36 | | yRA1R2SGRLNSc | 16 | | | | |
| yGLS2YVDRSc | 33 | yY1RY22Lc | yY1RY22Lc | 16 | | | | |

Four species bound to FXIIa (Table 9, F1 to F4) and seven species bound to IFNGR1 (Table 10, I1-1 to 11-6 and 12) in Tables 9 and 10 were subjected to chemical synthesis (solid-phase synthesis) and further analysis. The C-terminal TGTGT linker sequence was omitted in the chemical synthesis. In addition, negative control peptides having cβAA residue substituted with alanine were synthesized.

The binding kinetics of those peptides to FXIIa and IFNGR1 was analyzed by surface plasmon resonance. The control peptides showed significantly low affinity for the target, showing that the cβAA residue was essential for firm binding to the target. The binding affinity was analyzed at 25°C by SPR by using a Biacore T200 apparatus (GE Healthcare). The peptides, each used at five different concentrations, were injected at a flow rate of 30 µL/min and the rate constant was measured by single cycle kinetics method. The binding sensorgrams thus obtained were analyzed using a Biacore evaluation software and was fitted to a standard 1:1 interaction model.

Furthermore, the inhibitory activity of F1, F2, F3, and F4 against the enzymatic activity of FXIIa was analyzed and their Ki values were measured. Their inhibitory activity was significantly stronger than that of a corn trypsin inhibitor (CTI, Ki=24 nM, M.W.=12.5kDa), a small protein inhibitor against FXIIa. The Ki value of FXIIa was evaluated by cleavage of substrate peptide H-D-Pro-Phe-Arg-pNA (reagent S-2302). The release of the pNA chromophore by cleavage reaction was monitored using a plate reader Infinite M1000PRO (TECAN) at an absorbance at 405 nm.

The structure, surface plasmon resonance measurement results, and Ki value measurement results of the synthesized peptides are shown in Table 11 and Table 12.

**[Table 11]**

| Peptide name | Sequence | Read number | *kₐ* (10^{6.}M^{-1.}s⁻¹) | *k*_{d} (10^{-2.}s⁻¹) | *K*_{D} (nM) | *K*ᵢ (nM) |
|---|---|---|---|---|---|---|
| F1 | S AcyNDRSTR2RLVAcG-NH2 | 10796 | 2.74 | 2.25 | 8.21 | 22.1 |
| F1A | S AcyNDRSTRARLVAcG-NH2 | 0 | 0.58 | 2.97 | 51.3 | not tested |
| F2 | S AcyPRLFN2SYLRRcG-NH2 | 10761 | 30.7 | 1.78 | 0.58 | 2.14 |
| F2A | S AcyPRLFNASYLRRcG-NH2 | 0 | 4.75 | 43.8 | 92.2 | not tested |
| F3 | S AcyFAYDRR1LSNN1RNYcG-NH2 | 1601 | 1.59 | 0.16 | 0.98 | 1.02 |
| F3A | S AcyFAYDRRALSNNARNYcG-NH2 | 0 | --- | --- | --- | not tested |
| F4 | S AcyRYT1NRLF1NAcG-NH2 | 204 | 0.55 | 0.85 | 15.5 | 4.32 |
| F4A | S AcyRYTANRLFANAcG-NH2 | 0 | --- | --- | --- | not tested |

**[Table 12]**

| Peptide name | Sequence | Read number | kₐ (10⁶·M⁻¹·s⁻¹) | k_{d}(10⁻²·s⁻¹) | K_{D} (nM) |
|---|---|---|---|---|---|
| I1-1 | S AcyFGVR2FYNRTcG-NH2 | 15739 | 1.74 | 0.87 | 5.00 |
| I1-2 | S Acy1GLS2YVNRScG-NH2 | 8376 | --- | --- | --- |
| 11-3 | S AcyVGLN2SLNRTcG-NH2 | 4401 | 1.41 | 21.1 | 150 |
| I1-4 | S ^AcyFSLR2SFNRSRGcG-NH₂ | 2118 | 3.39 | 1.95 | 5.76 |
| I1-5 | S AcyFALN2R1NRRcG-NH2 | 779 | 3.01 | 101 | 336 |
| I1-6 | S AcyFGV32FYNRTcG-NH2 | 31 | 0.92 | 0.17 | 1.87 |
| I1-1A | S AcyFGVRAFYNRTcG-NH2 | 0 | 0.02 | 4.12 | 2390 |
| I2 | S AcyPRYNL3GASPRF2NcG-NH2 | 63 | 1.98 | 2.43 | 12.3 |
| I2A | S AcyPRYNLAGASPRFANcG-NH2 | 0 | --- | --- | --- |

### [Example 5: Serum stability of peptides bound to FXIIa and IFNGR1]

The serum stability of peptides was analyzed using, for FXIIa, F1, F2, F3, and F4 and Ala mutants F1A, F3A, and F4A and for IFNGR1, 11-6 and a mutant thereof I1-1A.

The aforesaid peptides and the following peptidase-resistant internal standard peptide were co-incubated at 37°C in the human serum.

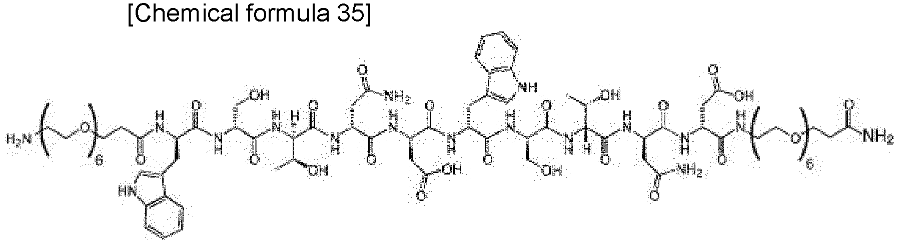

A relative amount of the sample peptides to the standard peptide at 0, 1, 3, 9, 24, 72, 144, and 240 hours was calculated by liquid chromatography/mass spectrum (LC/MS). A change in relative amount at 0, 1, 3, 9, 24, 72, 144, and 240 hours is shown in Fig. 9.

High serum resistance and binding/inhibitory activity of peptides were observed, revealing that a library including cyclic peptides containing cβAA expressed between ^{ClAc}D-Tyr and D-Cys can be applied to the development of a unique peptide drug.

### [Example 6: Cocrystal structure of F3 bound to FXIIa]

To elucidate the action mechanism and folding tendency of the cβAAcontaining peptide, the cocrystal structure of F3 bound to FXIIa was analyzed by X-ray crystallography. The analysis results are shown in Fig. 10.

The diffraction data revealed that F3 was folded into an antiparallel β sheet and in the folded sheet, (1S,2S)-2-ACHC at position 8 (ACHC8) was located at the edge of the turn of the β-sheet close to the active center of FXIIa. The ^{Ac}D-Tyr1 and D-Cys17 were located at the other edge of the β sheet distant from the active site of the enzyme, which is expected since the D-Cys17 is bound to the cognate mRNA via the peptide-puromycin linker during the selection.

It has also been found that Arg7, ACHC8, and Leu9 were present at positions i, i+1, and i+2, respectively and ACHC8 incorporated into a middle position of the sequence induced a pseudo γ turn.

The above results accorded with the previous report that a β amino acid at position i+1 was a pseudo γ turn inducer (Schumann, F., Muller, A., Koksch, M., Muller, G. & Sewald, N. Are β-amino acids γ-turn mimetics? Exploring a new design principle for bioactive cyclopeptides. J. Am. Chem. Soc. 122, 12009-12010 (2000)). Further, a reverse γ turn was formed by ACHC8, Leu9, and Ser10 (i, i+1, and i+2, respectively). Such two consecutive γ turns bound to ACHC8 were unique characteristics of the peptide, which were presumed to contribute to the folding of the peptide into the anti-parallel β sheet.

Another (1S,2S)-2-ACHC(ACHC13) at position 13 was involved in a β turn in which ACHC13, Arg14, Asn15, and Tyr16 held the positions i to i+3. The amide group of ACHC13 also formed an intramolecular β sheet-like hydrogen bond with Ala3 and its cyclic side-chain formed an intramolecular hydrophobic interaction with Ala3 and Tyr16.

As described above, it has been made clear that two (1S,2S)-2-ACHC residues contributed to the overall folding by inducing a turn structure and forming an intramolecular interaction.

F3 bound to the active site of FXIIa in a substrate-like manner and acted as an inhibitor having a so-called standard mechanism (Refer to Farady, C. J. & Craik, C. S. Mechanisms of macromolecular protease inhibitors. ChemBioChem 11, 2341-2346 (2010) and Laskowski, M. & Kato, I. Protein inhibitors of proteinases. Annu. Rev. Biochem. 49, 593-626 (1980)).

The peptide bond between Arg6 and Arg7 was located close to the catalytic Ser563 of FXIIa.

The Arg6 side chain formed a salt bridge with the Asp557 of FXIIa and was accommodated in the S1 pocket of the enzyme. Arg6 is a widely conserved amino acid among natural substrates of FXIIa as well as previously reported inhibitors such as PPACK, Infestin-4, EcTI (E. contortisiliquum trypsin inhibitor), CTI, FXII618, and FXII801.

The Arg7 side chain also formed a hydrogen bond with Ser395 and Cys413, while the ACHC8 side chain bound to a shallow hydrophobic pocket formed by Tyr515 and His507 of FXIIa. F3 also formed an intramolecular β sheet-like hydrogen bond with FXIIa Gly587 via its Tyr4 and its steric conformation was stabilized by the formation of an intramolecular β sheet with Asn11-ACHC13. In addition, there are extensive hydrophobic interactions around ACHC13, contributed by Ala3 and Tyr16 of F3 and Tyr458 and Trp586 of FXIIa.

### [Example 7: Incorporation test of γ amino acid into peptide]

An incorporation test of the following γ-amino acids was performed in accordance with the method of Example 1.

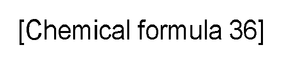

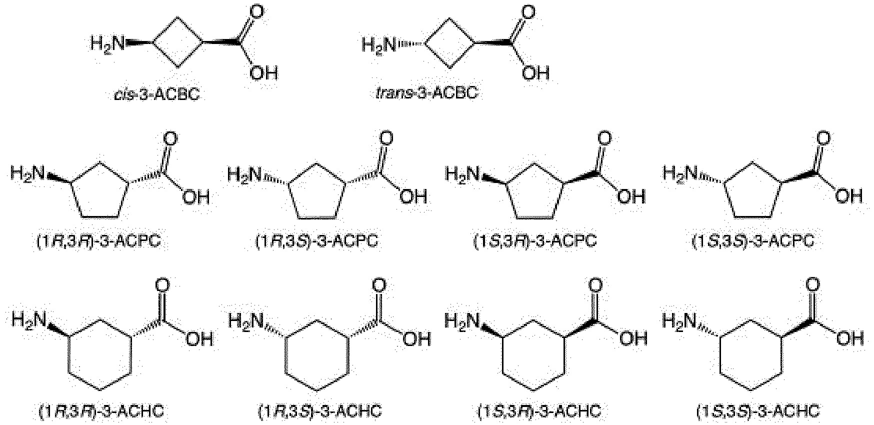

The following mR1 was used as a template mRNA and translated into peptide rP1. The γ amino acid was charged onto tRNA^{Pro1E2}_{CGG}.

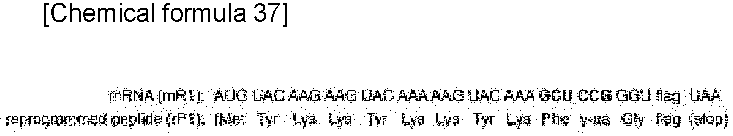

As a result, it was confirmed from MALDI-TOF MS that eight cyclic γ-amino acids, that is, cis-3-ACBC, trans-3-ACBC, (1R,3R)-3-ACPC, (1R,3S)-3-ACPC, (1S,3R)-3-ACPC, (1R,3S)-3-ACPC, (1R,3R)-3-ACHC, and (1R,3S)-3-ACHC were incorporated in the peptide rP1 (refer to Fig. 11).

In accordance with the method of Example 1, the incorporation efficiency of each of the steric isomers of the γ amino acids into the peptide rP1, depending on the presence or absence of EF-P was studied.

The results of the incorporation efficiency of each of the steric isomers of the β or γ amino acids studied through Example 1 and Example 7 are shown in Table 13.

**[Table 13]**

| Substrate | Effect of EF-P |
|---|---|
| (1R,2R)-2-ACPC | inhibition |
| (1R,2S)-2-ACPC | promotion |
| (1S,2R)-2-ACPC | inhibition |
| (1S,2S)-2-ACPC | inhibition |
| | |
| (1R,2R)-2-ACHC | promotion |
| (1R,2S)-2-ACHC | no effect |
| (1S,2R)-2-ACHC | no effect |
| (1S,2S)-2-ACHC | promotion |
| cis-3-ACBC | promotion |
| trans-3-ACBC | promotion |
| | |
| (1R,3R)-3-ACPC | promotion |
| (1R,3S)-3-ACPC | inhibition |
| (1S,3R)-3-ACPC | inhibition |
| (1S,3S)-3-ACPC | promotion |
| | |
| (1R,3R)-3-ACHC | promotion |
| (1R,3S)-3-ACHC | inhibition |
| (1S,3R)-3-ACHC | - |
| (1S,3S)-3-ACHC | - |

### [Example 8: Incorporation test of aromatic amino acid into peptide]

The incorporation test of the following aromatic amino acids was performed. The σ values of p-hydroxy-, p-methoxy-, and p-fluoro-substituted aromatic amino acids as the aromatic amino acid having a substituent are shown.

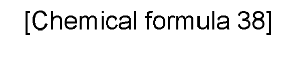

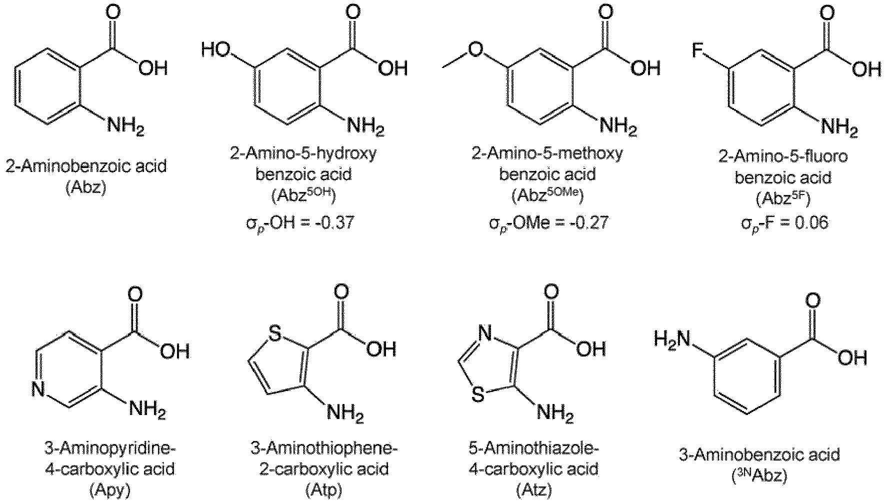

### (Preparation of tRNA)

The tRNAs to be charged with the aromatic amino acid were prepared in a manner similar to that described above (preparation of tRNA). Refer to Table 14 about the sequence of a primer.

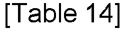

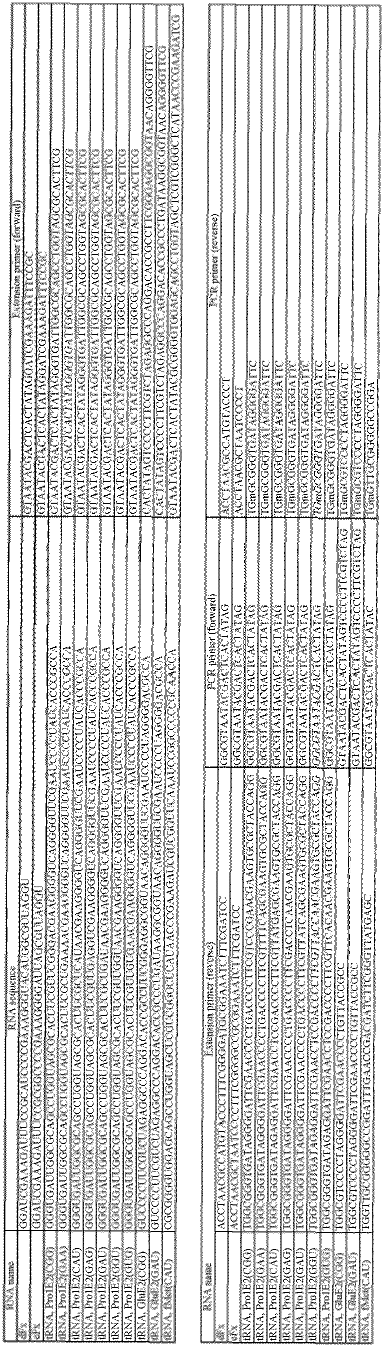

### (Aminoacylation of µhRNA and tRNA)

µhRNA, isatoic anhydride, 5-hydroxyisatoic anhydride, 5-methoxyisatoic anhydride, and 5-fluoroisatoic anhydride were purchased from Eurofin Genomics, Tokyo Chemical Industry, FUJIFILM Wako Chemicals, Combi-Blocks, and BLD Pharm, respectively (the anhydrides used in the experiment have the following structures).

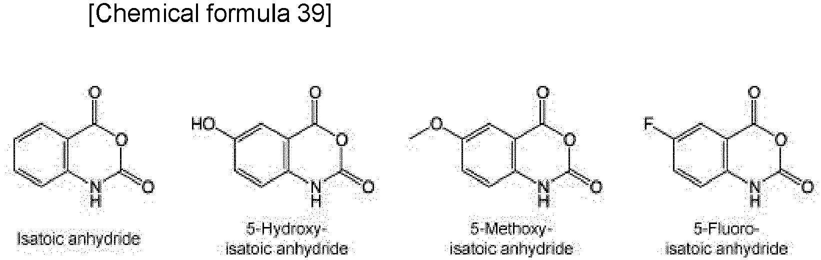

The aminoacylation using those anhydrides was performed at 25°C for 1 to 3 hours in 25 µL of a reaction mixture containing 80 µM µhRNA or tRNA containing 50 mM Bicine-KOH (pH 9.0), CHES-KOH (pH 9.5 or 10.0), or CAPS-KOH (pH 10.5). The concentration of the anhydrides except 5-fluoroisatoic anhydride (8 mM) was set at 10 mM.

The ^{Me}Ser, (1S,2S)-2-ACPC, and D-Cys were preactivated as a 3,5-dinitrobenzyl ester (DBE) form and the Apy, Atp, Atz, ^{3N}Abz, and ^{ClaAc}D-Phe were preactivated as a cyanomethyl ester (CME) form. Those activated amino acids were charged onto µhRNA or tRNA by using flexizyme (dFx for DBE and eFx for CME).

The aminoacylation of Apy, Atp, Atz, and ^{3N}Abz was performed at 4°C for 24 to 336 hours in a reaction mixture containing 600 mM MgCl₂, 20% DMSO, 25 µM eFx, 25 µM µhRNA or tRNA, and 20 mM activated amino acid. The pH of the mixture was adjusted with 50 mM Bicine-KOH (pH 9.0), CHES-KOH (pH 9.5 or 10.0), or CAPS-KOH (pH 10.5).

The aminoacylation of ^{ClAc}D-Phe, ^{Me}Ser, (1S,2S)-2-ACPC, and D-Cys was performed at 4°C in a 50 mM buffer (Bicine-KOH (pH 8.7) for (1S,2S)-2-ACPC and HEPES-KOH (pH 7.5) for ^{ClAc}D-Phe, ^{Me}Ser and D-Cys), 600 mM MgCl₂, 20% DMSO, 25 µM dFx or eFx, 25 µM tRNA, and 5 mM activated amino acid. The reaction time was 2 hours for ^{ClAc}D-Phe, 6 hours for ^{Me}Ser and D-Cys, and 16 hours for (1S,2S)-2-ACPC. Then, the aminoacyl-tRNAs thus obtained were subjected to ethanol precipitation and the pellet was washed twice with 70% ethanol.

Aminoacyl-µhRNA (20 pmol) was analyzed using 20% polyacrylamide gel containing 6 M urea. The pH was adjusted to 5.2 with 50 mM sodium acetate (pH 5.2) except in the analysis of Atp-µhRNA and Atz-µhRNA (pH 3.5). Electrophoresis was performed for 2.5 hours at 120 V, followed by ethidium bromide staining and detection using Typhoon FLA 7000 (GE Healthcare).

### (Translation of peptide and electrophoresis)

The translation of the peptide was performed at 37°C for 30 minutes in a system similar to the Escherichia-coli reconstituted translation system having the composition of Table 5 except for the addition of 0.11 µM LysRS, 0.68 µM PheRS, and 0.5 mM Phe. Then, 5 µL of a stop solution [0.9M Tris-HCI (pH 8.45), 8% SDS, 30% glycerol, and 0.001% xylene cyanol] was added to 5 µL of the reaction mixtures and the resulting mixtures were incubated at 95°C for 3 minutes. Then, the samples were subjected to 15% tricine SDS-PAGE and analyzed by autoradiography using Typhoon FLA 7000 (GE Healthcare). The expression level of the peptide was normalized by the intensity of the [¹⁴C]-Asp band.

### (MALDI-TOF MS of peptide and aminoacyl-µhRNA)

For MALDI-TOF MS, the translation reaction was performed for 60 minutes in the presence of 0.5 mM non-radioactive Asp instead of [¹⁴C]-Asp. The translation of the cyclic peptides (rP5 to rP10, refer to Fig. 14) was performed while omitting MetRS, Met, and 10-formyl-5,6,7,8-tetrahydrofolic acid from the aforesaid reaction mixture. For rP5 to rP7, PheRS and Phe were also omitted and instead, the following aminoacyl-tRNA synthetases and amino acids corresponding thereto were added: 0.04 µM SerRS, 0.02 µM HisRS, 0.16 µM ProRS, 0.09 µM ThrRS, 0.03 µM ArgRS, 0.38 µM AsnRS, 0.73 µM AlaRS, 0.02 µM VaIRS, 0.5 mM Ser, 0.5 mM His, 0.5 mM Pro, 0.5 mM Thr, 0.5 mM Arg, 0.5 mM Asn, 0.5 mM Ala, and 0.5 mM Val. To the translation reaction mixture was added an equivalent amount of an HBS buffer (100 mM HEPES-KOH (pH 7.6) and 300 mM NaCl) twice. The resulting mixture was mixed with 5 µL of an ANTI-FLAG M2 affinity gel (Sigma), followed by incubation at room temperature for 30 minutes. The peptides bound to the affinity gel were eluted by washing the gel twice with 25 µL of an HBS buffer (50 mM HEPES-KOH (pH 7.6) and 150 mM NaCl) and adding 20 µL of 0.2% trifluoroacetic acid. Then, the peptides were desalted using SPE C-Tip (Nikkyo Technos) and cocrystallized with α-cyano-4-hydroxycinnamic acid. Peptide calibration standard II (Bruker Daltonics), was used as external mass calibration. MALDI-TOF MS analysis was performed in a reflector/positive mode by using UltrafleXtreme (Bruker Daltonics). The aminoacyl-µhRNA was desalted with SPE C-Tip (Nikkyo Technos), cocrystallized with 3-hydroxypicolic acid, and then, analyzed in a linear/positive mode by UltrafleXtreme (Bruker Daltonics).

The conditions of the following table were applied to the preparation of am inoacyl-tRNA^{Pro1E2}_{CGG}.

**[Table 15]**

| **Amino acid** | **Substrate** | **Conc. of substrate (mM)** | **pH** | **Temperature (°C)** | **Time (h)** |
|---|---|---|---|---|---|
| Abz | Isatoic anhydride | 10 | 9.5 | 25 | 3 |
| Abz^{5O} | 5-Hydroxyisatoic anhydride | 10 | 10.5 | 25 | 1 |
| Abz^{5OMc} | 5-Hydroxyisatoic anhydride | 10 | 10 | 25 | 3 |
| Abz^{5F} | 5-Fluoroisatoic anhydride | 8 | 10 | 25 | 3 |
| ^{3N}Abz | ^{3N}Abz-CME(+ eFx) | 20 | 10 | 4 | 144 |
| Apy | Apy-CME(+ eFx) | 20 | 9 | 4 | 144 |
| Atp | Atp-CME(+ eFx) | 20 | 9.5 | 4 | 192 |
| Atz | Atz-CME(+ eFx) | 20 | 10.5 | 4 | 192 |

By introducing the respective aminoacyl-tRNA^{Pro1E2}_{CGG} (Abz, Abz^{5OH}, Abz^{5OMe}, Abz^{5F}, Apy, Atp, and Atz) into the CCG codon of the mRNA (mR1), rP1 peptides were synthesized (Fig. 12a). The translation reaction was performed using a flexible in vitro translation (FIT) system including 6 amino acids ((Met, Tyr, Lys, Phe, Asp, and Gly) and aminoacyl-tRNA synthetases (ARS) corresponding thereto but lacking the other 14 amino acids and aminoacyl-tRNA synthetases thereof. The identity of the peptides was confirmed by MALDI-TOF MS and the desired m/z values of [M+H]⁺ and [M+2H]²⁺ ions without a byproduct were obtained (Fig. 12b). The same peptides were translated in the presence of [¹⁴C]-labeled Asp, subjected to tricine SDS-PAGE, and then, quantified by autoradiography (Fig. 20). The rP1 expression levels in the presence of EF-P, compared with that in the absence of EF-P, was raised from 1.5 times to 7 times (Fig. 12c).

Next, mRNAs (mR2 to mR4) were prepared to express peptides (rP2 to rP4, Fig. 13a) in the presence of Abz-tRNA^{Pro1E2}_{CGG} and EF-P in order to study the possibility of consecutive or multiple incorporation of Abz. The rP2 peptide has two consecutive Abz residues, while rP3 and rP4 peptides have one or two Phe residues inserted between two Abz residues. The expression of each of the peptides (rP2 to rP4) labeled with [¹⁴C]Asp was analyzed by tricine SDS-PAGE. Discrete bands in the rP4 expression were observed with a level (0.065 vs. 0.072 pmol/µL) comparable to that of rP1 (Fig. 13b and Fig. 21) and the identity of rP4 peptide with the expected molecular weight was confirmed by MALDI-TOF MS spectrum (Fig. 13c).

Next, cyclic peptides containing a plurality of extraneous amino acids including an Abz derivative were expressed. Template mRNAs (mR5 to mR10) were designed to express peptides (rP5 to rP10) in a reprogrammed genetic code (Fig. 14a). The reprogramming was performed to use N-chloroacetyl-D-tyrosine (^{ClAc}D-Tyr) or N-chloroacetyl-D-phenylalanine (^{ClAc}D-Phe) as an initiator (tRNA^{fMet}_{CAU} was charged with eFx/^{ClAc}D-Tyr-CME or eFx/^{ClAc}D-Phe-CME) and to introduce D-Cys at the downstream position (tRNA^{Pro1E2}_{GUG} was charged with dFx/D-Cys-DBE, in which DBE was a 3,5-dinitrobenzyl ester). The ClAc group on the D-amino acid and sulfhydryl group were spontaneously cyclized via a thioether bond when the entire peptide was synthesized (Fig. 14a). Between those residues for cyclization, an amino acid bound to tRNA^{Pro1E2}_{NNN} (NNN was an anticodon corresponding thereto) was introduced into the amino acid sequence of the peptide (rP5, rP6, or rP7) (Fig. 14a). As a result of the MALDI-TOF MS spectra of those peptides (rP5 to rP7) (Fig. 14b), it was confirmed that correct cyclic peptides were expressed. In order to express rP8 peptide, N-methyl-1-α-Ser (^{Me}Ser), (1S,2S)-2-ACPC, Abz, and Abz^{5OMe} were introduced using ^{Me}Ser-tRNA^{Pro1E2}_{GAU}, (1S,2S)-2-ACPC-tRNA^{GlUE2}_{CGG}, and Abz-tRNA^{Pro1E2}_{GAU} or AbZ^{50Me}-tRNA^{Pro1E2}_{GGU}. The MALDI-TOF MS spectrum of the rP8 peptide containing Abz or Abz^{5OMe} showed a desirable peak (Fig. 14b).

In the end, two Abz derivatives were incorporated into the cyclic structure of peptides (rP9 and rP10) to confirm the incorporation possibility of a plurality of Abz derivatives (Fig. 14b).

From the present examples, it was confirmed that one or more aromatic amino acids can be introduced into the main chain bond in the middle of the peptide sequence in the elongation process of a non-N-terminal site peptide chain. The most important advantage of the ribosomal peptide synthesis over the chemical synthesis is that since it can be confirmed that by the present invention, an aromatic amino acid-containing cyclic peptide can be expressed by the FIT system, it becomes a first step for developing a RaPID (Random Non-standard Peptides Integrated Discovery) platform of a physiologically active aromatic amino acid-containing cyclic peptide and sending a physiologically active "foldamer-like" peptide to a research program.

## Claims

1. A method of producing a library including two or more cyclic peptides, wherein at least one of the cyclic peptides included in the library has a cyclic structure having 4 to 30 amino acids or derivatives thereof and comprises, in the cyclic structure, at least one selected from cyclic β-, γ-, and δ-amino acids (cAAs), comprising:
a step of preparing an mRNA library encoding a peptide comprising a sequence represented by the following formula (1);
-(Xaa)ₙ₁- (1)
[in the formula (1),
Xaas are each an arbitrary amino acid or derivative thereof,
at least one of Xaas is one selected from cyclic β-, γ-, and δ-amino acids (cAAs), and
n1 is an integer of 2 to 28]; and
a step of using the mRNA library to express the peptide in a cell-free translation system and produce the library.

2. A method of producing a library including two or more cyclic peptides, wherein at least one of the cyclic peptides included in the library has a cyclic structure having 4 to 30 amino acids or derivatives thereof and comprises, in the cyclic structure, at least one selected from cyclic β-, γ-, and δ-amino acids (cAAs), comprising:
a step of preparing an mRNA library encoding a peptide comprising a sequence represented by the following formula (1);
-(Xaa)ₙ₁- (1)
[in the formula (1),
Xaas are each an arbitrary amino acid or derivative thereof,
at least one of Xaas is one selected from cyclic β-, γ-, and δ-amino acids (cAAs), and
n1 is an integer of 2 to 28];
a step of binding puromycin to the 3' end of each of the mRNAs of the mRNA library to produce a puromycin-bound mRNA library; and
a step of using the puromycin-bound mRNA library to express the peptide in a cell-free translation system and produce a peptide-mRNA complex library.

3. The method of producing a library according to Claim 1 or 2, wherein the cell-free translation system comprises a tRNA charged with one selected from cyclic β-, γ-, and δ-amino acids (cAAs) and the tRNA is a tRNA having a D-arm structure interactive with EF-P or a tRNA not having a D-arm structure interactive with EF-P.

4. The method of producing a library according to any one of Claims 1 to 3, wherein the cell-free translation system comprises a tRNA charged with one selected from cyclic β-, γ-, and δ-amino acids (cAAs) and the tRNA is at least one selected from tRNA^{Pro1E2} and tRNA^{GluE2}.

5. The method of producing a library according to any one of Claims 1 to 4, wherein the peptide comprising the sequence represented by the formula (1) is represented by the following formula (2);
Xaa1-(Xaa)ₙ₁-Xaa2-(Xaaₓ)ₘ (2)
[in the formula (2),
Xaas are each an arbitrary amino acid or derivative thereof,
at least one of Xaas is one selected from cyclic β-, γ-, and δ-amino acids (cAAs),
Xaa1 and Xaa2 are each an amino acid or derivative thereof which forms a ring of the cyclic peptide,
Xaaₓ is an arbitrary amino acid or derivative thereof, and
n1 is an integer of 2 to 28, and
m is an integer of 0 to 10].

6. The method of producing a library according to any one of Claims 1 to 5, wherein the cyclic β-, γ-, and δ-amino acids are each represented by any of the following formulas (I-1); 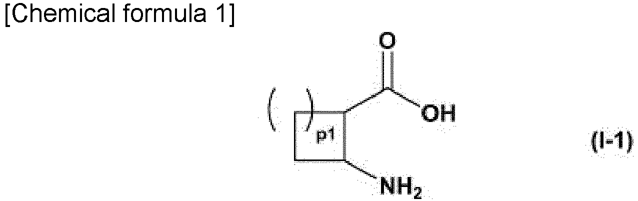
(in the formula (I-1), p1 is any integer of 1 to 4), the following formula (I-2); 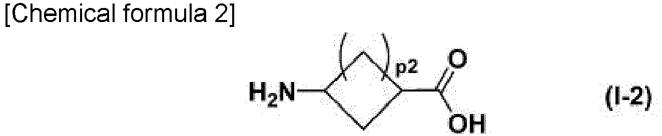
(in the formula (I-2), p2 is any integer of 1 to 4); the following formula (I-3); 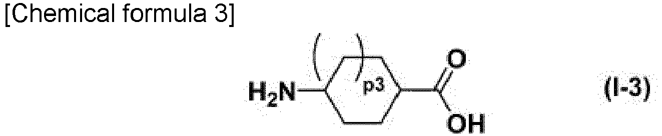
(in the formula (I-3), p3 is an integer of 1 or 2); and the following formula (I-4);
H₂N-Ar-COOH (I-4)
(in the formula (I-4), Ar is a divalent aromatic group whose aromatic ring may be substituted with one or more substituents).

7. The method of producing a library according to any one of Claims 1 to 6, wherein two or more cAAs are contained at random in the sequence represented by the formula (1).

8. The method of producing a library according to any one of Claims 1 to 6, wherein two or more consecutive cAAs are contained in the sequence represented by the formula (1).

9. The method of producing a library according to any one of Claims 1 to 6, wherein 3 or more and 15 or less consecutive cAAs are contained in the sequence represented by the formula (1) and are each a cyclic β-amino acid represented by
(1S,2S)-2-ACPC:

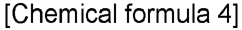

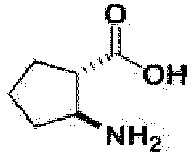

10. The method of producing a library according to any one of Claims 1 to 8, wherein the cAA is at least one cyclic β- or γ-amino acid (i) selected from: 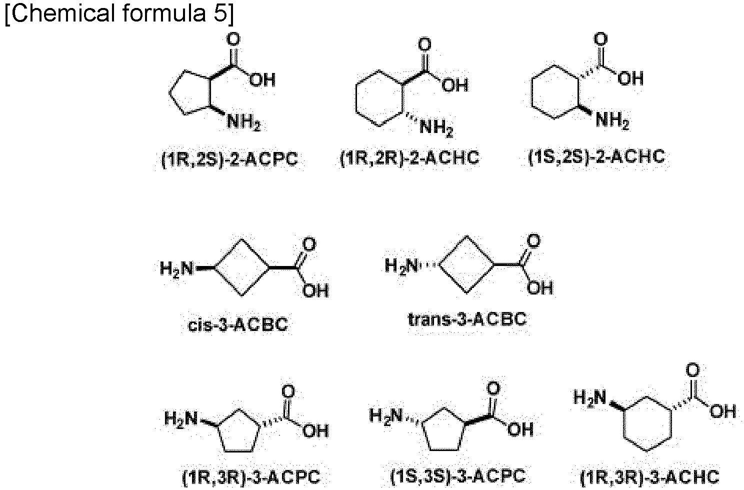 and/or at least one cyclic β- or γ-amino acid (ii) selected from: 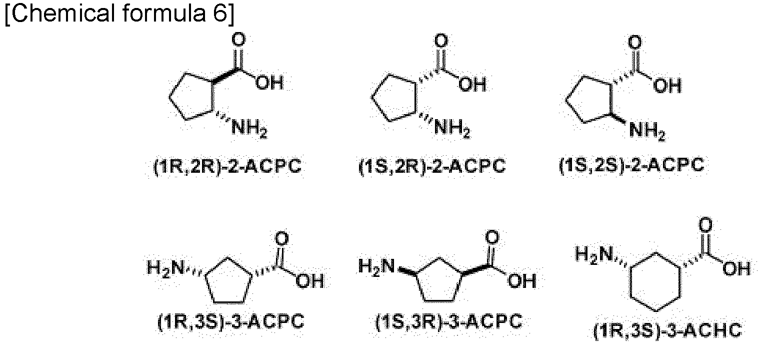 and
the cell-free translation system comprises a tRNA charged with the cyclic βor γ-amino acid (i) and having a D-arm structure interactive with EF-P, and/or
a tRNA charged with the cyclic β- or γ-amino acid (ii) and not having a D-arm structure interactive with EF-P.

11. The method of producing a library according to Claim 10, wherein the tRNA charged with the cyclic β- or γ-amino acid (i) and having a D-arm structure interactive with EF-P is tRNA^{Pro1E2} and the tRNA charged with the cyclic β- or γ-amino acid (ii) and not having a D-arm structure interactive with EF-P is tRNA^{GluE2}.

12. A cyclic peptide comprising a cyclic structure having 4 to 30 amino acids or derivatives thereof or a pharmaceutically acceptable salt of the cyclic peptide, wherein
of the 4 to 30 amino acids or derivatives of the cyclic structure, two amino acids or derivatives thereof, that is, Xaa1 and Xaa2 comprise a structure for forming the cyclic structure,
the Xaa1 and the Xaa2 have a linked structure via an amino acid sequence having 2 to 28 amino acids or derivatives thereof,
the amino acid sequence having 2 to 28 amino acids or derivatives thereof has at least one selected from cyclic β-, γ-, and δ-amino acids (cAAs) and an arbitrary amino acid or derivative thereof.

13. The cyclic peptide or pharmaceutically acceptable salt thereof according to Claim 12, wherein the cyclic peptide is represented by the following formula (3);
Xaa1-(Xaa)ₙ₁-Xaa2-(Xaaₓ)ₘ (3)
[in the formula (3),
Xaas are each an arbitrary amino acid or derivative thereof,
at least one of the Xaas is a cyclic β-amino acid (cβAA),
Xaa1 and Xaa2 are amino acids or derivatives thereof forming the ring of the cyclic peptide,
n1 is an integer of 10 to 16,
Xaaₓ is an arbitrary amino acid or derivative thereof, and
m is an integer of 0 to 10], and
supposing that Xaa1 is a first amino acid, at least one cAA is present in a 5 to 9-th position.

14. The cyclic peptide or pharmaceutically acceptable salt thereof according to Claim 13, wherein at least one of Xaas is a basic amino acid or derivative thereof.

15. The cyclic peptide or pharmaceutically acceptable salt thereof according to Claim 13 or 14, wherein (Xaa)ₙ₁ in the formula (3) is any one selected from the following f1 to f4: 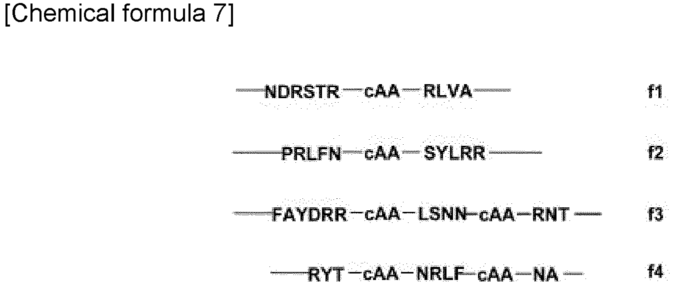 [in the formula, cAA is a cyclic β-amino acid].

16. The cyclic peptide or pharmaceutically acceptable salt thereof according to Claim 13, wherein the cyclic peptide is represented by the following formula (3-1); 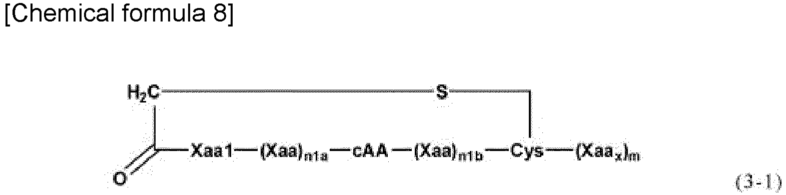 [in the formula (3-1),
Xaas are each an arbitrary amino acid or derivative thereof,
cAA is a cyclic β-amino acid,
n1a is an integer of 3 to 7 and n1b is an integer of 2 to 8 (with the proviso that the sum of n1a and n1b is an integer of 9 to 15),
Xaaₓ is an arbitrary amino acid or derivative thereof,
Xaa1 is Phe or Tyr, and
m is an integer of 0 to 10, with the proviso that (Xaa)ₙ₁ₐ does not comprise cAA].

17. The cyclic peptide or pharmaceutically acceptable salt thereof according to Claim 16, wherein at least one of Xaas is a basic amino acid or derivative thereof.

18. The cyclic peptide or pharmaceutically acceptable salt thereof according to Claim 16 or 17, wherein a difference (absolute value) between n1a and n1b is 4 or less.

19. The cyclic peptide or pharmaceutically acceptable salt thereof according to any one of Claims 13 to 18, wherein the cyclic peptide is any of the following F1 to F4: 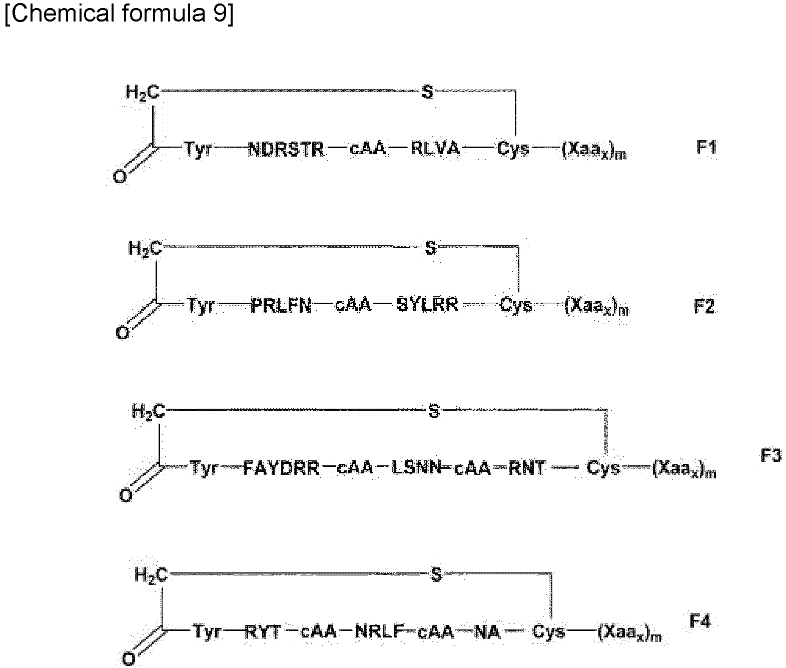 [in the formula,
cAAs are each independently a cyclic β-amino acid,
Xaaₓ is an arbitrary amino acid or derivative thereof, and
m is an integer of 0 to 10].

20. The cyclic peptide or pharmaceutically acceptable salt thereof according to any of Claims 13 to 19, wherein the cyclic β-amino acid is represented by the following formula (I-1); 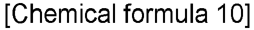 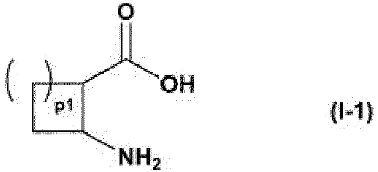 (in the formula (I-1), p1 is any integer of 1 to 4).

21. The cyclic peptide or pharmaceutically acceptable salt thereof according to Claim 12, wherein the cyclic peptide is represented by the following formula (4);
Xaa1-(Xaa)ₙ₁-Xaa2-(Xaaₓ)ₘ (4)
[in the formula (4),
Xaas are each an arbitrary amino acid or derivative thereof,
at least one of Xaas is a cyclic β-amino acid (cAA),
Xaa1 and Xaa2 are amino acids or derivatives thereof forming the ring of the cyclic peptide,
n1 is an integer of 9 to 15,
Xaaₓ is an arbitrary amino acid or derivative thereof, and
m is an integer of 0 to 10], and
supposing that Xaa1 is a first amino acid, at least one cAA is present in a 5 to 8-th position.

22. The cyclic peptide or pharmaceutically acceptable salt thereof according to Claim 21, wherein (Xaa)ₙ₁ in the formula (4) is any one selected from the following i1-1 to i1-6: 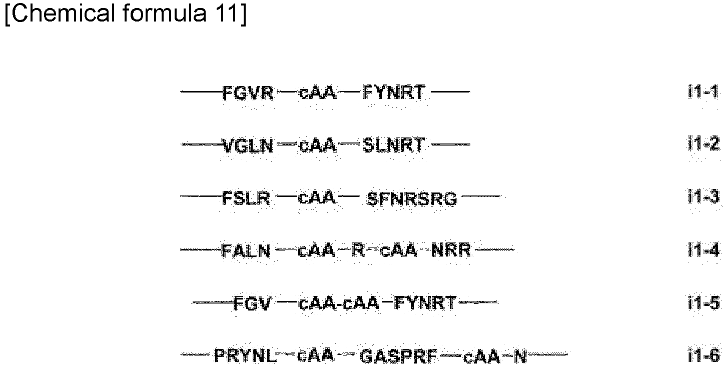 [in the formula, cAA is a cyclic β-amino acid].

23. The cyclic peptide or pharmaceutically acceptable salt thereof according to Claim 21, wherein the cyclic peptide is represented by the following formula (4-1); 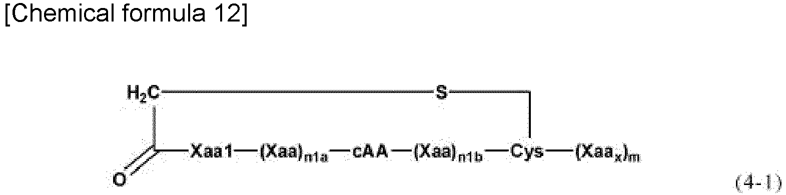 [in the formula (4-1),
Xaa is an arbitrary amino acid or derivative thereof,
cAA is a cyclic β-amino acid,
n1a is an integer of 3 to 6 and n1b is an integer of 5 to 8 (with the proviso that the sum of n1a and n1b is an integer of 8 to 14),
Xaaₓ is an arbitrary amino acid or derivative thereof,
Xaa1 is Phe or Tyr, and
m is an integer of 0 to 10, with the proviso that (Xaa)ₙ₁ₐ does not comprise cAA].

24. The cyclic peptide or pharmaceutically acceptable salt thereof according to any one of Claims 21 to 23, wherein the cyclic peptide is any of the following I1-1 to I1-6: 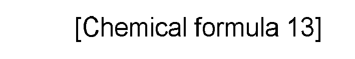 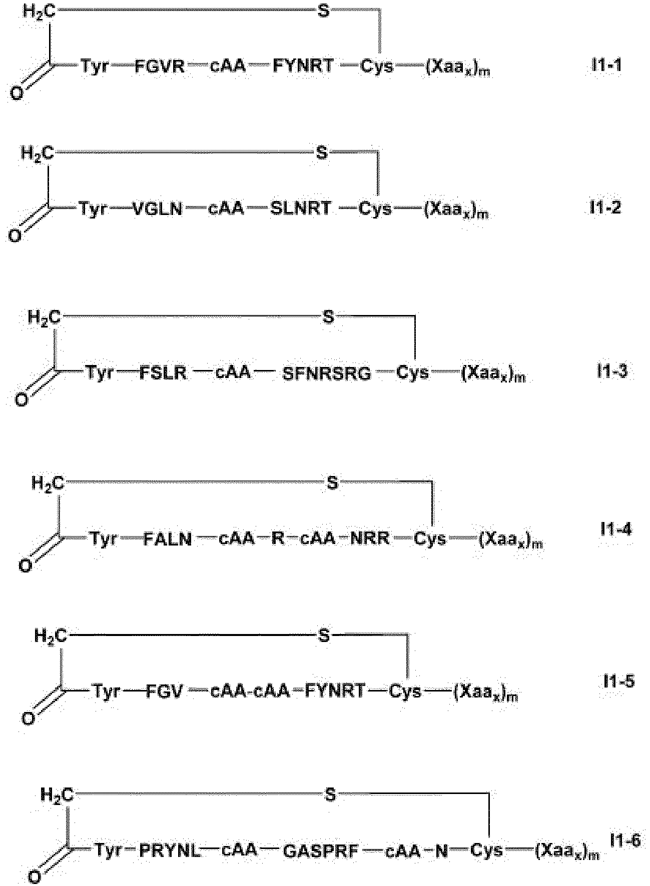 [in the formula,
cAAs are each independently a cyclic β-amino acid,
Xaaₓ is an arbitrary amino acid or derivative thereof, and
m is an integer of 0 to 10].

25. The cyclic peptide or pharmaceutically acceptable salt thereof according to any of Claims 21 to 24, wherein the cyclic β-amino acid is represented by the following formula (I-1); 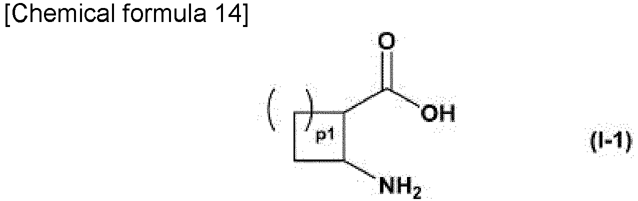 (in the formula (I-1), p1 is any integer of 1 to 4).

26. A binding agent for activated blood coagulation factor XII (FXIIa), comprising the cyclic peptide or pharmaceutically acceptable salt thereof as claimed in any one of Claims 13 to 20.

27. A binding agent for type II interferon receptor complex (IFNGR1), comprising the cyclic peptide or pharmaceutically acceptable salt thereof as claimed in any one of Claims 21 to 25.
